(19) Europäisches Patentamt
European Patent Office
Office européen des brevets

(11) **EP 3 067 027 B1**

(12) **EUROPEAN PATENT SPECIFICATION**

(45) Date of publication and mention
of the grant of the patent:
**27.09.2017 Bulletin 2017/39**

(51) Int Cl.:
***A61F 13/49*** *(2006.01)*

(21) Application number: **15158501.5**

(22) Date of filing: **10.03.2015**

(54) **Absorbent articles with improved barrier leg cuffs**

Absorbierende Artikel mit verbessertem Abschluss an den Beinteilen

Articles absorbants avec des revers de protection améliorés sur les jambes

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**14.09.2016 Bulletin 2016/37**

(73) Proprietor: **The Procter & Gamble Company Cincinnati, OH 45202 (US)**

(72) Inventors:
• **Liebe, Tina**
**65824 Schwalbach am Taunus (DE)**

• **Hippe, Matthias Konrad**
**65824 Schwalbach am Taunus (DE)**

(74) Representative: **Heide, Ute**
**Procter & Gamble Service GmbH**
**IP Department**
**Frankfurter Strasse 145**
**61476 Kronberg im Taunus (DE)**

(56) References cited:
**EP-A2- 0 904 759      EP-A2- 1 132 068**
**US-A1- 2005 267 434    US-A1- 2006 142 723**
**US-B1- 6 186 996**

**Description**

FIELD OF THE INVENTION

The invention relates to personal hygiene absorbent articles for wearing in the lower torso, such as but not limited to diapers and training pants for babies, toddlers and adults, or sanitary napkins. The absorbent articles comprise barrier leg cuffs. More particularly, the invention relates to barrier leg cuffs with improved gasketing when the absorbent article sags upon loading of the absorbent article.

BACKGROUND OF THE INVENTION

Absorbent articles for personal hygiene, such as disposable diapers and training pants for babies, toddlers and adults, are designed to absorb and contain body exudates, in particular large quantity of urine. These absorbent articles comprise several layers providing different functions, for example a topsheet, a backsheet and an absorbent core. Absorbent articles should be able to absorb a large amount of fluid while preventing the fluid from leaking.

Such absorbent articles typically comprise elasticized containment elements at their longitudinal sides to help maintaining the article in contact with the body. It is thus common for diapers to comprise raised flaps, often referred to as barrier leg cuffs (or simply barrier cuffs) which improve the containment of fluid in the leg-torso region of the wearer. Each barrier leg cuff may comprise one or more elastic strands, however, some simpler articles may have non-elasticized barrier leg cuffs. Another type of containment elements commonly found in diapers is often referred to (outer) leg cuffs. These typically comprise one or more elastic strands or elasticized elements inserted in the chassis of the diaper, for example between topsheet and backsheet, between topsheet and an extension of the barrier leg cuff material or between the backsheet and an extension of the barrier leg cuff (if the topsheet has a narrower width than the backsheet). They are positioned in the area of the leg openings longitudinally outboard of the barrier leg cuffs.

Many patents have proposed improvement to these containment elements. For example US4,808,178 and US 4,909,803 (Aziz) describe disposable diapers having such raised elasticized flaps referred to herein as barrier leg cuffs. US 4,695,278 (Lawson) and US 4,795,454 (Dragoo) describe disposable diapers having dual cuffs, including gasketing cuffs and barrier leg cuffs. US 4,704,116 (Enloe) discloses an absorbent garment comprising a pair of gasketing cuffs and a pair of barrier leg cuffs which attached to or formed from the topsheet and spaced inwardly from said elasticized leg openings, defining a waste-containment pocket. Different solutions have been proposed to attach the barrier leg cuffs to the chassis of the diapers. US 4,795,454 (Dragoo) teaches attachment means such as an adhesive or heat/pressure sealing, ultrasonic bonding. Seal means such as an adhesive bead are further provided to present a barrier to the wicking of liquids through the topsheet. The seal means are said to prevent liquids from wicking underneath the barrier leg cuffs to the edges of the diaper. The seal means are positioned more inwardly of the diaper than the attachment means. US 7, 189,219 (Kasai) discloses creating liquid shut-off regions between the leg cuffs and the topsheet using a heat seal.

Barrier leg cuffs typically provide good containment of fluid, which collects on the surface of the topsheet prior to absorption into the absorbent article, when the (absorbent core of the) absorbent article, is little or moderately loaded with urine. Once absorbent articles approach their maximum capacity for liquid absorption, or when absorbent articles are worn for a prolonged period of time, they may tend to sag downward on the wearer due to the weight of the absorbent article. In such circumstances, the barrier leg cuffs may lose sufficient contact with the skin of the wearer, as the elastic elements at least partly contract.

There is thus a need for absorbent articles with barrier leg cuffs providing improved gasketing and containment. There is also a need for absorbent articles with barrier leg cuffs providing improved comfort for the wearer and reducing the risk of red marking on the wearer's skin.

US 6,186,996 relates to a disposable absorbent sanitary article including an absorbent pad placed between a liquid impervious external backing sheet and a hydrophobic internal covering sheet provided with a central opening extending over the absorbent pad. A first set of elastic members is fixed to the backing sheet and to the covering sheet along external edges thereof at least in a crotch area thereof and a second set of elastic members is fixed to the covering sheet along longitudinal edges of the central opening. Two hydrophobic flaps are each realized by fixing a first longitudinal edge of a strip of hydrophobic material close to an external longitudinal edge of the absorbent pad, and a second longitudinal edge to a lower face of the covering sheet close to the elastic members of the second set

US 2006/142723 discloses a sanitary napkin including: a napkin body having a liquid-absorbent layer for absorbing and retaining liquid; and first and second projections each exerting an elastic contractive force between longitudinally opposing front and rear ends to concavely curve the body surface of the napkin body and raise itself from the body surface of the napkin body.

EP 1 132 068 A2 is concerned with an absorbent article including: a support body; a liquid absorbing member positioned on the liquid-receiving side of the support body; and two side wall sheets disposed on two sides of the support body lying opposite one another in the widthwise direction.

EP 0 904759 A2 relates to an absorbent article provided with a pair of side flaps along transversely opposite side edges of the article. Each of these side flaps is formed on its upper surface with a plurality of pleats extending longitudinally of the side flap. Each of the pleats contains an elastically stretchable/contractile member extending along its distal edge so as to operate longitudinally of the pleat.

SUMMARY OF THE INVENTION

An absorbent article is provided, having a longitudinal dimension and a transverse dimension and comprising a liquid pervious topsheet, a liquid impervious backsheet; and an absorbent core disposed between the topsheet and the backsheet. The absorbent article further comprises a barrier leg cuff disposed adjacent to each of the longitudinal edges of the absorbent article and extending substantially parallel to the longitudinal dimension of the absorbent article. Each barrier leg cuff comprises a structure. The structure is attached to the absorbent article with a proximal end. The structure has a longitudinal dimension, which is substantially parallel with the longitudinal dimension of the absorbent article, a lateral dimension and a caliper. The structure comprises one or more elastic elements; and/or the structure being associated with one or more elastic elements. The structure is in a flat configuration when the absorbent article is flattened out and when the one or more elastic elements are stretched along the longitudinal dimension of the structure. Contraction of the one or more elastic elements applies a force along the longitudinal dimension of the structure, thus converting the structure into an erected, three-dimensional configuration, wherein the structure has a higher caliper in its erected configuration compared to the caliper of the structure in its flat configuration. The erected structure spaces a distal end of the barrier leg cuff away from the topsheet and causing the barrier leg cuff to stand up.

The non-elastic materials comprised by the structure may have a bending stiffness of less than 500 mNm, preferably less than 400 mNm, more preferably less than 200 mNm. The non-elastic materials comprised by the structure may have a tensile strength of less than 80 N/cm, preferably less than 50 N/cm, more preferably less than 40 N/cm.

The structure is a multi-level structure, such a multi-level structure comprising a first outermost layer, a second outermost layer, and at least a first intermediate layer. All layers have a longitudinal dimension parallel to the longitudinal dimension of the multi-level structure and are confined by first and second spaced apart lateral edges, and a lateral dimension parallel to the lateral dimension of the multi-level structure and are confined by two spaced apart longitudinal edges. The layers at least partly overlap each other. The at least first intermediate layer is positioned superjacent the first outermost layer and subjacent the second outermost layer. Each level of the multi-level structure is confined in a direction perpendicular to the longitudinal and lateral dimension by one of the layers forming the superjacent layer of the level and further confined by another of the layers forming the subjacent layer of the level. The first and second outermost layer are able to shift relative to each other along the longitudinal multi-level structure dimension upon release of a contractive force, which has provided by the stretched one or more elastic elements along the longitudinal dimension, whereby the multi-level structure is converted from an initial flat configuration into an erected configuration in a direction perpendicular to the longitudinal and the lateral dimension. Each level in the multi-level structure comprises one or more ligament, each ligament having a first surface and a second surface, a longitudinal dimension confined by two spaced apart lateral ligament edges, and a lateral dimension confined by two spaced apart longitudinal ligament edges. Each ligament is provided between the superjacent layer and the subjacent layer of the respective level and is attached with a portion at or adjacent to one of the ligament's lateral edges in a first ligament attachment region to the surface of the subjacent layer which faces towards the superjacent layer of the respective level, and is further attached with a portion at or adjacent to the ligament's other lateral edge in a second ligament attachment region to the surface of the superjacent layer which faces towards the subjacent layer of the respective level. The region of each ligament between the first and second ligament attachment region forms a free intermediate portion. The ligaments in each level are spaced apart from one another along the longitudinal dimension of the multi-level structure when the multi-level structure is in its erected configuration, and the attachment of all ligaments is such that the free intermediate portions of all ligaments in the multi-level structure are able to convert from an initial flat configuration to an erected configuration upon release of a contractive force, which has provided by the stretched one or more elastic elements along the longitudinal dimension of the multi-level structure, thus converting the multi-level structure as a whole from an initial flat configuration into an erected configuration, wherein the erection is in the direction perpendicular to the longitudinal and the lateral dimension of the multi-level structure.

When the ligaments are in their erected configuration, each ligament may either adopt a Z-like shape, a C-like shape, a shape forming a T in one of the first or second ligament attachment region and taking a tilted shape in the other of the first or second ligament attachment region (hereinafter simply referred to as "T-like shape"), or a Double-T-like shape.

The names "Z-like shape", "C-like shape", "T-like shape", and "Double-T-like shape" reflect the appearance and shape of the ligaments when viewed from the side parallel to the lateral direction.

For all ligaments in the multi-level structure, a first surface of the free intermediate portion may face towards the inner surface of the first outermost layer and a second surface of the free intermediate portion may face towards the inner surface second outermost layer in the multi-level structure's flat configuration, and the first surface of the free intermediate portion may face towards the second surface of the neighboring ligament when the multi-level structure is in the erected configuration.

Moreover, for all ligaments in the multi-level structure, the ligament's first surface may not be facing towards the inner surface of the second outermost layer when the multi-level structure is in its initial flat configuration, and the ligament's second surface may not be facing towards the inner surface of the first outermost layer when the multi-level structure is in its initial flat configuration.

The longitudinal dimension of the ligaments in the initial flat configuration of the multi-level structure may be substantially parallel with the longitudinal dimension of the first and second outermost layer and with the intermediate layer(s).

The longitudinal dimension of the free intermediate portion of one or more of the ligaments within a level may or may not differ from the longitudinal dimension of the free intermediate portion of one or more other ligaments within the same level.

The first and second outermost layers, the intermediate layer(s) and/or the ligaments may be made of film, nonwoven material, paper, sheet-like foam, woven fabric, knitted fabric or combinations thereof.

The longitudinal dimension of the ligaments in the initial flat configuration of multi-level structure may be substantially or fully parallel with the longitudinal dimension of the first and second outermost layer and with the intermediate layer(s).

The ligaments may differ from each other in tensile strength and/or in bending stiffness.

In one or more ligaments, those portions of the free intermediate portion which are directly adjacent to the first and/or second ligament attachment region may have a different bending stiffness and/or different tensile strength than the remaining free intermediate portion of said ligament.

The ligaments of the multi-level structure may have a tensile strength of from 1N/cm to 100N/cm and/or may have a bending stiffness of from 0.01mNm to 1Nm.

In the multi-level structure, the longitudinal dimension of the free intermediate portion of the ligaments in the center of the structure may be larger than the longitudinal dimension of the free intermediate portion the ligaments towards the lateral edges of the structure.

The multi-level structure may further comprise a second intermediate layer provided between the first intermediate layer and the first outermost layer such that the structure comprises a third level.

The multi-level structure may further comprise one or more additional intermediate layer(s), provided between the second intermediate layer and the first outermost layer such that the structure comprises a one or more additional levels.

One or more intermediate layers may be discontinuous along the longitudinal layer dimension such that the intermediate layer(s) comprise(s) at least two sections, wherein one or more than one ligament may be attached to each of the sections with one of their first and second ligament attachment regions.

The ligaments of one level may differ from the ligaments of another level in bending stiffness, tensile strength, or both.

The ligaments of a level may differ from each other in bending stiffness and/or tensile strength, preferably one or more ligaments which are closer to the lateral edges of the multi-level structure may have a lower bending stiffness and/or lower tensile strength compared to one or more ligaments which are arranged in or towards the center of the multi-level structure along the longitudinal dimension.

In the erected multi-level structure, the dimension perpendicular to the longitudinal and lateral dimension may be higher in the center of the multi-level structure along the longitudinal dimension compared to the areas being closer towards the lateral edges of the multi-level structure.

In the erected multi-level structure, the ligaments of the different levels may be aligned with each other, such that the position of the ligaments in one level coincide with the position of the ligaments in the one of more levels when viewed perpendicular to the longitudinal and lateral dimension. Alternatively, in the erected multi-level structure, the ligaments of the different levels may be staggered versus each other, such that the position of the ligaments in one level does not coincide with the position of the ligaments in the one of more levels when viewed perpendicular to the longitudinal and lateral dimension. Still alternatively, some ligaments may be aligned with each other while other ligaments are staggered versus each other.

BRIEF DESCRIPTION OF THE DRAWINGS

These and other features, aspects and advantages of the present invention will become better understood with regard to the following description, appended claims, and accompanying drawing where:

Fig. 1A is a side view (parallel to the lateral dimension) of a multi-level cell-forming structure- with ligaments in both levels having Z-like shape -, wherein the structure is in its initial flat configuration

Fig. 1B is a side view of the embodiment of Fig. 1A, wherein the structure is now in its erected configuration

Fig. 2A is a side view (parallel to the lateral dimension) of a multi-level cell-forming structure - with ligaments having Z-like shape -, wherein the structure is in its initial flat configuration and wherein the structure comprises a layer-to-layer stop aid

Fig. 2B is a side view (parallel to the lateral dimension) of the embodiment of Fig 2A, now in its erected configuration

Fig. 3A is a side view (parallel to the lateral dimension) of a multi-level cell-forming structure - with ligaments having Z-like shape -, wherein the structure is in its initial flat configuration and wherein the structure comprises a layer-to-ligament stop aid

Fig. 3B is a side view (parallel to the lateral dimension) of the embodiment of Fig 3A, now in its erected configuration

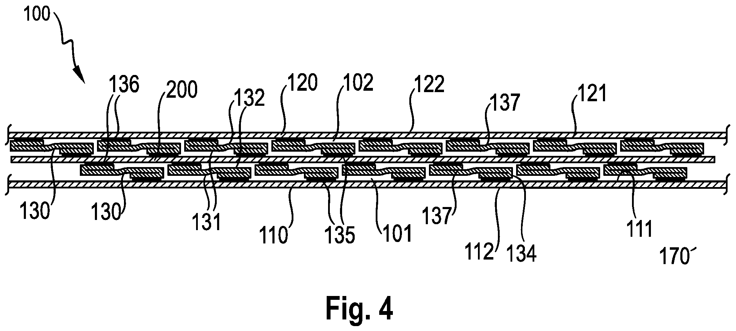

Fig. 4 is a side view of a multi-level cell-forming structure - with ligaments having Z-like shape and wherein the ligaments are folded when the multilevel structure is in its flat configuration

Fig. 5A is a side view of a multi-level cell-forming structure -with ligaments having C-like shape-, wherein the structure is in its initial flat configuration

Fig. 5B is a side view of the embodiment of Fig. 5A, wherein the structure is in its partly erected configuration

Fig. 5C is a side view of the embodiment of Fig. 5A, wherein the structure is in its maximum erected configuration

Fig. 6A is a side view of a multi-level cell-forming structure -with ligaments having Double-T-like shape-, wherein the structure is in its initial flat configuration

Fig. 6B is a side view of the embodiment of Fig. 6A, wherein the structure is in its partly erected configuration

Fig. 6C is a side view of the embodiment of Fig. 6A, wherein the structure is in its maximum erected configuration

Fig. 7A is a side view of a multi-level cell-forming structure -with ligaments having T-like shape-, wherein the structure is in its initial flat configuration

Fig. 7B is a side view of the embodiment of Fig. 7A, wherein the structure is in its partly erected configuration

Fig. 7C is a side view of the embodiment of Fig. 7A, wherein the structure is in its maximum erected configuration

Fig. 8A is a side view of a multi-level cell-forming structure - with ligaments of Z-like shape having inverse configuration, wherein the structure is in its initial flat configuration

Fig. 8B is a side view of the embodiment of Fig. 8A, wherein the structure is in its erected configuration

Fig. 9A is a side view of a multi-level cell-forming structure - with ligaments of C-like shape having inverse configuration, wherein the structure is in its initial flat configuration

Fig. 9B is a side view of the embodiment of Fig. 9A, wherein the structure is in its erected configuration

Fig. 10 is a side view of a multi-level cell-forming structure not comprised by the present invention, wherein the structure cannot be converted into an erected configuration

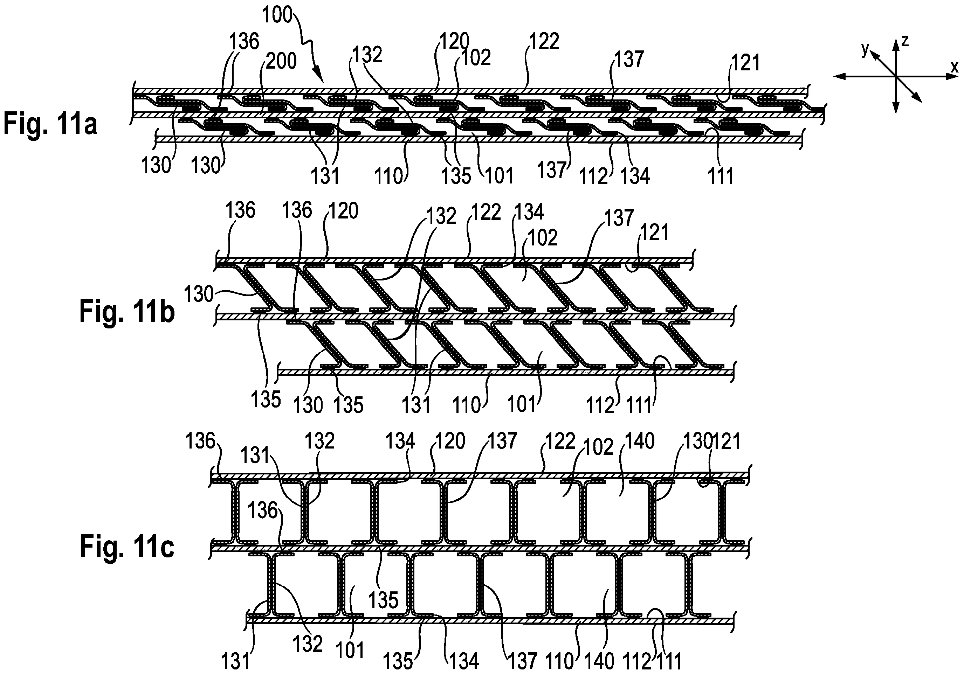

Fig. 11A is a side view of a multi-level cell-forming structure -with ligaments made of two-layered laminates and having Double-T-like shape-, wherein the structure is in its initial flat configuration

Fig. 11B is a side view of the embodiment of Fig. 11A, wherein the structure is in its partly erected configuration

Fig. 11C is a side view of the embodiment of Fig. 11A, wherein the structure is in its maximum erected configuration

Fig. 12A is a side view of a multi-level cell-forming structure -with ligaments compiled of separate pieces of material and having Double-T-like shape-, wherein the structure is in its initial flat configuration

Fig. 12B is a side view of the embodiment of Fig. 12A, wherein the structure is in its partly erected configuration

Fig. 12C is a side view of the embodiment of Fig. 12A, wherein the structure is in its maximum erected configuration

Fig. 13A is a side view of a multi-level cell-forming structure -with ligaments compiled of separate pieces of material and having Z-like shape-, wherein the structure is in its initial flat configuration

Fig. 13B is a side view of the embodiment of Fig. 13A, wherein the structure is in its partly erected configuration

Fig. 13C is a side view of the embodiment of Fig. 13A, wherein the structure is in its maximum erected configuration

Fig. 14A is a side view of a multi-level cell-forming structure -with ligaments having cut out areas and having Z-like shape-, wherein the structure is substantially in its initial flat configuration

Fig. 14B is a side view of the embodiment of Fig. 14A, wherein the structure is in its erected configuration

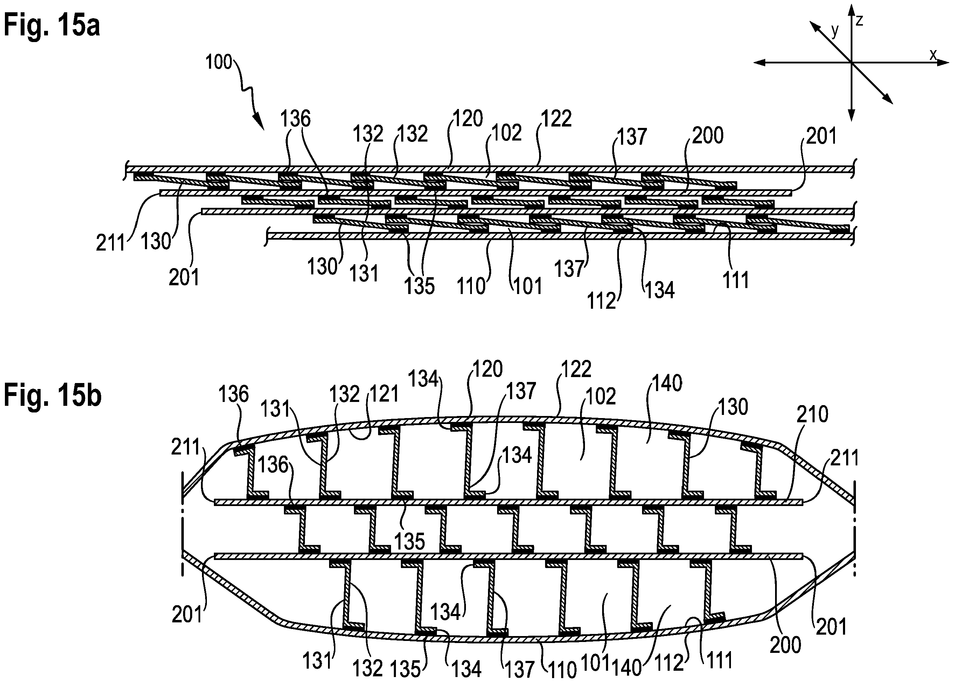

Fig. 15A is a side view of a multi-level cell-forming structure - with ligaments having Z-like shape- wherein the structure has two intermediate layers and three levels

Fig. 15B is a side view of the embodiment of Fig. 15A, wherein the structure is in its erected configuration

Fig. 16A is a side view of a multi-level cell-forming structure - with ligaments having Z-like shape- wherein the intermediate layer is interrupted and consists of two sections

Fig. 18B is a side view of the embodiment of Fig. 18A, wherein the structure is in its erected configuration

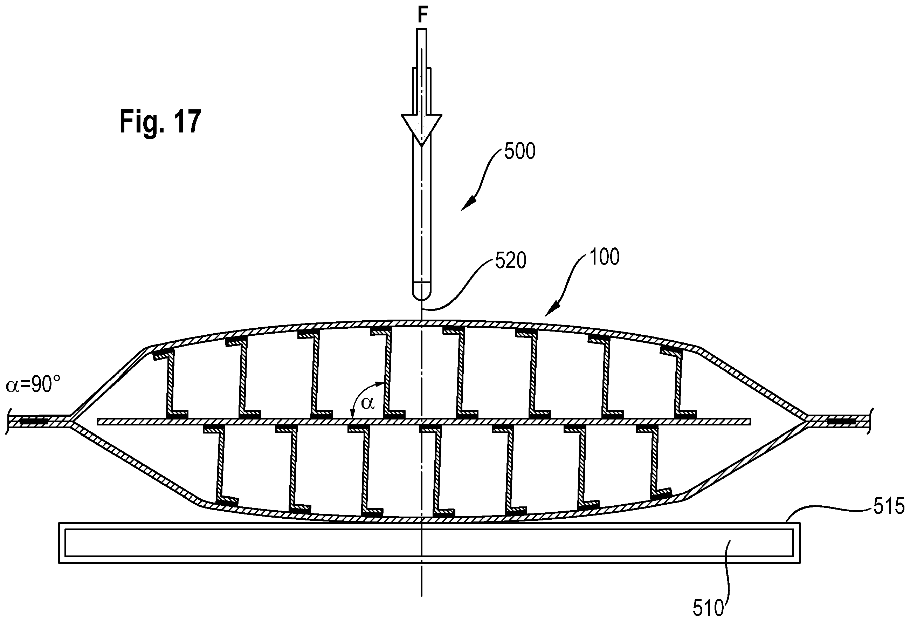

Fig. 17 is a schematic drawing of parts of the equipment used for the modulus test method

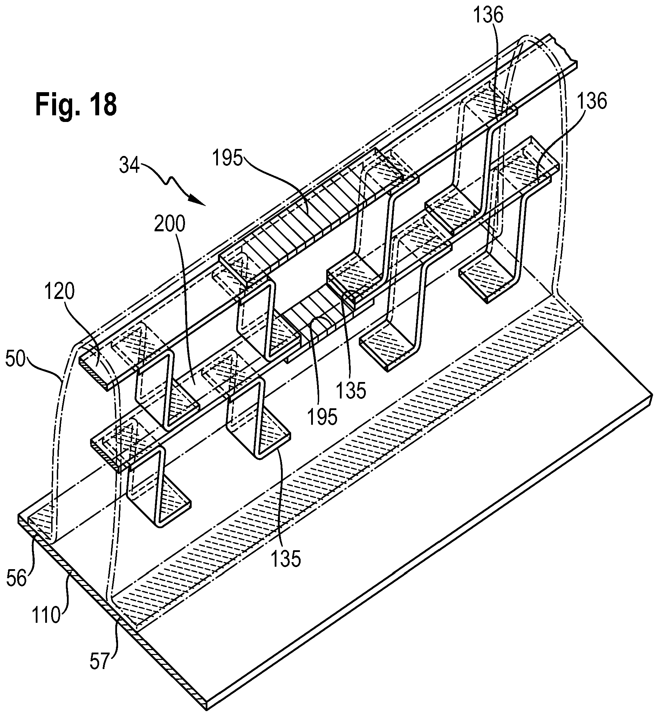

Fig. 18 is a schematic drawing of a barrier leg cuff of the present invention, wherein the structure is in its erected configuration

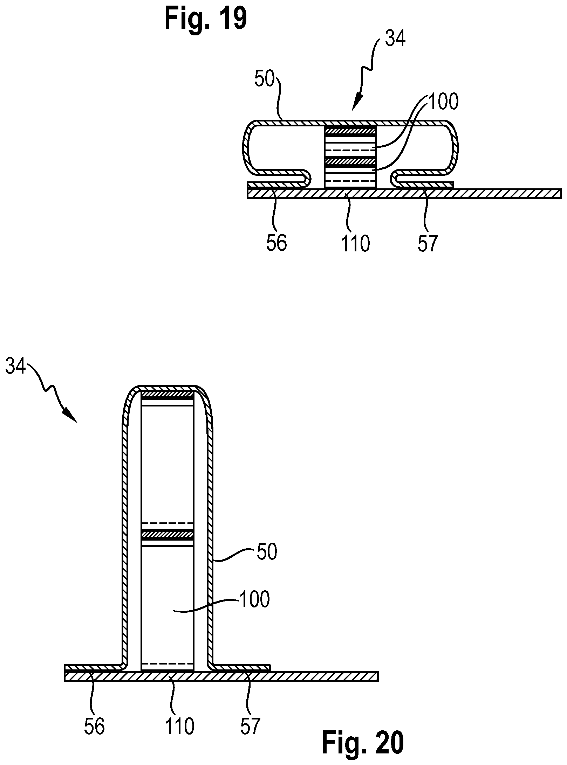

Fig. 19 is a front view (along the longitudinal dimension of the barrier leg cuff) of Fig. 18, wherein the structure is in its flat configuration (whereas Fig. 18 shows the erected configuration)

Fig. 20 is a front view (along the longitudinal dimension of the barrier leg cuff) of Fig. 18, wherein the structure is in its erected configuration

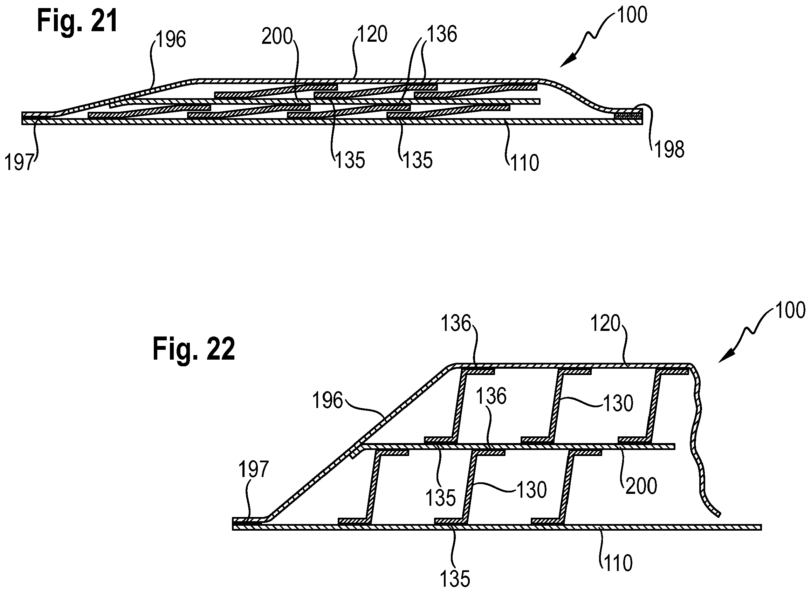

Fig. 21 is a schematic drawing of a structure comprising an elastic element, wherein the structure is in its flat configuration

Fig. 22 is a schematic drawing of the embodiment of Fig. 21, wherein the structure is in its erected configuration.

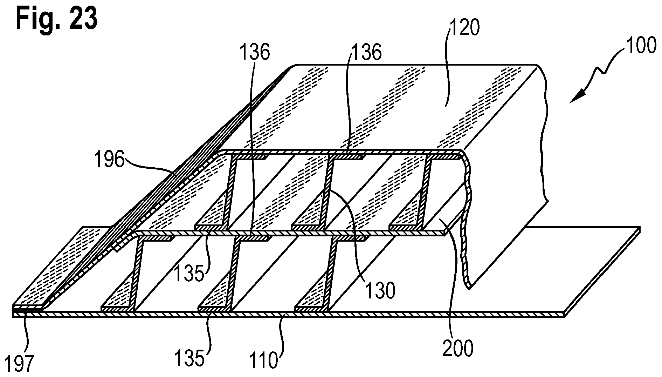

Fig. 23 is a spatial representation of the structure of Fig. 22

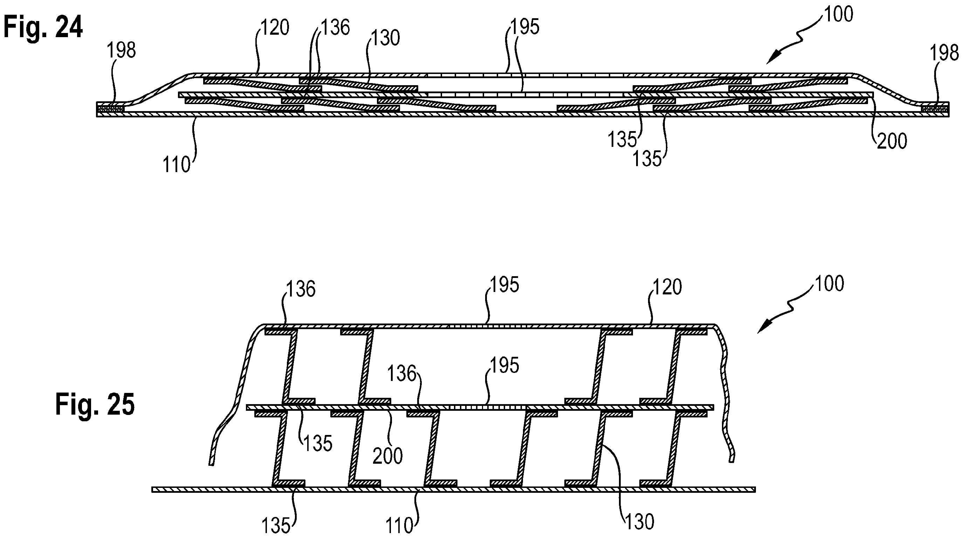

Fig. 24 is a schematic drawing of an alternative structure to the structure shown in Fig.21 and comprising an elastic element, wherein the structure is in its flat configuration

Fig. 25 is a schematic drawing of the embodiment of Fig. 24, wherein the structure is in its erected configuration.

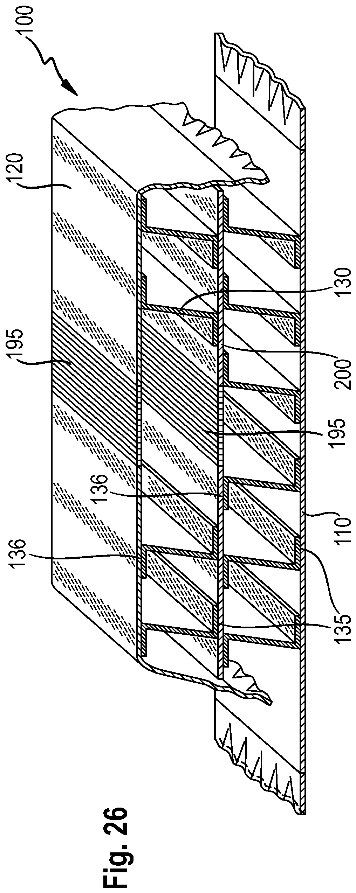

Fig. 26 is a spatial representation of the structure of Fig. 25

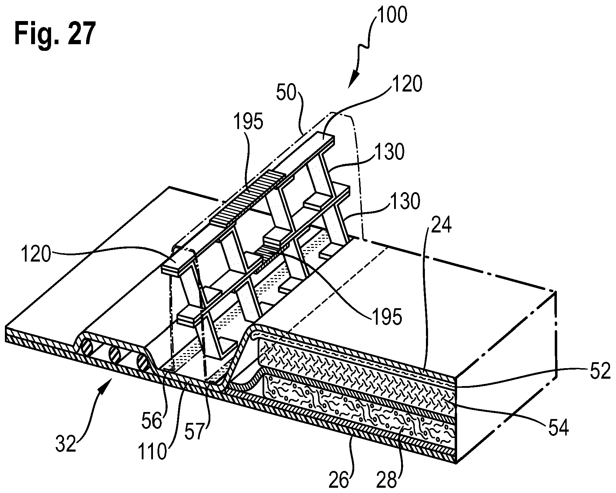

Fig. 27 is a schematic drawing of a section of an absorbent article comprising the barrier leg cuff of the present invention.

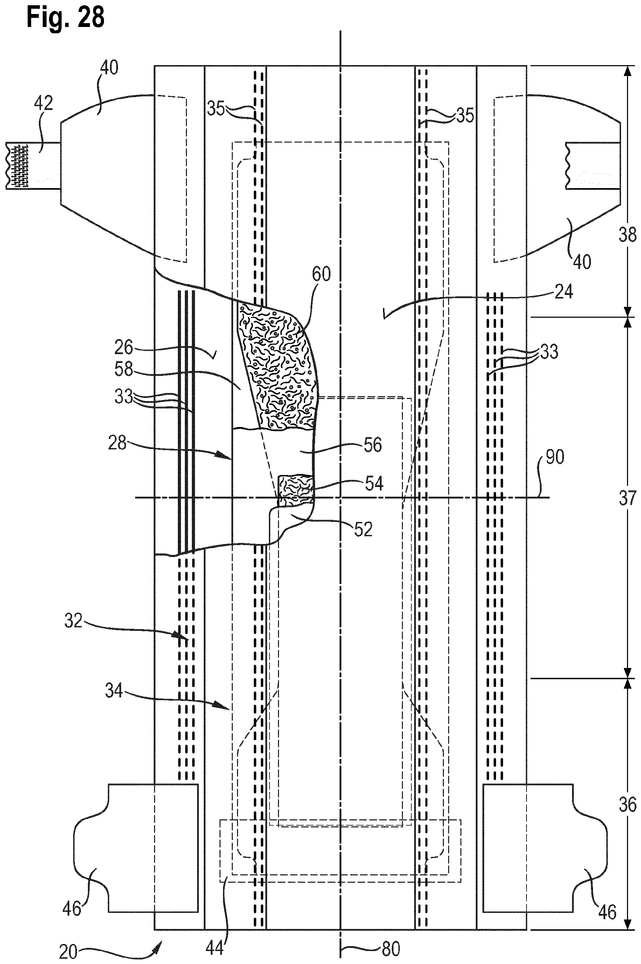

Fig. 28 is a top view of an exemplary absorbent article in the form of a diaper.

DETAILED DESCRIPTION OF THE INVENTION

Definitions

"Absorbent article" refers to devices that absorb and contain body exudates, and, more specifically, refers to devices that are placed against or in proximity to the body of the wearer to absorb and contain the various exudates discharged from the body. Absorbent articles may include diapers (baby diapers and diapers for adult incontinence), pants, inserts, feminine care absorbent articles such as sanitary napkins or pantiliners, and the like. As used herein, the term "exudates" includes, but is not limited to, urine, blood, vaginal discharges, sweat and fecal matter. Preferred absorbent articles of the present invention are disposable absorbent articles, more preferably disposable diapers and disposable pants.

"Disposable" is used in its ordinary sense to mean an article that is disposed or discarded after a limited number of usage over varying lengths of time, for example, less than 20 usages, less than 10 usages, less than 5 usages, or less than 2 usages. If the disposable absorbent article is a diaper, a pant, sanitary napkin, sanitary pad or wet wipe for personal hygiene use, the disposable absorbent article is most often intended to be disposed after single use.

"Diaper" and "pant" refers to an absorbent article generally worn by babies, infants and incontinent persons about the lower torso so as to encircle the waist and legs of the wearer and that is specifically adapted to receive and contain urinary and fecal waste. In a pant, as used herein, the longitudinal edges of the first and second waist region are attached to each other to a pre-form waist opening and leg openings. A pant is placed in position on the wearer by inserting the wearer's legs into the leg openings and sliding the pant absorbent article into position about the wearer's lower torso. A pant may be pre-formed by any suitable technique including, but not limited to, joining together portions of the absorbent article using refastenable and/or non-refastenable bonds (e.g., seam, weld, adhesive, cohesive bond, fastener, etc.). A pant may be preformed anywhere along the circumference of the article (e.g., side fastened, front waist fastened). In a diaper, the waist opening and leg openings are only formed when the diaper is applied onto a wearer by (releasable) attaching the longitudinal edges of the first and second waist region to each other on both sides by a suitable fastening system.

The term "film" as used herein refers to a substantially non-fibrous sheet-like material wherein the length and width of the material far exceed the thickness of the material. Typically, films have a thickness of about 0.5 mm or less. Films may be configured to be liquid impermeable and/or vapor permeable (i.e., breathable). Films may be made of polymeric, thermoplastic material, such as polyethylene, polypropylene or the like.

"Garment-facing surface" means the outermost portion of an element of a wearable absorbent article when the absorbent article is worn as intended. The opposing surface, or innermost portion, of the same element is referred to as the "wearer-facing surface." It is to be understood that the garment-facing surface and the wearer-facing surface of an element are relative to the wearer of the article with the garment-facing surface being furthest from the wearer and the wearer-facing surface being closest to the wearer. In the example of a typical disposable diaper, the portion of the outer cover that faces away from the wearer is the garment-facing surface while the opposing surface of the outer cover is the wearer-facing surface.

"Longitudinally inboard" and "longitudinally outboard" as used herein in relation to the absorbent article describes the position of a component or material relative to another component or material with respect to the longitudinal axis of the absorbent article. Longitudinally inboard means closer to the longitudinal axis whereas longitudinally outboard means further away from the longitudinal axis.

"Non-extensible" as used herein refers to a material which, upon release of a contractive force, which has provided by the stretched one or more elastic elements, elongates beyond its original length by less than 20 % if subjected to the following test:

A rectangular piece of the material having a width of 2.54 cm and a length of 25.4 cm is maintained in a vertical position by holding the piece along its upper 2.54 cm wide edge along its complete width. A force of 10 N is applied onto the opposite lower edge along the complete width of the material for 1 minute (at 25°C and 50% rel. humidity; samples should be preconditioned at these temperature and humidity conditions for 2 hours prior to testing). Immediately after one minute, the length of the piece is measured while the force is still applied and the degree of elongation is calculated by subtracting the initial length (25.4 cm) from the length measured after one minute.

If a material elongates beyond its original length by more than 20 % if subjected to the above described test, it is "extensible" as used herein.

"Highly non-extensible" as used herein refers to a material, which, upon release of a contractive force, which has provided by the stretched one or more elastic elements, elongates beyond its original length by less than 10% if subjected to the test described above for "non-extensible" material.

"Non-elastic" as used herein refers to a material which does not recover by more than 20% if subjected to the following test, which is to be carried out immediately subsequent to the test on "non-extensibility" set out above.

Immediately after the length of the rectangular piece of material has been measured while the 10N force is still applied, the force is removed and the piece is laid down flat on a table for 5 minutes (at 25°C and 50% rel. humidity) to be able to recover. Immediately after 5 minutes, the length of the piece is measured again and the degree of elongation is calculated by subtracting the initial length (25.4 cm) from the length after 5 minutes.

The elongation after one minute while the force has been applied (as measured with respect to "non-extensibility") is compared to the elongation after the piece has been laid down flat on a table for 5 minutes: If the elongation does not recover by more than 20%, the material is considered to be "non-elastic".

If a material recovers by more than 20%, the material is considered "elastic" as used herein.

"Highly non-elastic" as used herein refers to a material, which is either "non-extensible" or which does not recover by more than 10% if subjected to the test set out above for "non-elastic".

For use in the cell forming structures of the present invention, extensible, non-extensible, highly non-extensible, elastic, non-elastic and highly non-elastic relate to the dimension of the material, which, once the material has been incorporated into the structure, is parallel to the longitudinal dimension of the structure. Hence, the sample length of 25.4 cm for carrying out the tests described above corresponds to the longitudinal dimension of the cell forming structure once the material has been incorporated into the structure.

A "nonwoven web" is a manufactured web of directionally or randomly oriented fibers, consolidated and bonded together. The term does not include fabrics which are woven, knitted, or stitch-bonded with yarns or filaments. The fibers may be of natural or man-made origin and may be staple or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes such as meltblowing, spunbonding, solvent spinning, electrospinning, and carding. Nonwoven webs may be bonded by heat and/or pressure or may be adhesively bonded. Bonding may be limited to certain areas of the nonwoven web (point bonding, pattern bonding). Nonwoven webs may also be hydro-entangled or needle-punched. The basis weight of nonwoven fabrics is usually expressed in grams per square meter ($g/m^2$).

A "pantiliner" and a "sanitary napkin" generally have two end regions and a middle region (i.e. a crotch region). The pantiliner and the sanitary napkin has a body-facing surface and a garment facing surface. The size and shape of the absorbent structure positioned between the topsheet and the backsheet can be altered to meet absorbent capacity requirements, and to provide comfort to the wearer. The garment facing surface of the pantiliner and of the sanitary napkin can have thereon pressure sensitive adhesive for affixing to a wearer's undergarments. Typically, such adhesive is covered with a release strip which is removed before affixing to the undergarment. Pantiliners can also be provided with lateral extensions known commonly in the art as "flaps" or "wings" intended to extend over and cover the panty elastics in the crotch region of the user's undergarment. However, wings are normally not used with pantiliners but are more often used in sanitary napkins. Sanitary napkins and pantiliners of the present invention comprise barrier leg cuffs.

A "paper" refers to a wet-formed fibrous structure comprising cellulose fibers.

"Releasably attached", as used herein, refers to a temporary attachment. Temporary attachment can be achieved, e.g. by using an adhesive which allows for release of the attachment by use of moderate force, such as can be applied by a person without application of undue forces and leading to rupturing or substantial damaging (affecting proper and intended use) of the absorbent article. Releasably attachment can also be achieved by using an adhesive which provides strong attachment initially when the adhesive is applied, e.g. during manufacturing of the absorbent article, and wherein the adhesive forces decrease over time, e.g. by evaporation of certain solvents comprised by the adhesive during application of the adhesive or by degradation of certain ingredients comprised by the adhesive. Such adhesives may become brittle over time. It should be noted that though such releasable attachments may, under certain conditions, already lead to detachment prior to a consumer using the absorbent article (e.g. by removing it from a package prior to use), the releasable attachment will nevertheless be useful in the present invention, as long as the releasable attachment provides for intial attachment, e.g. during manufacture and packaging of the absorbent article. Unless specifically mentioned herein that an attachment/bond is meant to be releasable, all attachments/bonds described herein are permanent, i.e. they do not detach and open under conditions to which an absorbent article is normally subjected during its lifetime.

"Sheet-like foam", as used herein is a solid sheet that is formed by trapping pockets of gas. The solid foam may be closed-cell foam or open-cell foam. In closed-cell foam, the gas forms discrete pockets, each completely surrounded by the solid material. In open-cell foam, the gas pockets connect with each other. "Sheet-like" means that the length and width of the material far exceed the thickness of the material

"Transversally inboard" and "transversally outboard" as used herein in relation to the absorbent article describes the position of a component or material relative to another component or material with respect to the transversal axis of the absorbent article. Transversally inboard means closer to the transversal axis whereas transversally outboard means further away from the transversal axis.

The terms "upper", "above", "on top of", as used herein in respect of the absorbent article relates to a position on a wearer-facing surface. Likewise, the terms "lower", "below", "beneath", "underneath" and "under" as used herein in respect of the absorbent article relates to a position on a garment-facing surface.

Disposable absorbent articles

The structures of the present invention can find a wide variety of applications in absorbent articles, such as disposable absorbent articles.

A typical absorbent article is represented in Fig. 28 in the form of a diaper 20. However, the structure may be applied in other disposable absorbent articles, such as pants, pantiliners and sanitary napkins in the same manner and configuration as described herein below for a diaper.

In more details, Figure 28 is a plan view of an exemplary diaper 20, in a flat-out state, with portions of the diaper being cut-away to more clearly show the construction of the diaper 20. As said, this diaper 20 is shown for illustration purpose only as the structure of the present invention may be comprised in a wide variety of diapers or other absorbent articles.

As shown in Figure 28, the absorbent article, here a diaper, can comprise a liquid pervious topsheet 24, a liquid impervious backsheet 26, an absorbent core 28 which is preferably positioned between at least a portion of the topsheet 24 and the backsheet 26. The absorbent core 28 can absorb and contain liquid received by the absorbent article and may comprise absorbent materials 60, such as superabsorbent polymers and/or cellulose fibers, as well as other absorbent and non-absorbent materials commonly used in absorbent articles (e.g. thermoplastic adhesives immobilizing the superabsorbent polymer particles). The diaper 20 may also include optionally an acquisition system with an upper 52 and lower 54 acquisition layer.

The diaper also comprises barrier leg cuffs 34 and may further comprise elasticized leg cuffs 32. Moreover, the absorbent article may comprise a fastening system, such as an adhesive fastening system or a hook and loop fastening member, which can comprise tape tabs 42, such as adhesive tape tabs or tape tabs comprising hook elements, cooperating with a landing zone 44 (e.g. a nonwoven web providing loops in a hook and loop fastening system). The diaper 20 as shown in Figure 28 can be notionally divided in a first waist region 36, a second waist region 38 opposed to the first waist region 36 and a crotch region 37 located between the first waist region 36 and the second waist region 38. The longitudinal centerline 80 is the imaginary line separating the diaper along its length in two equal halves. The transversal centerline 90 is the imagery line perpendicular to the longitudinal line 80 in the plane of the flattened out diaper and going through the middle of the length of the diaper. The periphery of the diaper 20 is defined by the outer edges of the diaper 20. The longitudinal edges of the diaper may run generally parallel to the longitudinal centerline 80 of the diaper 20 and the end edges run between the longitudinal edges generally parallel to the transversal centerline 90 of the diaper 20. The crotch region, the first and the second waist region each constitutes 1/3 of the absorbent article along the longitudinal centerline.

Further, the diaper may comprise other elements, such as a back waist feature, which may be non-elastic or elastic, and a front waist feature, which may be non-elastic or elastic, a lotion applied onto the wearer-facing surface of the topsheet, back ears 40, and/or front ears 46. The front and/or back ears 40, 46 may be separate components attached to the diaper or may instead be continuous with portions of the topsheet and/or backsheet -and/or even portions of the absorbent core - such that these portions form all or a part of the front and/or back ears 40, 46. Also combinations of the aforementioned are possible, such that the front and/or back ears 40, 46 are formed by portions of the topsheet and/or backsheet while additional materials are attached to form the overall front and/or back ears 40, 46. The front and/or back ears may be elastic or non-elastic.

The topsheet 24, the backsheet 26, and the absorbent core 28 may be assembled in a variety of well known configurations, in particular by gluing or heat embossing. Exemplary diaper configurations are described generally in US3,860,003; US5,221,274; US5,554,145; US5,569,234; US5,580,411; and US6,004,306.

As the structure has a relatively low caliper when in its initial flat configuration, it does not significantly contribute to the volume and bulk of the absorbent article prior to use. Hence, the structures do not add significantly to the overall packaging and storage volume of the absorbent articles.

Barrier leg cuffs

The absorbent article comprises barrier leg cuff 34 disposed adjacent to each of the longitudinal edges of the absorbent article and extending substantially parallel to the longitudinal dimension of the absorbent article. Each barrier leg cuff 34 comprises a structure 100. The structure may comprise one or more elastic elements 195, 196, or, alternatively, the structure may be associated with one or more elastic elements. The proximate end 110 of the structure is permanently attached to the absorbent article, such as to the topsheet 24, to the backsheet 26 and/or to the (optional) barrier material 50. Alternatively, the proximate end of the structure may be formed by a portion of the topsheet, the backsheet and/or the (optional) barrier material.

The structure 100 is in a flat configuration when the absorbent article is flattened out, wherein the one or more elastic elements 195, 196 are stretched along the longitudinal dimension of the structure (which is coextensive with the longitudinal dimension of the barrier leg cuff). Upon contraction of the one or more elastic elements, the structure is able to convert into an erected, three-dimensional configuration. Contraction of the one or more elastic elements often also leads to contraction of the absorbent article as a whole, and hence, the absorbent article will no longer be flattened out, similar to contraction of elastic members comprised by conventional, known barrier leg cuffs. The erected structure spaces one end (the distal end) of the barrier leg cuff away from the topsheet and causes the barrier leg cuff to stand up. The proximate end of the erected structure remains permanently attached to the absorbent article, such as to the topsheet, to the backsheet and/or to a barrier material, which is described below in more detail (or the proximate end is formed by a portion of the topsheet, the backsheet and/or the (optional) barrier material).

The structure has a longitudinal dimension parallel to the longitudinal dimension of the barrier leg cuff, a lateral dimension perpendicular to its longitudinal dimension and a caliper perpendicular to the longitudinal and lateral dimension of the structure. The caliper increasing when the structure converts from its flat configuration into its erected three-dimensional configuration, thus causing the barrier leg cuff to stand up.

Different to conventional barrier leg cuffs, which are typically two-dimensional, flat cuffs, the barrier leg cuff of the present invention has a three-dimensional shape in its erected configuration. This enables the barrier leg cuff to withstand forces applied to it by a wearer in an improved manner. Moreover, the caliper of the erected structure can be relatively high while still providing a relatively stiff and stable barrier leg cuff. Moreover, the barrier leg cuff of the present invention can improve comfort for the wearer, as barrier leg cuff can adapt more smoothly to the skin of the wearer and the forces can be distributed over a larger surface area (of the wearer's skin): In conventional two-dimensional barrier leg cuffs, the body contact is typically achieved by a thin elastic strand. The three-dimensional shape of the structure enables a body contact over a larger area as the distal end of the barrier leg cuff is in contact with a larger area of wearer's body.

The increase in caliper of the erected structure versus the flat structure may be more than 5 mm, or more than 8 mm, or more than 10 mm, or more than 15 mm, or more than 20 mm. Also, the increase caliper of the erected structure versus the flat structure may be less than 80 mm, or less than 60 mm, or less than 50 mm, or less than 40 mm. Generally, the increase caliper will depend on the intended use, with absorbent articles intended to be worn by babies and small toddlers requiring a smaller caliper whereas absorbent articles to be worn by larger children or adults may require a higher caliper.

The lateral dimension of the structure can be from 3 mm to 50 mm, or from 3 mm to 40 mm, or from 4 mm to 30 mm, depending on the intended use of the absorbent article. For example, in absorbent articles, such as diapers or pants, which are intended for use by babies or small toddlers, the lateral dimension of the structure may be relatively small, such as from 3 mm to 10 mm, or from 3 mm to 8 mm, or from 3 mm to 5 mm. Thereby, the barrier leg cuff does not extent excessively wide towards the longitudinal axis of the absorbent article to avoid discomfort and an adverse effect on the ability of the absorbent article to properly absorb liquid. If the barrier leg cuff extends too much towards the longitudinal axis 80, it might more or less "block" the central area where liquid will typically be introduced into the article (e.g. the area around the so-called "pee-point").

However, if the absorbent article is to be worn by larger children or by adults, the width of the absorbent article in the crotch region will be larger and thus, the lateral dimension of the structure may be larger accordingly, such as from be from 5 mm to 50 mm, or from 5 mm to 40 mm, or from 5 mm to 30 mm, or from 10 mm to 40 mm.

The longitudinal dimension of the structure will likewise depend on the intended use of the absorbent article. Generally, the length of longitudinal dimension of the structure may be the same as the longitudinal dimension of the absorbent article. Alternatively, the length may be somewhat shorter than the longitudinal dimension of the absorbent article. For example, the longitudinal dimension of the structure may be from 50% to 95%, or from 50% to 90%, or from 60% to 80% of the longitudinal dimension of the absorbent article.

The structure of each barrier leg cuff may extend along the complete crotch region 37. The structure may extend into the first and second waist regions 36, 38. For example the structure of each barrier leg cuff may extend along at least 30%, or at least 50% of the first and second waist region.

The longitudinal and lateral dimensions are determined when the structure is in its initial flat configuration and are defined by the dimensions of the distal end of the structure (if the structure is a so-called multi-level cell-forming structure 100 as described below, the dimensions are defined by the dimensions of the second outermost layer 120).

Each barrier leg cuff 34 may further comprise a barrier material 50, which partially or completely enwraps the structure 100. The barrier material may, for example, be a nonwoven web or a film. The barrier material will typically be liquid impermeable and may be hydrophobic to avoid leakage of urine through the barrier leg cuff.

If the barrier leg cuff comprises a barrier material, it should be configured accordingly in order not to obstruct and hinder proper erection of the structure. For example, the barrier material may not be attached to the structure at all or it may only be attached to the structure at or adjacent to the area of the structure, where the structure is attached to the absorbent article (e.g. to the topsheet, and/or backsheet).

The one or more elastic elements may be comprised by the barrier material and be associated with (e.g. may be attached to) the structure via the barrier material. Alternatively, the one or more elastic elements may be comprised by the structure and be associated with (e.g. attached to) the barrier material. Still alternatively, the one or more elastic elements may be comprised by the structure, such as by the distal end of the structure, and the distal end of the structure is associated with (e.g. attached to) the barrier material.

If the barrier material is partly enwrapped around the structure, it may be folded loosely over the structure in its flat configuration and attached to the topsheet and/or backsheet at the proximal end of the barrier leg cuff, as is shown in Figures 18 to 20. If the barrier material is fully enwrapped around the structure, it may be folded loosely over the structure in its flat configuration and attached to the topsheet, the backsheet, to itself and/or to the proximate end of the structure (such as to the first outermost layer of the so-called "multi-level cell-forming structure" which is described in detail below).

By folding the barrier material loosely over/around the structure, the barrier material provides sufficient leeway to allow conversion of the structure from its flat configuration into its erected configuration. Alternatively, the barrier material may be made of extensible material, which can elongate upon erection of the structure, such as extensible nonwoven. The material may also be rendered extensible, i.e. by incrementally stretching the material (so-called "ring-rolling").

However, the barrier material may be non-elastic or highly non-elastic (e.g. in order to not apply tension on the erected structure).

However, instead of using a separate barrier material, the distal end of the structure may also be configured such that the lateral dimension of the structure is wider than the lateral dimension of the structure components between the distal end and the proximate end. That way, the material forming the distal end can be folded over towards the topsheet of the absorbent article and may be attached to the respective components of the absorbent article, such as to the topsheet, to the backsheet and/or to the material forming the proximate end of the structure along its lateral side edges. For example, if the structure is a multi-level cell-forming structure as described below, the second outermost layer may have a larger lateral dimension than the ligaments (and and the stop aids, if present), such that the second outermost layer may be attached to the topsheet, to the backsheet and/or to the first outermost layer of the multi-level cell-forming structure.

The non-elastic materials comprised by the structure have a bending stiffness of less than 500 mNm, preferably less than 400 mNm, more preferably less than 200 mNm.

Also, the non-elastic materials comprised by the structure have a tensile strength of less than 80 N/cm, preferably less than 50 N/cm, more preferably less than 40 N/cm.

The increase in caliper of the erected structure vs. the flat structure may be more than 5 mm, or more than 8 mm, or more than 10 mm, or more than 15 mm, or more than 20 mm, or more than 25 mm, or more than 30 mm, or more than 40 mm. Also, the increase in caliper of the erected structure may be less than 100 mm, or less than 80 mm, or less than 60 mm, or less than 50 mm vs. the flat structure. Generally, the increase in caliper will depend on the intended use, e.g. absorbent articles intended to be worn by babies and small toddlers ay require a smaller caliper whereas absorbent articles to be worn by larger children or adults may require higher caliper. Also, for sanitary napkins or pantiliners or other absorbent inserts to be worn by placing it in the wearer's undergarment, a smaller caliper may be sufficient versus the use in a diaper or pant.

The absorbent article of the present invention may comprise more than one barrier leg cuff disposed adjacent to each of the longitudinal edges of the absorbent article and extending substantially parallel to the longitudinal dimension of the absorbent article, such as one barrier leg cuff comprising a structure of the present invention in combination with one conventional, two-dimensional barrier leg cuff.

**Gasketing cuff**

The absorbent article may further comprise a gasketing cuff 32 along each of the longitudinal edges of the article. The gasketing cuff 32 is typically positioned longitudinally outboard of the barrier leg cuff. The gasketing cuff may comprise one or more elastic elements, such as elastic strands. Due to the one or more elastic elements 33, the gasketing cuff is made elastically contractible. The gasketing cuff may further comprise portions of the backsheet, of the topsheet and/or of a nonwoven gasketing material. The nonwoven gasketing material may be the same or similar to the barrier material. The nonwoven gasketing material may be coextensive and continuous with the barrier material. The one or more elastic elements may be comprised in between the portions of the backsheet, of the topsheet and/or of a nonwoven gasketing material.

**Multilevel cell forming structures**

Examples of structures suitable for use in barrier leg cuffs of the present invention are so-called "multilevel cell forming structures".

The name "cell forming structures" is due to the cells formed in each level between neighboring ligaments in the erected structure configuration, the cells being delimited by two neighboring ligaments and a layer forming the superjacent layer of the respective level and further delimited by a layer forming the subjacent layer of the respective level. These structures are initially relatively flat. When a force is applied along the longitudinal dimension (i.e. along the lengthwise extension) of the structure, the structure adopts an erected configuration. Thus, the structure increases in caliper. As used herein, the terms "caliper" and "thickness" are used interchangeably and refer to a direction perpendicular to the lateral and longitudinal dimension.

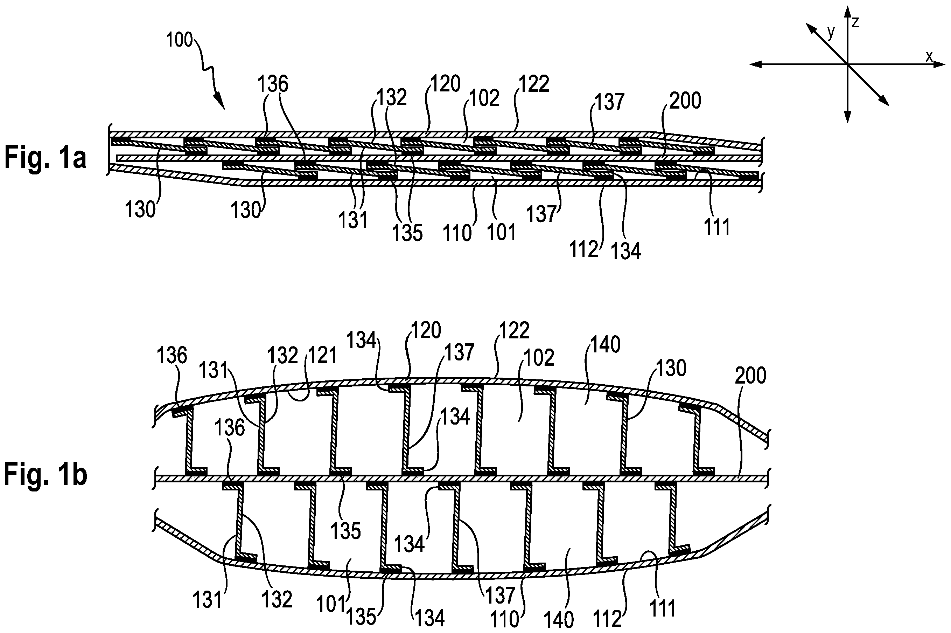

Figure 1A shows a multi-level cell-forming structure -with ligaments having Z-like shape- in its flat configuration whereas Figure 1B shows the structure in its erected configuration. The multilevel structure illustrated in Figs. 1A and 1B has two levels. The first level (101) is confined in a direction perpendicular to the longitudinal and lateral dimension by the first outermost layer (110) forming the subjacent layer of this level and by the first intermediate layer (200) (in this two-level structure there is however only one intermediate layer) forming the superjacent layer of this level. The second level (102) is confined in a direction perpendicular to the longitudinal and lateral dimension by the second outermost layer (120) forming the superjacent layer of this level and by the first intermediate layer (200) forming the subjacent layer of this level. Generally, for a multilevel structure, any given intermediate layer (200) forms the subjacent layer of one of the levels and also forms the superjacent layer of another level.

Generally, the structure (100) of the present invention comprises a first and a second outermost layer (110, 120).

Moreover, the structure (100) comprises at least a first intermediate layer (200) and may comprise further intermediate layers, such as a second intermediate layer (210), third intermediate layer and additional intermediate layers.

The first outermost layer (110) may be attached to the component of the absorbent article, which is below the structure. Hence, the first outermost layer may be attached to the topsheet, the backsheet, the barrier material of the barrier leg cuff or combinations thereof. Alternatively, the first outermost layer may be made of a portion of the component of the absorbent article, which is below the structure. Hence, the first outermost layer may be made of a portion of the topsheet, of a portion of the backsheet, or a portion of the barrier material. Also the first outermost layer (110) may be made non-extensible or highly non-extensible material. The first outermost layer may form the proximate end of the erected structure.

The second outermost layer (120) and/or the intermediate layer(s) (200, 210) may predominately be made of non-extensible or highly non-extensible material. However, the second outermost layer and/or the intermediate layer(s) (200, 210) may comprise the one or more elastic element of the structure, such that the second outermost layer and/or the intermediate layer(s) have elastic portions comprising the one or more elastic elements and non-elastic portions made of non-elastic material. Alternatively, the one or more elastic element may be directly or indirectly associated with the second outermost layer (120) (such that the one or more elastic elements may be comprised by the topsheet, or by the barrier material described above), in which case the second outermost layer may be made of non-elastic material. The second outermost layer may form the distal end of the erected structure.

The first and second outermost layers and the intermediate layer(s) may be made of nonwovens, film, paper, sheet-like foam, woven fabric, knitted fabric or combinations of these materials. Combinations of these materials may be laminates, e.g. a laminate of a film and a nonwoven. Generally, a laminate may consist of only two materials joined to each other in a face to face relationship and lying upon another but alternatively may also comprise more than two materials joined to each other in a face to face and lying upon another.

The one or more elastic elements which may be comprised by the second outermost layer may be elastic film, elastic nonwoven, elastic scrim or elastic strands.

The first and second outermost layer may be made of the same material (apart from the one or more elastic elements if they are comprised by the second outermost layer). Alternatively, the first outermost layer may be made of material which is different from the material of the second outermost layer.

Generally, the first and second outermost layer and the intermediate layer(s) may be made of separate sheets of material.

The materials of the first outermost layer, the (non-elastic) material of the second outermost layer and the (non-elastic) material of the intermediate layer(s) may be chosen such that the first and second outermost layers and/or the intermediate layer(s) have the same basis weight, tensile strength, bending stiffness, and/or breathability. Alternatively, the first and second outermost layer, and the intermediate layer(s) may differ from each other in one or more properties, such as basis weight, tensile strength, bending stiffness, and/or breathability.

The basis weight of the first outermost layer, the second outermost layer and the intermediate layer(s) may be at least 2 g/m², or at least 3 g/m², or at least 5 g/m²; or at least 8 g/m², or at least 10 g/m², or at least 12 g/m², and the basis weight may further be not more than 500 g/m², or not more than 200 g/m², or not more than 100 g/m², or not more than 50 g/m², or not more than 30 g/m². If the second outermost layer and/or the intermediate layer(s) comprise(s) the one or more elastic elements, the basis weight of the second outermost layer and of the intermediate layer(s), as used herein, refers to the non-elastic material of the second outermost layer as well as to the one or more elastic elements.

The tensile strength of the first and second outermost layers and of the intermediate layer(s) may be at least 3 N/cm, or at least 4 N/cm, or at least 5 N/cm. The tensile strength may be less than 100 N/cm, or less than 80 N/cm, or less than 50 N/cm, or less than 30 N/cm, or less than 20 N/cm.

The bending stiffness of the first and second outermost layer and the bending stiffness of the intermediate layer(s) may be at least 0.1 mNm, or at least 0.2 mNm, or at least 0.3 mNm. The bending stiffness may be less than 200 mNm, or less than 150 mNm, or less than 100 mNm, or less than 50 mNm, or less than 10 mNm, or less than 5 mNm. If the second outermost layer and/or the intermediate layer(s) comprise(s) the one or more elastic elements, the bending stiffness of the second outermost layer refers to the non-elastic materials only.

Generally, the higher the tensile strength and the bending stiffness of the first and second outermost layer and of the intermediate layer(s), the more rigid, but also the more stable the overall structure will become. Hence, the choice of tensile strength and bending stiffness for the first and second outermost layer and for the intermediate layer(s) depends on the application of the structure, balancing overall softness, drape and conformability requirements with overall stability and robustness. The bending stiffness of the first and second outermost layer may be chosen such as to ensure that the contracting forces of the one or more elastic elements do not result in excessive buckling of the structure, so that erection of the structure is not adversely impacted.

The respective superjacent layer and subjacent layer of a level within the structure are connected to each other via ligaments (130).

In the structure (100), each of the first and second outermost layers (110, 120) have an inner (111, 121) and an outer surface (112, 122) wherein the inner surfaces (111, 121) face towards the ligaments (130). Each of the first and second outermost layer (110, 120) and each intermediate layer (200, 210) in the structure (100) has a longitudinal dimension which is parallel to the longitudinal dimension of the structure (100) and which is confined by two spaced apart lateral edges. Each of the first and second outermost layers (110, 120) and each intermediate layer (200, 210) also has a lateral dimension parallel with the lateral dimension of the structure and confined by two spaced apart longitudinal edges. The first and second outermost layer (110, 120) and the intermediate layer(s) (200, 210) in the structure (100) at least partly overlap each other.

Each level of the structure comprises one or more than one ligament (130). Each ligament (130) is provided between the superjacent layer and the subjacent layer of the respective level. Each ligament (130) has a longitudinal dimension confined by two spaced apart lateral edges (134) and a lateral dimension confined by two spaced apart longitudinal edges.

In Figure 1, a coordinate system is shown with X-, Y- and Z-directions. The longitudinal dimension of the overall structure (100) and of the first and second outermost layer (110, 120) and the intermediate layer(s) (200, 210) extends along the longitudinal direction X of the coordinate system. The longitudinal dimension of the ligaments (130) in the structure's initial flat configuration may substantially extend along the longitudinal direction X of the illustrated coordinate system.

Likewise, the lateral dimension of the overall structure (100) and of the first and second outermost layer (110, 120) and the intermediate layer(s) (200, 210) extends along the lateral direction Y of the coordinate system. The lateral dimension of the ligaments (130) in the structure's initial flat configuration may substantially extend along the lateral direction Y of the illustrated coordinate system.

The caliper of the structure (100) extends along the Z direction of the coordinate system.

Each ligament (130) is attached in a first ligament attachment region (135) at or adjacent to one of the ligament's lateral edges (134) to the subjacent layer of the respective level (i.e. the level by which the respective ligament is comprised). The ligament is attached to that surface of the subjacent layer which faces towards the superjacent layer of the respective level. Each ligament (130) is also attached in a second ligament attachment region (136) at or adjacent to other the ligament's lateral edges (134) to the superjacent layer of the respective level. The ligament is attached to that surface of the superjacent layer which faces towards the subjacent layer of the respective level. Due to this attachment, the ligaments (130) will generally adopt a C-like, Z-like, T-like, or Double-T-like shape when the structure is in its erected configuration.

Each ligament has a first surface (131) and a second surface (132). When the ligament adopts a Z-like shape when the structure is in its erected configuration, the ligament (130) is attached to the subjacent layer in the first ligament attachment region (135) with the ligament's first surface (131) at or adjacent to one of its lateral ligament edges (134) and is further attached to the superjacent layer in the second ligament attachment region (136) with the ligament's second surface (132) at or adjacent to its other lateral ligament edge (134).

Moreover, for Z-like shapes (shown, e.g. in Figs. 1A and 1B), the ligament's (130) first surface (131) may not be facing towards the superjacent layer when the structure (100) is in its initial flat configuration, and the ligament's second surface (132) may not be facing towards the subjacent layer when the structure (100) is in its initial flat configuration. With such embodiments, it is possible to obtain structures with no folds and hinges (or very few folds and hinges, e.g. when the ligaments have different longitudinal dimension in their free intermediate portion (137), see below). Hence, these structures will generally exhibit a lower tendency to partly erect in the absence of an applied force along the longitudinal dimension and will consequently remain in their initial flat configuration. Also, such structures will generally exhibit a greater tendency to return to their initial flat configuration when the applied force is released.

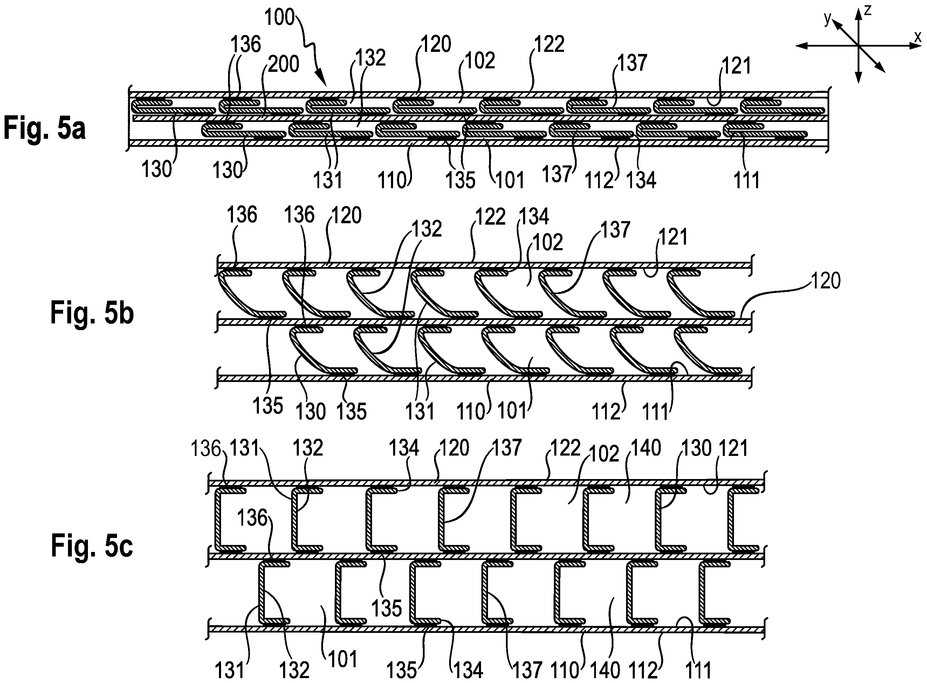

Alternatively, when the ligament adopts a C-like shape when the structure is in its erected configuration (exemplified in Figs. 5A to 5C), the ligament (130) is attached to the subjacent layer in the first ligament attachment region (135) with the ligament's first surface (131) at or adjacent to one of its lateral ligament edges (134) and is further attached to the superjacent layer in the second ligament attachment region (136) with the ligament's first surface (131) at or adjacent to its other lateral ligament edge (134).

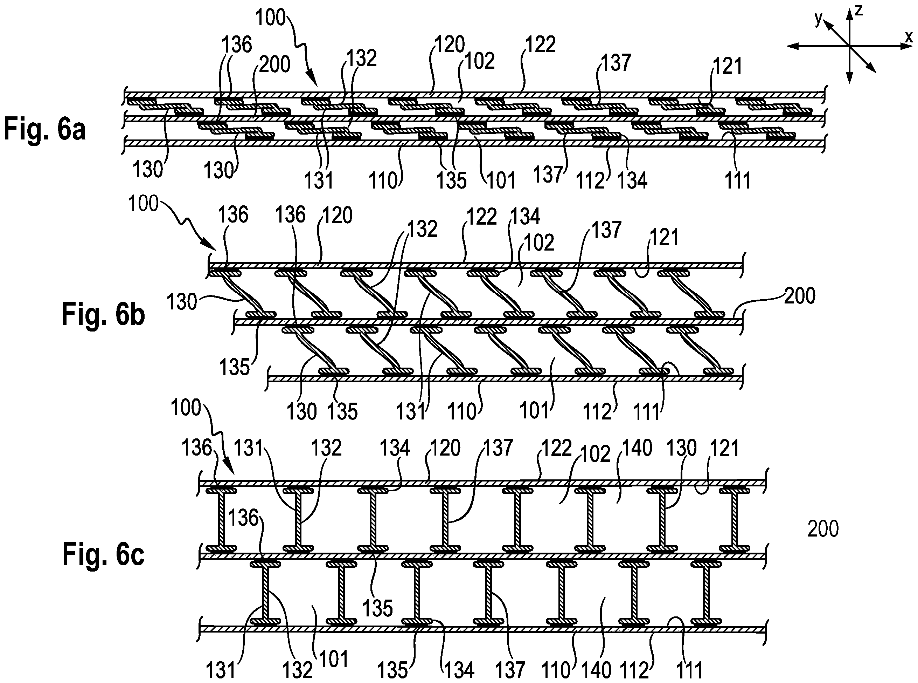

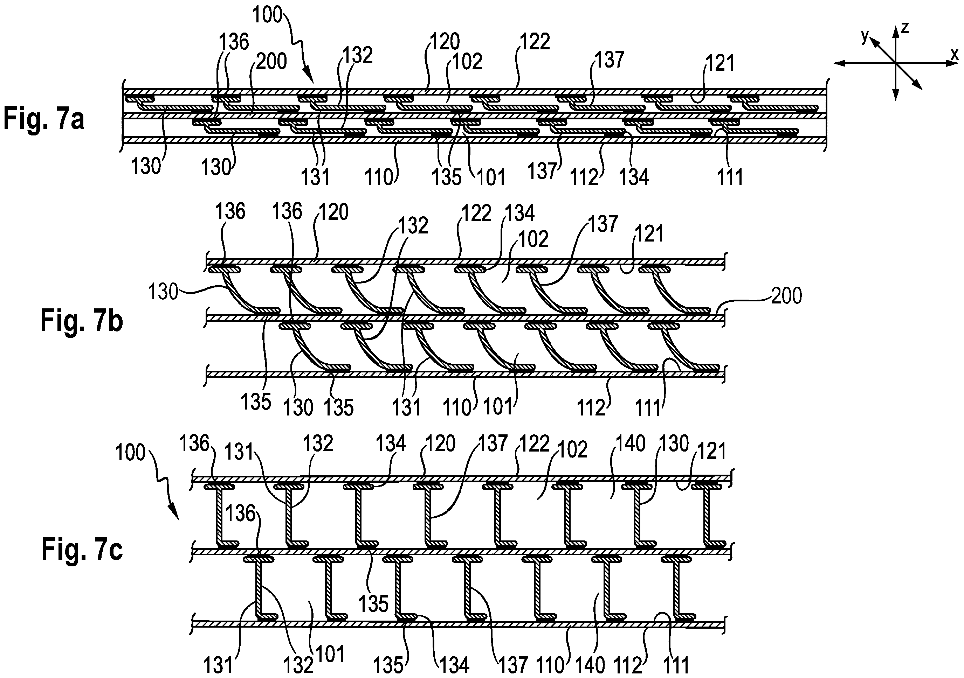

Still alternatively, one portion of the ligament at or adjacent to one of its lateral ligament edges may be attached to the subjacent layer such as to form a T-like (exemplified in Figs. 7A to 7C), or Double-T-like shape (exemplified in Figs. 6A to 6C). To facilitate such attachment, the portion of the ligament adjacent to a first lateral ligament edge has to comprise at least two ligament layers and the two ligament layers are not attached to each other in the portion of the ligament which is attached to the first outermost layer. Thereby, the ligament can be split up and unfolded such that both ligament layers can be respectively attached to the subjacent layer in the first ligament attachment region (135) to form a T-like shape when the structure is in its erected configuration.

For embodiments where a ligament adopts a T-like shape, the portion of the ligament at or adjacent to the second lateral ligament edge is attached to the superjacent layer with either its first or second surface to form the second ligament attachment region (136). If the ligament is also made of a laminate in the second ligament attachment region (136), the ligament layers are not split up in this area (i.e. only the portion adjacent to the first lateral ligament edge forms a T-like shape).

When the ligament adopts a Double-T-like shape when the structure is in its erected configuration, the portion of the ligament at or adjacent to the second lateral ligament edge is attached to the superjacent layer similar to the manner in which the portion at or adjacent to the first lateral ligament edge is attached to the subjacent layer, i.e. the ligament laminate is split up and unfolded such that two ligament layers can be respectively attached to the superjacent layer to form the second ligament attachment region (136).

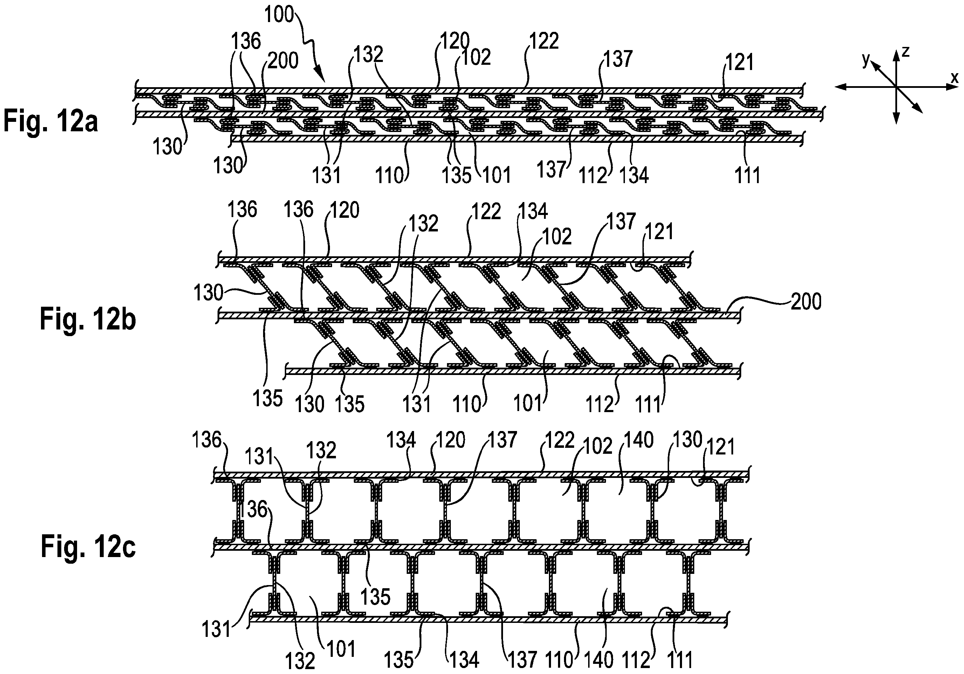

Splitting up and unfolding the ligament layers to form the respective first and second ligament attachment regions (135, 136) is exemplified in Figures 12A through 12C (showing ligaments with Double-T-like shape)

In a given structure, all ligaments may adopt a Z-like shape when the structure is in its erected configuration. Alternatively, all ligaments may adopt a C-like shape, all ligaments may adopt a T-like shape or all ligaments may adopt a Double-T-like shape when the structure is in its erected configuration. In a further alternative, a given structure may have a combination of ligaments selected from the group consisting of: ligaments adopting a Z-like shape, C-like shape, T-like shape and Double-T-like shapes.

In a given structure, ligaments adopting a Z-like shape when the structure is in its erected configuration may all adopt the same orientation (see e.g. Figs. 1A and 1B). That means, in such embodiments none of the ligaments adopting a Z-like shape has an inverse configuration of another ligament adopting a Z-like shape.

Likewise, in a given structure, ligaments adopting a C-like shape when the structure is in its erected configuration may all adopt the same orientation (see e.g. Figs. 5A to 5C).. That means, in such embodiments none of the ligaments adopting a C-like shape has an inverse configuration of another ligament adopting a C-like shape.

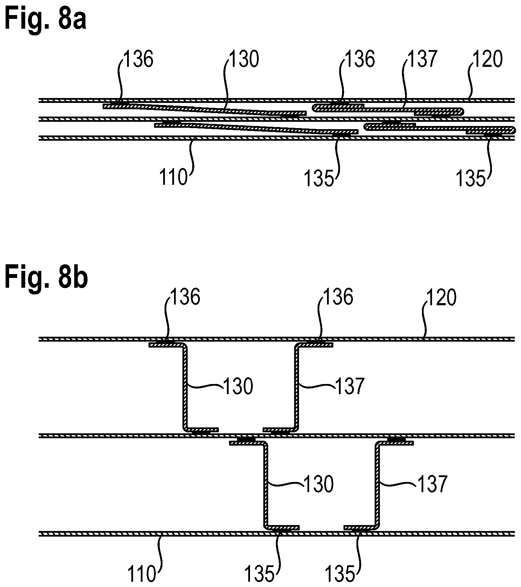

However, it is also possible to facilitate the structure such that that one or more ligaments adopt a Z-like shape which has an inverse configuration of one or more other ligaments with Z-like shape. An example of such structure is illustrated in Fig. 8A (flat configuration) and Fig. 8B (erected configuration).

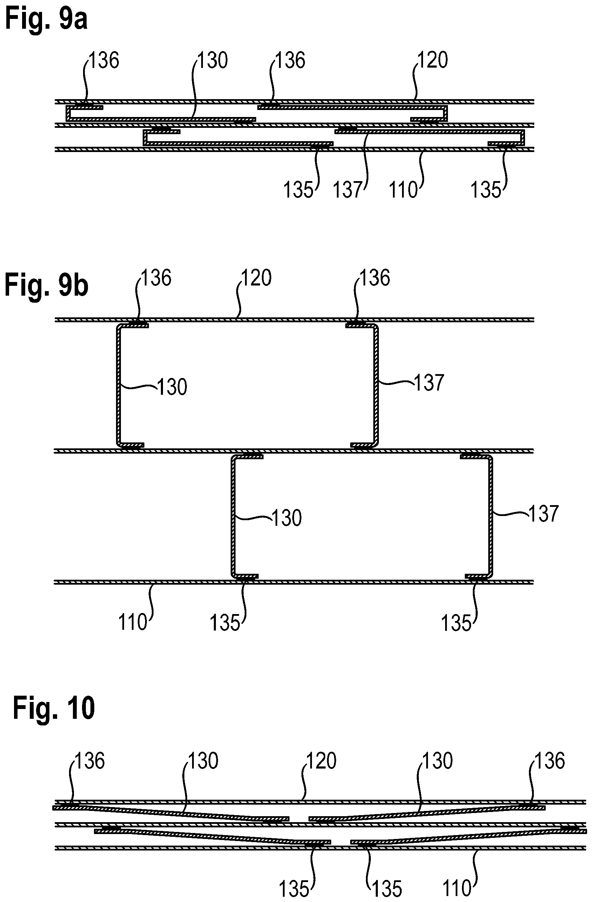

Similarly, a structure with one or more ligaments adopting a C-like shape which has an inverse configuration of one or more other ligaments with C-like shape is exemplified in Fig. 9A (flat configuration) and Fig.9B (erected configuration).

Such inverse configurations are feasible as long as the structure can be readily converted from its flat configuration to its erected configuration. An example of a structure having Z-like ligaments with inverse configuration which can only be converted from its flat to erected configuration when the one or more elastic elements are provided in the superadjecent layer (120) between the two neighboring ligament attachment regions (136) shown in Fig. 10. If no elastic element(s) are provided between the two neighboring ligaments, the ligaments on the right side of the Figure will "block" the ligaments on the left side of the Figure from being erected upon release of a contractive force, which has provided by the stretched one or more elastic elements along the longitudinal direction (and vice versa, the ligaments on the left side of the Figure "block" the ligaments on the left side of the Figure from being erected). Generally, the ligaments shown on the right side (or left side) of the Figure also cannot serve as a stop aid (explained below in detail) as no leeway is provided.

Generally, ligaments in a given structure have to be configured and attached accordingly such that the structure is able to be converted from an initial flat configuration into an erected configuration whereby the ligaments convert from an initial flat configuration into an erected configuration.

The longitudinal dimension of each ligament (130) between the first and second ligament attachment regions (135, 136) remains unattached to the subjacent and superjacent layer or is releasable attached to the subjacent and/or superjacent layer and/or to their neighboring ligament(s). This unattached or releasable attached portion is referred to as the "free intermediate portion" (137) of the ligament (130). "Releasable attachment of ligaments" means a temporary attachment to the subjacent and/or superjacent layer and/or to the neighboring ligament(s) in a way, that the bond strength is sufficiently weak to allow easy detachment from the subjacent and/or superjacent layer and/or the neighboring ligament(s) upon initial contraction of the structure (100) along the longitudinal dimension without rupturing or otherwise substantially damaging the ligaments (130) and/or the subjacent and/or superjacent layer and without substantially hindering the conversion of the structure from its initial flat configuration into its erected configuration. Such releasable attachments may help to maintain the structure in its initial flat configuration, e.g. during manufacturing processes when the structure is incorporated into an article.

When the structure is in its initial flat configuration, the first surface (131) of a ligament's free intermediate portion (137) faces towards the subjacent layer (110) and the second surface (132) of a ligament's free intermediate portion (137) faces towards the superjacent layer. When the structure (100) is converted into its erected configuration, the first surface (131) of a ligament's (130) free intermediate portion (137) faces towards the second surface (132) of its neighboring ligament (130). This is irrespective as to whether the ligament has been attached to adopt a Z-like, C-like, T-like or Double-T-like shape. Unless expressly mentioned herein, neighboring ligaments refers to ligaments which are neighboring along the longitudinal dimension.

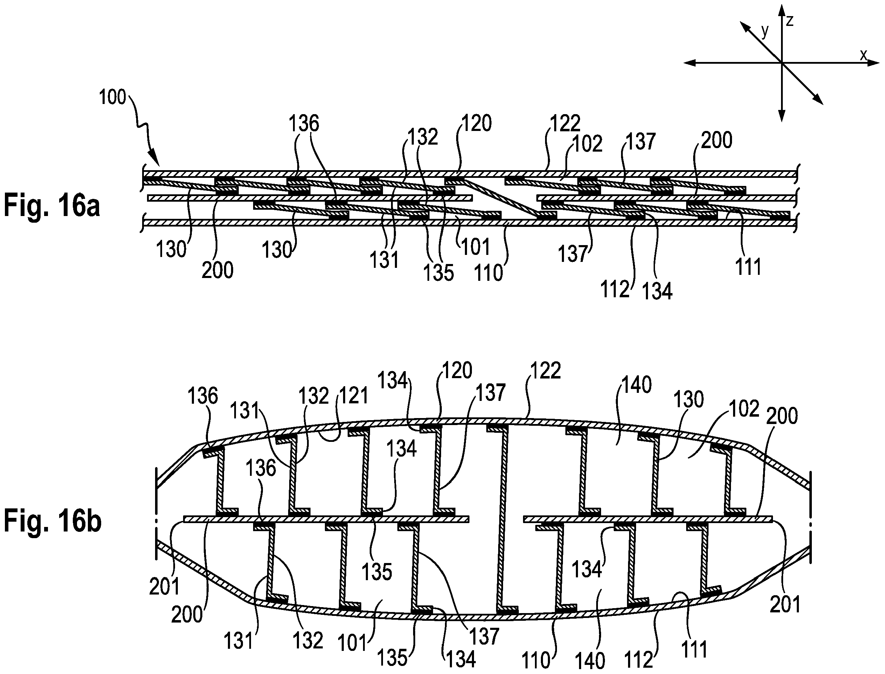

The structure may comprise one or more additional ligaments extending over more than one level across the caliper of the structure. For such ligaments, the ligament may be attached at or adjacent to one of its lateral ligament edges to the subjacent layer in a first ligament attachment area and the ligament may be further attached at or adjacent to its other lateral ligament edges to a layer above the subjacent layer in a second ligament attachment area. Such ligaments may be present if at least one intermediate layer is discontinuous across the longitudinal structure dimension and/or if at least one intermediate layer is not directly attached to another intermediate layer which neighbors the respective intermediate layer in the caliper direction (i.e. the neighboring intermediate layers are not attached to each other in the areas longitudinally outboard of the areas where the ligaments of the respective levels are provided). An example of such ligament is illustrated in Figs. 16A and 16B. Generally, for such ligaments extending over more than one level across the caliper of the structure, the same conditions and considerations apply with regard to the configuration of the attachment and ligament shape, as is set out above for the ligaments being comprised by only one level of the structure.

The ligaments (130) may be attached in their first and second ligament attachment region by any means known in the art, such as by use of adhesive, by thermal bonding, by mechanical bonding (such as pressure bonding), by ultrasonic bonding, or by combinations thereof. The attachment of the ligaments to the subjacent and superjacent layer is permanent, i.e. the attachment should not be releasable by forces which can typically be expected during use of the structure.

One, more than one or all of the intermediate layer(s) (200, 210) may not be attached to the first and/or second outermost layer (110, 120) other than indirectly via one or more ligaments and optionally indirectly via one or more stop aids (described below). Examples are shown in Figs. 1A and 1B. In such embodiments, the structure may comprise more than one elastic element and the intermediate layer(s) which are not directly attached to the first and/or second layer may comprise some of the elastic elements. These elastic elements may be comprised between adjacent ligament attachment regions.

One or more intermediate layers may also be discontinuous across the longitudinal dimension of the intermediate layer, such that the one or more intermediate layers comprise at least a first and a second section. An example of such a structure is shown in Figs. 16A and 16B, wherein the first intermediate layer (200) has a first and a second sub-section. In structures with one or more discontinuous intermediate layers, at least one ligament will be attached to each of the intermediate layer portions with one of its first or second ligament attachment regions.

Structures (100) as described supra are able to adopt an initial flat configuration when no external forces are applied. Upon release of a contractive force, which has provided by the stretched one or more elastic elements along the longitudinal dimension, the structure will increase in caliper, i.e. in the direction perpendicular to the longitudinal and lateral dimension.

The force along the longitudinal dimension may be applied by appropriately positioning the one or more elastic elements. For example, one or more elastic element (196) may be comprised by and/or attached to the second outermost layer longitudinally outboard of the second ligament attachment region (136) which is closest to one of the lateral edges of the level for which the second outermost layer constitutes the superjacent layer. The second outermost layer may be permanently attached (197) to the first outermost layer (110), to the topsheet, to the backsheet and/or to the barrier material longitudinally outboard of the one or more elastic elements or may be permanently attached to the first outermost layer (110), to the topsheet, to the backsheet and/or to the barrier material with the elastic material comprised by the second outermost layer. The one or more elastic elements may be comprised by or associated with the second outermost layer only in the area longitudinally outboard of the second ligament attachment region (136) of the level to which the second outermost layer forms the superjacent layer and which is closest to one of the lateral edges of the structure. Alternatively, the one or more elastic elements may be continuously comprised by or be associated with the second outermost layer from the area longitudinally outboard of the second ligament attachment region (136) of the level to which the second outermost layer forms the superjacent layer and which is closest to one of the lateral edges of the structure into the area between neighboring second ligament attachment regions (136) (such that in the erected structure, the one or more elastic element is also comprised by or associated with the second outermost layer between neighboring ligaments). Still alternatively, the one or more elastic elements may be comprised by or be associated with the second outermost layer continuously along the complete longitudinal dimension of the second outermost layer. The second outermost layer may also be completely formed by the elastic element.

When the structure is in its flat configuration, the one or more elastic elements are in a stretched condition. On the respective other side of the one or more elastic elements along the longitudinal dimension of the structure, e.g. at or adjacent to the respective other lateral edge of the structure, the second outermost layer (120) may be releasably attached (198) to the first outermost layer (110), to the topsheet, to the backsheet and/or to the barrier material. If the one or more elastic element extends over the complete longitudinal dimension of the second outermost layer, the releasable attachment (198) may also encompass the one or more elastic element, i.e. the releasable attachment is between the one or more elastic element of the second outermost layer and the first outermost layer (110), the topsheet, the backsheet and/or the barrier material.

The one or more elastic element may take the form or one or more elastic strands comprised by the second outermost layer. Alternatively, the one or more elastic elements may be sheet-like materials, such as elastic film or elastic nonwoven, which is comprised by the second outermost layer.

Upon release of the releasable attachment, the one or more elastic element contracts, thus applying a force along the longitudinal dimension of the structure. Thereby, the free intermediate portion of the ligaments lifts upward away from the first outermost layer (and away from the second outermost layer), thus lifting the second outermost layer upwardly and converting the structure into its erected configuration. An example of such a structure is shown in Figures 18-20.

Alternatively or in addition to providing or associating the one or more elastic element in the second outermost layer and/or attaching one or more elastic element to the second outermost layer longitudinally outboard of the ligament which is comprised by the level to which the second outermost layer forms the superjacent layer and which is closest to one of the lateral edges of the structure, one or more elastic element (195) may be comprised by the second outermost layer (120) in an area between two neighboring ligaments of the level to which the second outermost layer forms the superjacent layer (i.e. between neighboring second ligament attachment regions) and may not be comprised by or associated with the second outermost layer in the areas longitudinally outboard of the second ligament attachment region (136) which is closest to one of the lateral edges of the structure. Likewise to the above, these one or more elastic elements (195) are stretched when the structure is in its flat configuration. Also similar to the above, the structure is maintained in its initial flat configuration due to a releasable attachment (198) of the second outermost layer (120) to the first outermost layer (110), to the topsheet, to the backsheet and/or to the barrier material. However, compared to the configuration described in the previous paragraph, here the second outermost layer is releasably attached to the first outermost layer (110), to the topsheet, to the backsheet and/or to the barrier material on both sides outside of the one or more elastic elements (viewed along the longitudinal dimension of the structure). There may hence not be a permanent attachment of the second outermost layer to the first outermost layer (110), to the topsheet, to the backsheet and/or to the barrier material. Upon release of these releasable attachments (198), the one or more elastic element (195) comprised by the second outermost layer (120) between two neighboring ligaments (comprised by the level to which the second outermost layer forms the superjacent layer) contracts, thus applying a force along the longitudinal dimension of the structure.

Other than in the above embodiment (having a permanent attachment on one side and a releasable attachment on the other side of the one or more elastic elements), where the force is applied in the same direction along the longitudinal dimension of the structure along the complete structure, here the forces are applied in opposite directions along the longitudinal dimension of the structure. I.e. for the areas of the structure on one side (along the longitudinal dimension) of the one or more elastic elements the force is applied towards the one or more elastic element and for the areas of the structure on the respective other side (along the longitudinal dimension) of the one or more elastic elements, the force is applied towards the one or more elastic element and hence, in the opposite direction vs. the area on the other side of the one or more elastic elements. Examples for such structures are shown in Figures 21 through 26.

It is possible to provide one or more elastic elements in the second outermost layer between neighboring second ligament attachment regions for more than two neighboring second ligament attachment regions, e.g. by providing one or more elastic element between two neighboring second ligament attachment regions and providing further elastic element(s) between two different neighboring second ligament attachment regions. Likewise, several elastic elements can be provided between a number of neighboring second ligament attachment regions in the second outermost layer.

In addition to providing one or more elastic elements such that they are associated with (e.g. attached to) and/or comprised by the second outermost layer, the one or more intermediate layer(s) may also comprise one or more elastic elements. The one or more elastic elements may be comprised in the area where the ligaments are attached to the one or more intermediate layer(s). For example, one or more elastic element may be comprised by the intermediate layer(s) in the area which is directly below the area where one or more elastic elements are comprised by or associated with the second outermost layer. When the structure is in its flat configuration, all of the one or more elastic elements are be in their stretched state. Contraction of the one or more elastic elements (e.g. by release of the releasable attachment(s) of the second outermost layer to the first outermost layer (110), to the topsheet, to the backsheet and/or to the barrier material, leads to erection of all levels of the multi-level structure. In such embodiments, the intermediate layer(s) do not need to be attached to the second outermost layer, to the first outermost layer (110), to the topsheet, to the backsheet and/or to the barrier material.

Alternatively or in addition, the intermediate layer(s) may be attached to the one or more elastic elements of the second outermost layer - especially if the one or more elastic element is provided longitudinally outboard of the ligament attachment region of th ligament which is comprised by the level to which the second outermost layer forms the superjacent layer and which is closest to one of the lateral edges of the structure, one or more elastic element (195). In this case, the lateral edge of the intermediate layer(s) which is on the same side (along the longitudinal dimension) as the one or more elastic element of the second outermost layer can be attached to the one or more elastic elements, as is shown e.g. in Figure 21 to 23. Upon contraction of the one or more elastic elements, the levels of the multi-level structure are simultaneously erected.

Still alternatively or in addition, one or more elastic element (196) may be comprised by and/or attached to the intermediate layer(s) longitudinally outboard of the second ligament attachment region (136) which is closest to one of the lateral edges of the intermediate layer level. The intermediate layer may be directly attached to the first outermost layer (110), to the topsheet, to the backsheet and/or to the barrier material in a similar manner as the second outermost layer may be attached to these components. Hence, the intermediate layer is attached to the first outermost layer (110), to the topsheet, to the backsheet and/or to the barrier material longitudinally outboard of the one or more elastic elements (or by a portion comprising the one or more elastic elements). Likewise to the above, contraction of the one or more elastic elements comprised by or associated with the second outermost layer (e.g. by release of the releasable attachment(s)), leads to erection of all levels of the multi-level structure. In such embodiments, the intermediate layer(s) do not need to be attached to the second outermost layer.

The one or more elastic elements, especially the dimension of the one or more elastic elements, should be facilitated accordingly, such that upon full contraction of the elastic element(s), the free intermediate portions of the ligaments are properly lifted and the overall structure is transferred into its erected configuration. The exact configuration of the one or more elastic elements thus need to be adapted to the respective overall design of the structure, taking into consideration aspects such as longitudinal dimension of the ligament's free intermediate portions, the distance between neighboring ligaments, the dimension of the one or more elastic elements in their contracted state etc.

Also, the orientation of the ligaments needs to be configured accordingly to enable proper erection of the ligaments upon contraction of the one or more elastic elements. Examples are shown in Figures 18 through 27.

By permanently attaching the second outermost to the first outermost layer (110), to the topsheet, to the backsheet and/or to the barrier material, the one or more elastic elements can be coupled to the rest of the absorbent article, which helps bending of the absorbent article such that, if desired, the absorbent article adopts an overall arcuate form upon contraction of the one or more elastic elements of the structure.

In the structure described above, where the second outermost layer is releasably attached to the first outermost layer (110), to the topsheet, to the backsheet and/or to the barrier material, on both sides (along the longitudinal dimension of the structure) of the one or more elastic elements, the second outermost layer may be permanently attached to the first outermost layer (110), to the topsheet, to the backsheet and/or to the barrier material indirectly via a layer-to-layer stop aid or a layer-to-ligament stop aid as described below in detail, in order to couple the one or more elastic elements to the rest of the absorbent article, and help bending of the absorbent article, if desired, such that the absorbent article adopts an overall arcuate form upon contraction of the one or more elastic elements of the structure. Alternatively or in addition, the second outermost layer may be permanently attached to the first outermost layer (110), to the topsheet, to the backsheet and/or to the barrier material longitudinally outboard of the releasable attachments. In such cases, a leeway has to be provided in the second outermost layer between the releasable attachment and the permanent attachment. Upon release of the releasable attachment, the one or more elastic elements contract and the structure erects. The leeway flattens out (or extends, in case the leeway is provided by extensible or elastic material -which is in a non-stretched state when the structure is in the flat configuration similar to the provision of the stop aid, see below), which can induce an overall arcuate form of the absorbent article.

Unless specifically mentioned herein, "attached" or "attachment" means directly attached.

It is also possible to combine one or more elastic element which is associated with the second outermost layer with one or more elastic elements which are comprised by the second outermost layer.

The ligaments must be attached appropriately for each of the described structures to enable erection of the structure upon contraction of the one or more elastic elements. I.e. the ligaments should not be attached such as to hinder proper erection (see also above with respect to Fig. 10).

The structure can be facilitated such that, in its initial flat configuration, no hinges and folds may be created in the structure (100) and the first and second outermost layers (110, 120), the intermediate layer(s) as well as the ligaments (130) lie flat and outstretched. Such structures allow having a very thin caliper in the flat configuration. In these kinds of structures, all ligaments will adopt a Z-like shape when the structure is in its erected configuration.

In structures where no hinges may be created, the complete first surface (131) of each ligament (130) (i.e. not only the free intermediate portion) does not face towards the superjacent layer when the structure (100) is in its initial flat configuration, and the complete second surface (132) of each ligament (130) (i.e. not only the free intermediate portion) does not face towards the subjacent layer when the structure is in its initial flat configuration. Such a structure is illustrated in Figs. 1A and 1B.

Figs. 7 and 8 show structures where the ligaments are folded when the structure is in its initial flat configuration.

Also, for certain other executions, e.g. those where the longitudinal dimension of the free intermediate portion (137) of the ligaments (130) differs from each other, the initial flat configuration may have some folds and hinges.

Upon erection of the structure (100), the first and second outermost layer (110, 120) shift relative to each other along the longitudinal dimension. At the same time, the structure (100) erects due to the erection of the ligaments (130), a space, a so-called "cell" (140) is formed between neighboring ligaments, which is confined by the subjacent and superjacent layer and the respective neighboring ligaments. When viewed from the side, along the lateral direction, the cells may take for example a rectangular shape, a trapezoid shape, a rhomboid shape, or the like. However, for use in absorbent articles according to the present invention, the structure will typically not form any rectangular shapes as the absorbent article will tend to flex upwardly along the longitudinal axis of the article, thus forming an overall arcuate form. The structure, being attached to the absorbent article, will follow this arcuate shape, and the shape of the cells will not be completely rectangular accordingly.

For structures wherein, for all levels, the longitudinal dimension of the free intermediate portion (137) is the same for all ligaments within a level, the structure (100) will adopt its highest possible caliper when the ligaments (130) are in an upright position, i.e. when the free intermediate portion (137) of the ligaments (130) is perpendicular to the first and second outermost layer (110, 120) between neighboring ligaments. However, the formation of this upright position may possibly be hindered, at least in some areas, when a force towards the caliper of the structure is applied at the same time, if this force is sufficiently high to deform the structure in the caliper dimension. The formation of such fully upright position may also be hindered when the absorbent article flexes and takes an arcuate form due to erection of the structure.

In structures, where the longitudinal dimension of the free intermediate portion (137) differs between different ligaments (130) within a level, the ligaments (130) may not be perpendicular to the first and second outermost layer (110, 120) in the erected configuration In such embodiments, the first and second outermost layer (110, 120) are not parallel to each other when the structure is in its erected configuration but instead, the first and/or second outermost layer (110, 120) take(s) an inclined shape. This may also happen due to the arcuate form of the absorbent article described above.

Tensile strength, and especially bending stiffness, impacts the resistance of the structure (especially of the structure in its erected configuration) against compression forces. Thus, when the ligaments have a relatively high tensile strength and bending stiffness, the structure is more resistant to forces applied in the Z-direction (i.e. towards the caliper of the structure). Also, if the first and/or second outermost layers have relatively high tensile strength and relatively high bending stiffness, resistance of the structure against forces applied in the Z-direction (i.e. towards the caliper of the structure) is increased. For the present invention, the compression resistance of the erected structure is measured in terms of the structure's modulus according to the test method set out below.

As a difference in bending stiffness results in a difference in the resistance against compression forces (and hence, the modulus) applied in the Z-direction (i.e. towards the caliper of the structure), using ligaments with different bending stiffness enables structures which have improved resistance to compression (higher modulus) in the Z-direction in areas where the ligaments have higher bending stiffness, whereas the structure adjusts more readily e.g. to curved surfaces in the areas where ligaments with lower bending stiffness are applied (lower structure modulus). For example, the bending stiffness of the ligaments which are arranged in the center of the structure along the longitudinal dimension may be higher compared to the ligaments arranged towards the lateral edges of the structure.

Not only the bending stiffness and/or tensile strength of different ligaments comprised by the same level may differ from each other, but in addition to or instead of having ligaments with varying bending stiffness and/or tensile strength within the same level, ligaments comprised by one or more levels may also differ in bending stiffness and/or tensile strength from ligaments comprised by one or more other levels.

The ligaments of the level which is closest to the skin of the wearer when the article is in use, may have a lower bending stiffness and/or lower tensile strength compared to ligaments of levels being farther from the skin of the wearer.

When the ligaments of a level differ from each other in bending stiffness and/or tensile strength, one or more ligaments which are closer to the lateral edges of the multi-level structure may have a lower bending stiffness and/or lower tensile strength compared to one or more ligaments which are arranged in or towards the center of the multi-level structure along the longitudinal dimension.

In addition to the tensile strength and the bending stiffness of the materials used for the structure, the resistance of the (erected) structure against compression forces is also impacted by the number of ligaments which are provided, and the distance between neighboring ligaments. Neighboring ligaments which have a relatively small gap between them along the longitudinal dimension of the structure will provide for higher resistance of the erected structure against compression forces (higher modulus) compared to a structure wherein neighboring ligaments are more widely spaced apart along the longitudinal dimension of the structure (lower modulus) as long as the material used for the different ligaments and their size does not differ from each other.

Different levels may have different numbers of ligaments. In addition to or instead of having different number of ligaments in different levels, the ligaments may also be spaced at different distances from each other. Generally, the distance between neighboring ligaments may be constant or may vary within a given level.

Furthermore, the ligaments of different levels may be arranged such that their position coincides in the thickness direction of the structure (i.e. perpendicular to the longitudinal and lateral structure dimension). Alternatively, the ligaments of different levels may be staggered such that their position does not coincide in the thickness direction of the structure. Combinations of the aforementioned are also possible, i.e. the position of ligaments between two or more levels coincides while the position of ligaments between these two or more levels and one or more other levels does not coincide. In a still further alternative, the position of some ligaments within a level may coincide with the position of some or all (if the other level has fewer ligaments) ligaments of one or more other levels, while the position of other ligaments within this level does not coincide with the position of one or more ligaments of another level. Generally, a multitude of arrangements, positions and/or distributions of ligaments in the different levels are feasible.

Moreover, if the modulus is measured between neighboring ligaments, the modulus will typically be lower than the modulus measured in the location where a ligament is positioned.

Modulus is measured following the test method set out below and is measured in the Z-direction of the structure. Generally, the modulus of a structure is not measured while the structure is built into the absorbent article by ideally, prior to incorporating the structure into the absorbent article (otherwise, the structure needs to be carefully removed from the absorbent article).

The structure in its erected configuration may have a modulus of at least 0.004 N/mm$^2$, or at least 0.01 N/mm$^2$, or at least 0.02 N/mm$^2$, or at least 0.03 N/mm$^2$ in those areas where a ligament is positioned as well as in the areas between neighboring ligaments.

Moreover, it may also be desirable to avoid excessively high compression resistance (i.e. too high modulus), e.g. to avoid that the erected structure is too stiff, thus possibly leading to red marking on the wearer's skin in the area of the structure. For such structures, the structure in its erected configuration may have a modulus of not more than 1.0 N/mm$^2$, or not more than 0.5 N/mm$^2$, or not more than 0.2 N/mm$^2$, but at least 0.1 N/mm$^2$, or at least 0.5 N/mm$^2$, or at least 1.0 N/mm$^2$ in those areas where a ligament is positioned as well as in the areas between neighboring ligaments.

Alternatively, the structure in its erected configuration may have a modulus of at least 0.05 N/mm$^2$, or at least 0.08 N/mm$^2$, or at least 0.1 N/mm$^2$, or at least 0.13 N/mm$^2$, but not more than 2.0 N/mm$^2$, or not more than 1.0 N/mm$^2$, or not more than 0.5 N/mm$^2$, or not more than 0.3 N/mm$^2$ in the locations where the ligaments are positioned, while the structure in its erected configuration may have a modulus of at least 0.004 N/mm$^2$, or at least 0.01 N/mm$^2$, or at least 0.02 N/mm$^2$, or at least 0.03 N/mm$^2$ between neighboring ligaments.

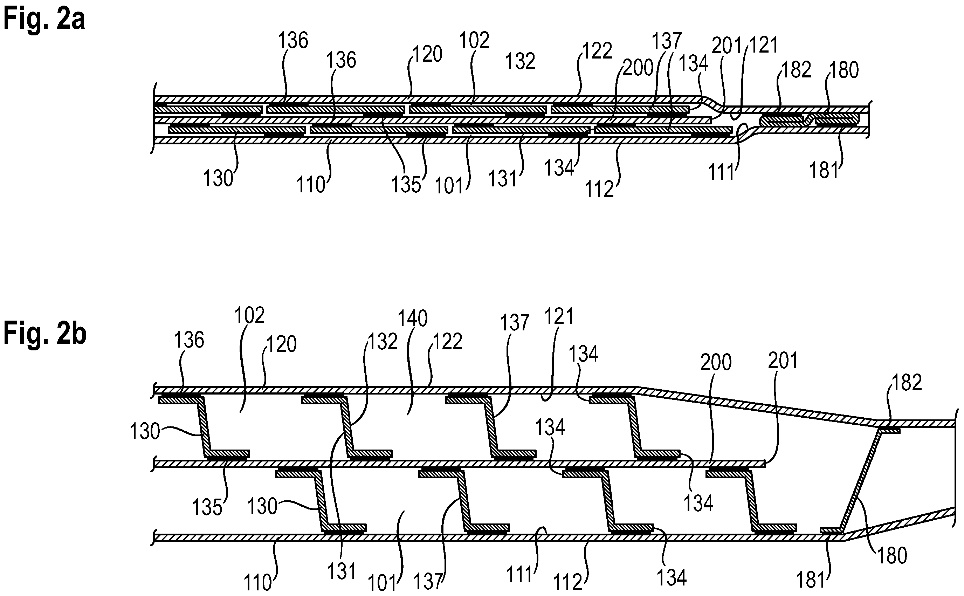

Generally, the ligaments (130) of a given level may be spaced apart from each other along the longitudinal dimension at equal distances or, alternatively, at varying distances (this may be applicable to each level or to only one or some levels). Also, the ligaments (130) of a given level may be spaced apart from each other such, that the ligaments (130) of given level do not overlap with each other when the structure (100) is in its initial flat configuration (again, this may be applicable to each level or to only one or some levels). Thereby, it is possible to provide structures (100) with very small caliper when the structure (100) is in its initial flat configuration, as the ligaments (130) do not "pile up" one on top of each other when the structure is in its initial flat configuration. An example of such structure is shown in Figs. 2A and 2B, wherein the ligaments do not overlap with each other within both, the first and the second level.

The free intermediate portion (137) of the ligaments (130) within each level may all have the same longitudinal dimension (the free intermediate portion (137) of the ligaments between different levels may have different longitudinal dimensions). Thereby, the structure will have a constant caliper across its longitudinal (and lateral) dimension between neighboring ligaments when the structure (100) is in its erected configuration. An example of such an embodiment is shown in Figure 1B. Alternatively, the longitudinal dimension of the free intermediate portion (137) may vary for different ligaments (130) within a level of a structure (100). Thereby, the caliper of the structure will vary across the longitudinal dimension.

For example, the one or more ligaments (130) in the center of the structure (as seen along the longitudinal dimension) of one or more levels (may also be all levels) may have a longer free intermediate portion (137) than the ligaments towards the lateral edges of the structure, resulting in a structure with a overall higher caliper in the center than towards the edges when the structure is in its erected configuration. Generally, the caliper of the erected structure depends on the length of the free intermediate portion (137) of the ligaments (130).

Generally, the maximum increase in caliper of the structure is defined by the longitudinal dimension of the free intermediate portion (137) of the ligaments (130). If the ligaments (130) differ from each other in the longitudinal dimension of their free intermediate portions (137), the maximum increase in caliper of the structure in its erected configuration, as used herein, is defined by the longitudinal dimension of the ligament with the largest longitudinal dimension of free intermediate portion. If a stop aid (180, 190) is used (as described below), the structure may not be able to adopt its maximum increase in caliper in its erected configuration as the erection is stopped by the stop aid before the maximum erection, which would have been possible in the absence of a stop aid, is reached.

If formation of wrinkles in the first and second outermost layer (110, 120), in the intermediate layer(s) (200, 210) and/or in the ligament (130) shall be avoided when the structure is in its erected configuration, it is desirable, that the ligaments (130) are arranged such that, when the structure is in its initial flat configuration, the longitudinal dimension of the ligaments is substantially parallel with the longitudinal dimension of the first and second outermost layer and with the intermediate layer(s). "Substantially parallel" means that the orientation of the longitudinal dimension of the ligaments does not deviate by more than 20°, or not more than 10°, or not more than 5°, or not more than 2° from the longitudinal dimensions of the first and second outermost layer and the intermediate layer(s). The orientation of the longitudinal dimension of the ligaments may also not deviate at all from the longitudinal dimension of the first and second outermost layer and of the intermediate layer(s).

Typically, the free intermediate portions (137) are not attached to each other. Generally, the first and second outermost layer (110, 120) may have the same lateral dimension and the longitudinal edges of the first and second outermost layer may be congruent with each other. The intermediate layer(s) (200, 210) may also have the same lateral dimension and the longitudinal edges of the intermediate layer(s) may be congruent with each other (if more than one intermediate layer is used) and may also be congruent with the first and second outermost layers.

Also, the lateral dimension of all ligaments (130) may be the same, and the lateral dimension of all ligaments (130) may also be the same as the lateral dimension of the respective subjacent and superjacent layers. The longitudinal edge of the first outermost layer (110) may coincide with the longitudinal edge of the second outermost layer (120), may further coincide with the longitudinal edge of the intermediate layer(s) and/or with the longitudinal edges of the ligaments (130).

However, the intermediate layer(s) (200, 210) may also have a smaller or wider longitudinal dimension as the first and second outermost layer, such that the structure has narrower or wider portions in along its caliper.

Also, one or more of the ligaments (130) may have the same lateral dimension as the respective subjacent and superjacent layers and one or more of the ligaments, which do not have the same lateral dimension as the respective subjacent and superjacent layer may be flanked on one or both longitudinal edges by other ligaments, such that the structure has laterally neighboring ligaments.

Ligaments

The ligaments may be elastic, but it is desirable that the ligaments are non-elastic or highly non-elastic. Also, the ligaments may be extensible, but it is desirable that the ligaments are non-extensible or highly non-extensible. The ligaments may be made of nonwovens, film, paper, or sheet-like foam, woven fabric, knitted fabric, or combinations of these materials. Combinations of these materials may be laminates, e.g. a laminate of a film and a nonwoven.

The ligaments within a structure may all be made of the same material or, alternatively, the different ligaments within a structure may be made of different materials.

The material may be the same throughout each ligament, i.e. the ligament may be made of a single piece of material or of a laminate wherein each laminate layer extends over the complete ligament.

The ligaments may have the same properties throughout the ligament, especially with regard to bending stiffness and tensile strength.

Alternatively, the ligaments may have areas with properties (such as bending stiffness and/or tensile strength) which differ from the properties in one or more other areas of a given ligament.

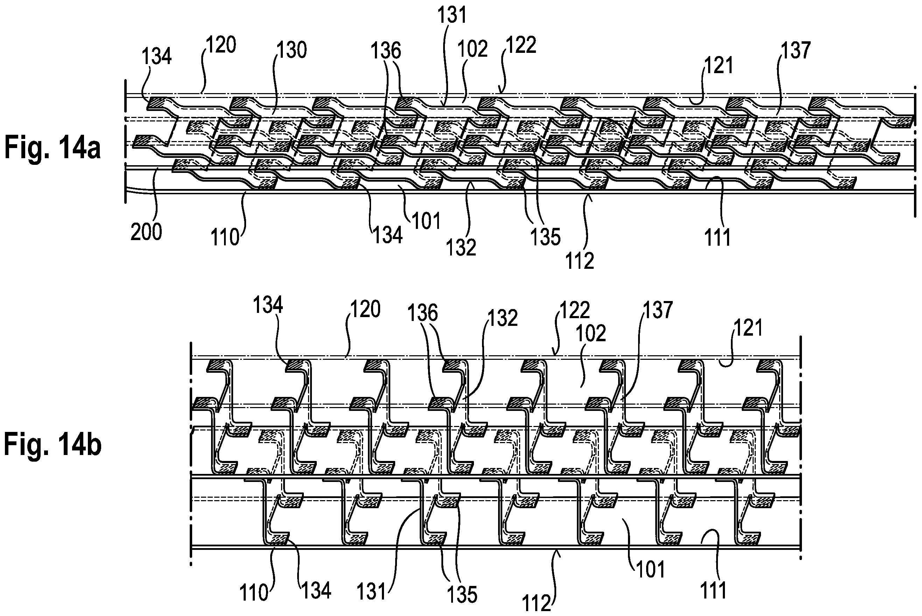

Such areas with differing properties can be facilitated by modifying the material in one or more ligament areas, e.g. by mechanical modification. Non-limiting examples of mechanical modifications are the provision of cut outs in one or more areas of the ligament to reduce tensile strength and bending stiffness in those areas; incremental stretching (so-called "ring-rolling") one or more ligament areas to reduce tensile strength and bending stiffness; slitting one or more ligament areas to reduce tensile strength and bending stiffness; applying pressure and/or heat to one or more areas of ligaments; or combinations of such mechanical modifications. Application of heat and/or pressure may either increase or reduce tensile strength and bending stiffness: For example, if heat and/or pressure are applied on a ligament made of nonwoven with fibers made of thermoplastic material, the fibers may be molten together and bending stiffness and tensile strength can be increased. However, if an excessive amount of heat and/or pressure is used, the material of the ligaments may be damaged (such as fiber breakage in a nonwoven web) and weakened areas are formed, thus reducing bending stiffness and tensile strength. Cutting out areas of the ligaments may either result in the formation of apertures within the ligament or the cut out may not be fully surrounded by uncut areas. An example is shown in Figs. 14A and 14B.

Alternatively, or in addition to the above, areas with different properties can also be obtained by chemically modifying one or more areas of the ligament, e.g. by adding chemical compounds, such as binders or thermoplastic compositions to increase bending stiffness and tensile strength, which may be followed by curing. One or more areas with different properties can also be obtained by providing different materials in different areas or by adding additional pieces of materials only in certain areas (thus, e.g. forming a laminate in these areas), while having another material (which may be the same or different from the piece of material added in certain areas) which is coextensive with the ligament and used throughout the ligament.

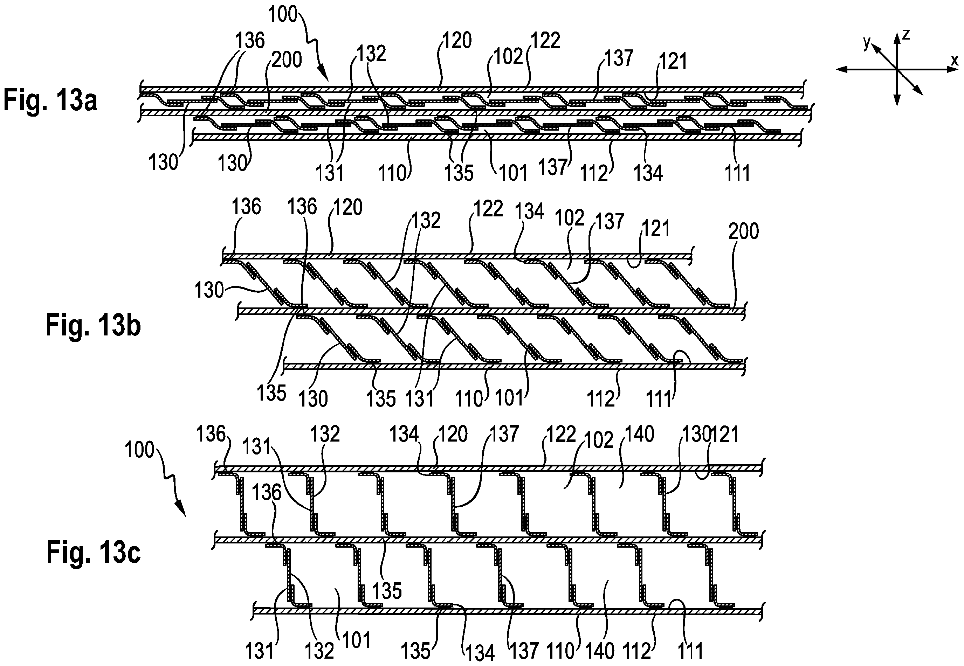

Still further, one or more areas with different properties may be achieved by providing ligaments which are assembled by attaching different pieces of material to each other so they overlap partly and partly do not overlap, such that together they form the overall ligament (instead of having one continuous material coextensive with the ligament to which additional pieces of material(s) are added only in certain areas). Examples of structures with ligaments being assembled of pieces of (different) materials are illustrated in Fig. 12A through 12C and 13A through 13C.

By having ligaments with one or more areas having properties different from the remaining ligament, the behavior of the structure with respect to e.g. bending stiffness and tensile strength can be fine-tuned to meet certain needs in different areas of the structure (e.g. the ability to accommodate readily and softly to the skin of a wearer in some areas and to be stiffer and more resistant to compression in other areas to close gaps).

If providing such areas of different properties by any of the above means, it may be especially desirable to provide them in the areas of the free intermediate portion of a ligament which are directly adjacent to the first and second ligament attachment region. The areas of the free intermediate portion which are directly adjacent to the first and second ligament attachment regions are those areas which bend upon release of a contractive force, which has provided by the stretched one or more elastic elements along the longitudinal direction of the structure, thus erecting the free intermediate portion.

The basis weight of each of the ligaments may be at least 1 $g/m^2$, or at least 2 $g/m^2$, or at least 3 $g/m^2$, or at least 5 $g/m^2$; and the basis weight may further be not more than 1000 $g/m^2$, or not more than 500 $g/m^2$, or not more than 200 $g/m^2$, or not more than 100 $g/m^2$, or not more than 50 $g/m^2$, or not more than 30 $g/m^2$.

If the tensile strength is the same throughout the ligament, the tensile strength of the ligaments may be at least 3 N/cm, or at least 5 N/cm or at least 10 N/cm. The tensile strength may be less than 100 N/cm, or less than 80 N/cm, or less than 70 N/cm, or less than 50 N/cm, or less than 40 N/cm.

If the tensile strength is the same throughout the ligament, the bending stiffness of the ligaments may be at least 0.1 mNm, or at least 0.2 mNm, or at least 0.3 mNm. The bending stiffness may be less than 500 mNm, or less than 300 mNm, or less than 200 mNm, or less than 150 mNm. Principally, for the ligaments the same considerations regarding overall softness, drape and conformability versus overall stability and robustness apply as set out above for the first and second outermost layer. However, the bending stiffness and tensile strength of the ligaments typically has a higher impact on the overall bending resistance of the erected structure (when a force is applied along the caliper of the structure, i.e. perpendicular to the lateral and longitudinal dimension of the structure) vs. the impact of the bending stiffness and tensile strength of the first and second outermost layer. Thus, it may be desirable that the ligaments have a higher bending stiffness and a higher tensile strength than the first and second outermost layer.

The different ligaments in a structure may vary from each other in basis weigh and/or tensile strength, bending stiffness and the like.

**Stop aid**

It may be desirable to define a maximum shifting of the first and second outermost layers (110, 120) relative to each other upon release of the contractive force, which has provided by the stretched one or more elastic elements along the longitudinal dimension of the structure (100). This can be facilitated by providing a stop aid (180; 190). However, for the present invention, the provision of such stop aid may not be required, as the maximum shifting of the first outermost layer and the second outermost layer relative to each other is generally defined by the contraction of the one or more elastic elements. Hence, when the one or more elastic elements are completely contracted, no further shifting of first and second outermost layer occurs. It may, however, be desirable to stop further erection by hindering further contraction of the one or more elastic elements, such that the one or more elastic elements do not fully contract. Therefore, a stop aid can be facilitated to provide a counter-force along the longitudinal dimension of the structure, such that, in the erected structure, the force applied by the one or more elastic elements in one direction along the longitudinal dimension (due to incomplete contraction of the elastic elements) is balanced against a force applied in the respective opposite direction by an appropriate stop aid.

By using a stop aid (180; 190), the structure (100) can thus be stopped in a defined erected configuration, i.e. with a defined caliper (which, however, is higher than the caliper of the structure in its flat configuration), even if the force along the longitudinal dimension is continued to be applied (due to incomplete contraction of the one or more elastic elements). The stop aid (180; 190) may facilitate that the structure (100) is stopped in the erected configuration with a certain caliper, which is higher than the caliper of the initial flat configuration but lower than the highest possible caliper which would be possible due to the longitudinal dimension of the free intermediate portion (137) of the ligaments (130).

There are many different ways to provide a stop aid (180; 190), for example:

a) A layer-to-layer stop aid (180) may be provided, which extends from the first outermost layer (110) to the second outermost layer (120). This layer-to-layer stop aid (180) is attached to the inner surface (111) or outer surface (112) of the first outermost layer (110) in a first layer-to-layer stop aid attachment region (181) and is further attached to the inner surface (121) or outer surface (122) of the second outermost layer (120) in a second layer-to-layer stop aid attachment region (182). The first layer-to-layer stop aid attachment region (181) may be longitudinally spaced apart from the second layer-to-layer stop aid attachment region (182) when the structure is in its initial flat configuration. The layer-to-layer stop aid (180) is provided with a layer-to-layer stop aid leeway between the first and second layer-to-layer stop aid attachment regions (181, 182) when the structure (100) is in its initial flat configuration. The leeway may be configured in form of a slack. Upon release of a contractive force, which has provided by the stretched one or more elastic elements along the longitudinal dimension the first and second outermost layers (110, 120) shift relative to each other along the longitudinal dimension, the structure contracts in areas where the one or more elastic elements are provided and erects, and the slack forming the layer-to-layer stop aid leeway between the first and second layer-to-layer stop aid attachment regions (181, 182) straightens out. Once the slack is flattened when the structure (100) is in its erected position, further longitudinal extension of the structure is inhibited also when the force in the longitudinal dimension is continued to be applied. An example of a layer-to-layer stop aid (180) is illustrated in Figs. 2A (initial flat configuration) and 2B (erected configuration).

Alternatively or in addition, the leeway of the layer-to-layer stop aid (180) can be generated by adapting the material between the first and second layer-to-layer stop aid attachment regions (181, 182) to create extensibility of the respective area of the layer-to-layer stop aid (180). Adapting the material can be done by modifying the material, e.g. by selfing (weakening the material of the first or second outermost layer to render it relatively easily extensible), creating holes or using extensible materials to form the leeway.

Alternatively or in addition, the layer-to-layer stop aid (180) may be made of extensible material. For such layer-to-layer stop aids (180), the material of the leeway elongates when the first and second outermost layers (110, 120) are shifted against each other until elongation of the layer-to-layer stop aid leeway is not possible any longer (without applying an excessive amount of force, which may even rupture the structure). Hence, the material in the leeway has reached its maximum elongation i.e. it cannot be elongated further upon application of force without causing damage to the structure that limits or impedes its intended use.

The material of the leeway may also be elastic. For such structures, the layer-to-layer stop aid (180) can retract when the force is no longer applied onto the structure such that the structure can substantially "snap back" into its initial flat configuration. The difference between the provision of an elastic leeway and the one or more elastic elements comprised by or associated with the structure is that the one or more elastic elements are stretched when the structure is in its flat configuration and contracts upon erection of the structure. Contrary thereto, an elastic leeway will be relaxed in the flat configuration of the structure and stretch upon erection of the structure.

It is also possible to provide a leeway that is a combination of a slack and the provision of extensible material in the leeway, such that, upon elongation, initially the slack straightens out and subsequently, the extensible material elongates.

Compared to the attachment of the ligaments to the first and second outermost layer and the resulting configuration, the layer-to-layer stop aid, when applied between the first and second outermost layer and being attached to the inner surfaces of the first and second outermost layer, does not adopt a Z-like or C-like shape having the same orientation as the ligaments with Z-like or C-like shape (otherwise, it would simply be an additional ligament). Instead, the first layer-to-layer stop aid attachment region may be between the first surface of the layer-to-layer stop aid and the inner surface of the first layer. The second layer-to-layer stop aid attachment region may be between the first surface of the layer-to-layer stop aid (i.e. on the same surface of the layer-to-layer stop aid as the first layer-to-layer stop aid attachment region) and the inner surface of the second outermost layer. Thus, the layer-to-layer stop aid adopts a C-like shape when the structure is in its erected configuration.

Alternatively or additionally, the first layer-to-layer stop aid attachment region may be between a first surface of the layer-to-layer stop aid and the inner surface of the first outermost layer. The second layer-to-layer stop aid attachment region may be between the second surface of the layer-to-layer stop aid (i.e. opposite surface of the layer-to-layer stop aid than the first layer-to-layer stop aid attachment region) and the inner surface of the second outermost layer. Thus, the layer-to-layer stop aid adopts a Z-like shape configuration when the structure is in its erected configuration. Attachment of the layer-to-layer stop aid (180) to the first and second outermost layer (110, 120) in the first and second layer-to-layer stop aid attachment regions (181, 182) can be obtained by any means known in the art, such as adhesive, thermal bonding, mechanical bonding (e.g. pressure bonding), ultrasonic bonding, or combinations thereof.

As the first outermost layer is attached to the topsheet, to the backsheet and/or to the barrier material (or a portion of the topsheet, the backsheet or the barrier material forms the first outermost layer), the layer-to-layer attachment regions may actually also be between the second outermost layer and the topsheet, the backsheet and/or the barrier material, as long as all necessary requirements set out above for the layer-to-layer stop aid are fulfilled to provide a stop aid function.

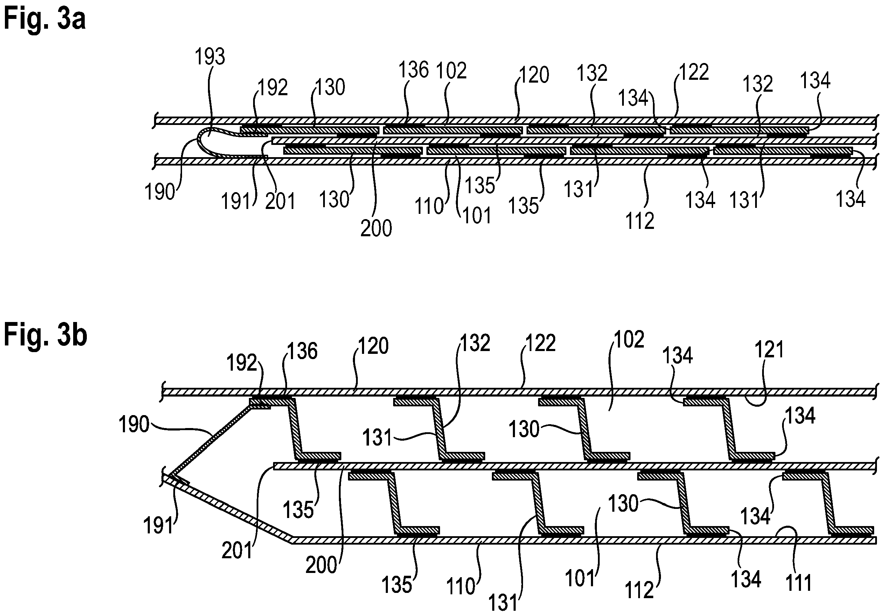

b) A layer-to-ligament stop aid (190) may be provided, which extends from the first or second outermost layer (110, 120) to one of the ligaments (130) comprised by the level which is farthest away from the respective first or second outermost layer in the direction perpendicular to the longitudinal and lateral dimensions (i.e. in caliper direction). This layer-to-ligament stop aid (190) is attached to the inner surface (111) or outer surface (112) of the first or second outermost layer (110, 120) in a first layer-to-ligament stop aid attachment region (191) and is further attached to the first surface (131) or second surface (132) of the ligament (130) in a second layer-to-ligament stop aid attachment region (192). The first layer-to-ligament stop aid attachment region (191) may be longitudinally spaced apart from the second layer-to-ligament stop aid attachment region (192). The layer-to-ligament stop aid (190) is provided with a layer-to-ligament stop aid leeway between the first and second layer-to-ligament stop aid attachment region (191, 192) when the structure (100) is in its initial flat configuration. The leeway may be configured in form of a slack (193). Upon release of a contractive force, which has provided by the stretched one or more elastic elements in the longitudinal dimension the first and second outermost layer (110, 120) shift relative to each other along the longitudinal dimension, the structure contracts in areas where the one or more elastic elements are provided and erects, and the slack (193) forming the layer-to-ligament stop aid leeway between the first and second layer-to-ligament stop aid attachment regions (191, 192) straightens out. Once the slack (193) is straightened out when the structure (100) is in its erected position, further longitudinal extension of the structure is inhibited also when the force in the longitudinal dimension is continued to be applied. A layer-to-ligament stop aid (190) is shown in Figs. 3A (initial flat configuration) and 3B (erected configuration).

Alternatively or additionally, the leeway of the layer-to-ligament stop aid (190) can be generated by adapting the material between the first and second layer-to-ligament stop aid attachment regions (191, 192) to create extensibility of the respective area of the layer-to-ligament stop aid (190). Adapting the material can be done by modifying the material, e.g. by selfing (weakening the material of the first or second outermost layer to render it relatively easily extensible), creating holes or using extensible materials to form the leeway.

Alternatively or additionally, the layer-to-ligament stop aid (190) may be made of extensible material. For such layer-to-ligament stop aids (190), the material of the leeway elongates when the first and second outermost layers (110, 120) are shifted against each other until elongation of the layer-to-ligament stop aid leeway is not possible any longer (without applying an excessive amount of force, which may even rupture the structure). Hence, the material in the leeway has reached its maximum elongation i.e. it cannot be elongated further upon application of force without causing damage to the structure that limits or impedes its intended use.

The material of the leeway may also be elastic. For such structures, the layer-to-ligament stop aid (190) can retract when the force is no longer applied onto the structure such that the structure can substantially "snap back" into its initial flat configuration.

For the material properties and appropriate selection of extensible or elastic leeways, and the difference between elastic leeways and the one or more elastic elements comprised by or associated with the structure, the same considerations apply as are set out above for the layer-on-layer stop aid.

It is also possible to provide a leeway that is a combination of a slack and the provision of extensible material in the leeway, such that, upon elongation, initially the slack straightens out and subsequently, the extensible material elongates.

Attachment of the layer-to-ligament stop aid (190) to the first and second outermost layer (110, 120) in the first and second layer-to-layer stop aid attachment regions

(181, 182) can be obtained by any means known in the art, such as adhesive, thermal bonding, mechanical bonding (e.g. pressure bonding), ultrasonic bonding, or combinations thereof.

Generally, the layer-to-ligament stop aid (190) may be attached in the first and second layer-to-ligament stop aid attachment regions such that the layer-to-ligament stop aid extends along or adjacent to one of the longitudinal edges of the at least one ligament (130) or, alternatively, such that it extends between the longitudinal edges of the at least one ligament.

As the first outermost layer is attached to the topsheet, to the backsheet and/or to the barrier material (or a portion of the topsheet, the backsheet or the barrier material forms the first outermost layer), the layer-to-ligament attachment regions may actually also be between the second outermost layer and the topsheet, the backsheet and/or the barrier material, as long as all necessary requirements set out above for the layer-to-ligament stop aid are fulfilled to provide a stop aid function.

c) The structure (100) may comprise an enveloping stop aid which encircles at least a portion of the first and second outermost layer (110, 120), the intermediate layer(s) and the ligaments (130) provided in the respective portion. This enveloping stop aid is attached to the first outermost layer (110), the second outermost layer (120), one or more of the intermediate layer(s) and/or at least one of the ligaments (130) in one or more enveloping stop aid attachment region. Attaching the enveloping stop aid to only one of the first outermost layer (110), the second outermost layer (120), one of the intermediate layer(s) or at least one of the ligaments (130) in only one enveloping stop aid attachment region is, however, sufficient.

The enveloping stop aid is attached to itself to form a closed loop with a defined circumference around at least a portion of the first and second outermost layer with the intermediate layer(s) and ligaments in between. Alternatively or in addition, if the structure is enwrapped by a barrier material as described above, the barrier material may serve as an enveloping stop aid. Also, the barrier material, if not fully enwrapping the structure, may, in conjunction with the topsheet and/or the backsheet to which it is attached, serve as an enveloping stop aid. The enveloping stop aid may (probably together with portions of the topsheet or with portions of the backsheet) encircle the first and second outermost layer (110, 120) along the lateral dimension (and the caliper).

The circumference of the enveloping stop aid defines the maximum shifting of the first outermost layer (110) and the second outermost layer (120) relative to each other upon release of a contractive force, which has provided by the stretched one or more elastic elements along the longitudinal dimension.

When the structure (100) is in its initial flat configuration, the enveloping stop aid is loose around the first and second outermost layer (and the respective ligaments between the first and second outermost layer). Upon release of a contractive force, which has provided by the stretched one or more elastic elements along the longitudinal dimension of the structure, the structure erects until the enveloping stop aid fits tightly around the first and second outermost layer (and the respective intermediate layer(s) and ligaments between the first and second outermost layer), which will stop further shifting of the first outermost layer relative to the second outermost layer also if the force along the longitudinal dimension is continued to be applied. To assist in avoiding overexpansion of the structure (100), the circumference of the enveloping stop aid may be such that further shifting of the first outermost layer (110) relative to the second outermost layer (120) along the longitudinal dimensions is inhibited before the ligaments (130) are in their fullest upright position.

*General considerations for the layer-to-layer stop aid, the layer-to-ligameht stop aid and, if expressly mentioned, the enveloping stop aid:*

The layer-to-layer stop aid and/or the layer-to-ligament stop aid may be non-elastic or highly non-elastic (apart from the leeway, if the leeway is provided by modifying the material to render it elastically extensible). Also the layer-to-layer stop aid and/or the layer-to-ligament stop aid may be non-extensible or highly non-extensible (apart from the leeway, if the leeway is provided by modifying the material to render it extensible).

The layer-to-layer stop aid and/or the layer-to-ligament stop aid and/or enveloping stop aid can be made of a sheet-like material, such as nonwoven, film, paper, sheet-like foam, woven fabric, knitted fabric, or combinations of these materials. Combinations of these materials may be laminates, e.g. a laminate of a film and a nonwoven. The layer-to-layer stop aid and/or the layer-to-ligament stop aid and/or enveloping stop aid may also be made of a cord-or string-like material.

The layer-to-layer stop aid and/or the layer-to-ligament stop aid and/or enveloping stop aid is not necessarily intended to contribute to the resistance of the structure against a force exerted onto the structure in the thickness-direction. However, the basis weight, tensile strength and bending stiffness of the layer-to-layer stop aid and/or the layer-to-ligament stop aid and/or enveloping stop aid should be sufficiently high to avoid inadvertent tearing of the layer-to-layer stop aid and/or the layer-to-ligament stop aid and/or enveloping stop aid upon expansion of the structure.

If the layer-to-layer stop aid and/or the layer-to-ligament stop aid and/or enveloping stop aid is made of a sheet-like material, the basis weight of the layer-to-layer stop aid and/or the layer-to-ligament stop aid and/or enveloping stop aid may be at least 1 g/m$^2$, or at least 2 g/m$^2$, or at least 3 g/m$^2$, or at least 5 g/m$^2$; and the basis weight may further be not more than 500 g/m$^2$, or not more than 200 g/m$^2$, or not more than 100 g/m$^2$, or not more than 50 g/m$^2$, or not more than 30 g/m$^2$.

If the layer-to-layer stop aid and/or the layer-to-ligament stop aid and/or enveloping stop aid is made of a cord-or string-like material, the basis weight of the layer-to-layer stop aid and/or the layer-to-ligament stop aid and/or enveloping stop aid may be at least 1 gram per meter (g/m), or at least 2 g/m, or at least 3 g/m, or at least 5 g/m; and the basis weight may further be not more than 500 g/m, or not more than 200 g/m, or not more than 100 g/m, or not more than 50 g/m, or not more than 30 g/m.

The basis weight of the layer-to-layer stop aid and/or the layer-to-ligament stop aid may be less than the basis weight of the ligaments, for example the basis weight of the layer-to-layer stop aid and/or the layer-to-ligament stop aid may be less than 80%, or less than 50% of the basis of the ligaments (if the ligaments vary in basis weight, then these values are with respect to the ligament(s) with the lowest basis weight).

The tensile strength of the layer-to-layer stop aid may be at least 2 N/cm, or at least 4 N/cm or at least 5 N/cm. The tensile strength may be less than 100 N/cm, or less than 80 N/cm, or less than 50 N/cm, or less than 30 N/cm, or less than 20 N/cm.

The bending stiffness of the layer-to-layer stop aid and/or the layer-to-ligament stop aid and/or enveloping stop aid may be at least 0.1 mNm, or at least 0.2 mNm, or at least 0.3 mNm. The bending stiffness may be less than 200 mNm, or less than 150 mNm, or less than 100 mNm, or less than 50 mNm, or less than 10 mNm, or less than 5 mNm. These values apply to sheet-like layer-to-layer stop aids and/or layer-to-ligament stop aids and/or enveloping stop aids, for cord- or string-like layer-to-layer stop aids and/or layer-to-ligament stop aids and/or enveloping stop aids, the bending stiffness is generally not seen as critical.

The tensile strength of the layer-to-layer stop aid and/or the layer-to-ligament stop aid and/or enveloping stop aid may be lower than the tensile strength of the ligaments, for example the tensile strength of the layer-to-layer stop aid may be less than 80%, or less than 50% of the tensile strength of the ligaments (if the ligaments vary in tensile strength, then these values are with respect to the ligament(s) with the lowest tensile strength).

The bending stiffness of the layer-to-layer stop aid and/or the layer-to-ligament stop aid and/or enveloping stop aid (when made of sheet-like material) may be lower than the bending stiffness of the ligaments, for example the bending stiffness of the layer-to-layer stop aid and/or the layer-to-ligament stop aid and/or enveloping stop aid may be less than 80%, or less than 50% of the bending stiffness of the ligaments (if the ligaments vary in bending stiffness, then these values are with respect to the ligament(s) with the lowest bending stiffness).

**Test methods:**

**Tensile Strength**

Tensile Strength is measured on a constant rate with extension tensile tester Zwick Roell Z2.5 with computer interface, using TestExpert 11.0 Software, as available from Zwick Roell GmbH &Co. KG, Ulm, Germany. A load cell is used for which the forces measured are within 10% to 90% of the limit of the cell. Both the movable (upper) and stationary (lower) pneumatic jaws are fitted with rubber faced grips, wider than the width of the test specimen. All testing is performed in a conditioned room maintained at about 23°C + 2°C and about 45 % $\pm$ 5% relative humidity.

With a die or razor knife, cut a material specimen which is 25.4 mm wide and 100 mm long. For the present invention, the length of the specimen correlates to the longitudinal dimension of the material within the structure.

If the ligament is smaller than the size of the material specimen specified in the previous paragraph, the material specimen may be cut from a larger piece such as the raw material used for making the ligaments. Care should be taken to correlate the orientation of such specimen accordingly, i.e. with the length o the specimen correlating to the longitudinal dimension of the material within the structure. However, if the ligament has a width somewhat smaller than 25.4 mm (e.g. 20 mm, or 15 mm) the width of the specimen can be accordingly smaller without significantly impacting the measured tensile strength.

If the ligament comprises different materials in different regions, the tensile strength of each material can be determined separately by taking the respective raw materials. It is also possible to measure the tensile strength of the overall ligament. However, in this case, the measured tensile test will be determined by the material within the ligament which has the lowest tensile strength.

Precondition the specimens at about 23 °C $\pm$ 2°C and about 45 % $\pm$ 5% relative humidity for 2 hours prior to testing.

For analyses, set the gauge length to 50 mm. Zero the crosshead and load cell. Insert the specimen into the upper grips, aligning it vertically within the upper and lower jaws and close the upper grips. Insert the specimen into the lower grips and close. The specimen should be under enough tension to eliminate any slack, but less than 0.025 N of force on the load cell.

Program the tensile tester to perform an extension test, collecting force and extension data at an acquisition rate of 50 Hz as the crosshead raises at a rate of 100 mm/min until the specimen breaks. Start the tensile tester and data collection. Program the software to record Peak Force (N) from the constructed force (N) verses extension (mm) curve. Calculate tensile strength as:

$$\text{Tensile Strength} = \text{Peak Force (N)} / \text{width of specimen (cm)}$$

$$\text{For rope/string like materials: tensile strength} = \text{peak force (N)}$$

Analyze all tensile Specimens. Record Tensile Strength to the nearest 1 N/cm. A total of five test samples are analyzed in like fashion. Calculate and report the average and standard deviation of Tensile Strength to the nearest 1 N/cm for all 5 measured specimens.

**Bending Stiffness**

Bending stiffness is measured using a Lorentzen & Wettre Bending Resistance Tester (BRT) Model SE016 instrument commercially available from Lorentzen & Wettre GmbH, Darmstadt, Germany. Stiffness off the materials (e.g. ligaments and first and second outermost layer) is measured in accordance with SCAN-P 29:69 and corresponding to the requirements according to DIN 53121 (3.1 "Two-point Method"). For analysis a 25.4 mm by 50 mm rectangular specimen was used instead of the 38.1 mm by 50 mm specimen recited in the standard. Therefore, the bending force was specified in mN and the bending resistance was measured according to the formula present below.

The bending stiffness is calculated as follows:

$$S_b = \frac{60 \times F \times l^2}{\pi \times \alpha \times b}$$

with:

$S_b$ = bending stiffness in mNm
$F$ = bending force in N
$l$ = bending length in mm
$\alpha$ = bending angle in degrees
$b$ = sample width in mm

With a die or razor knife, cut a specimen of 25.4 mm by 50 mm whereby the longer portion of the specimen corresponds to the lateral dimension of the material when incorporated into a structure. If the materials are relatively soft, the bending length "l" should be 1 mm. However, if the materials are stiffer such that the load cell capacity is not sufficient any longer for the measurement and indicates "Error", the bending length "l" has to be set at 10 mm. If with a bending length "l" of 10 mm, the load cell again indicates "Error", the bending length "l" may be chosen to be more than 10 mm, such as 20 mm or 30 mm. Alternatively (or in addition, if needed), the bending angle may be reduced from 30° to 10°.

For the ligament data given in Table 1, the bending length "l" was 10 mm. For the 1st and 2nd layer materials the bending length "l" was 1 mm. The bending angel has been 30° for the ligaments as well as for the 1st and 2nd layer.

Precondition the specimen at about 23 °C $\pm$ 2°C and about 45 % $\pm$ 5% relative humidity for two hours prior to testing.

Regarding the size of the ligament and the procedure in case the size of the ligament is smaller than the size of the specimen, the same applies as set out above for the tensile test.

Table 1: Basis weight, tensile strength and bending stiffness of suitable first and second outermost layers materials:

| Example No. | 1 | 2 | 3 | 4 |
|---|---|---|---|---|
| Basis weight of 1st and 2nd layer | 13 g/m$^2$ | 15 g/m$^2$ | 17 g/m$^2$ | 25 g/m$^2$ |
| Bending stiffness of 1st and 2nd layer | 0.3 mNm | 0.4 mNm | 0.5 mNm | 0.9 mNm |
| Tensile strength of 1st and 2nd layer | 6.9 N/cm | 7.9 N/cm | 7.8 N/cm | 8.1 N/cm |

Example materials 1 to 4 are all spunbond polypropylene nonwovens.

**Table 2:** Basis weight, tensile strength and bending stiffness of suitable ligament materials:

| Example No. | 1 | 2 | 3 | 4 |
|---|---|---|---|---|
| Basis weight of ligaments | 40 g/m$^2$ | 43 g/m$^2$ | 51 g/m$^2$ | 60 g/m$^2$ |
| Bending stiffness of ligaments | 10.8 mNm | 36.3 mNm | 52.6 mNm | 105.3 mNm |
| Tensile strength of ligaments | 16.2 N/cm | 16.1 N/cm | 18.8 N/cm | 26.1 N/cm |

Except for Example 1 (40 g/m$^2$ material), all ligament materials were spunbond PET nonwovens. Example 1 was a spunbond polypropylene material.

**Method to measure ligament caliper**

Average Measured caliper is measured using a Mitutoyo Absolute caliper device model ID-C1506, Mitutoyo Corp., Japan.

A sample of the material used for the ligaments with a sample size of 40 mm x 40 mm is cut. If the samples are taken from a ready-made structure and the size of the ligaments is smaller than 40 mm x 40 mm, the sample may be assembled by placing two or more ligaments next to each other with no gap and no overlap between them. Precondition the specimens at about 23 °C $\pm$ 2°C and about 45 % $\pm$ 5% relative humidity for 2 hours prior to testing.

Place the measuring plate on the base blade of the apparatus. Zero the scale when the probe touches the measuring plate (Measuring plate 40 mm diameters, 1.5 mm height and weight of 2.149 g). Place the test piece on the base plate. Place the measurement plate centrally on top of the sample without applying pressure. After 10 sec. move the measuring bar downwards until the probe touches the surface of the measuring plate and read the caliper from the scale to the nearest 0.01 mm.

**Method of measuring modulus of the structure**

The modulus of the structure is measured on a constant rate of structure compression using a tensile tester with computer interface (a suitable instrument is the Zwick Roell Z2.5 using TestExpert 11.0 Software, as available from Zwick Roell GmbH &Co. KG, Ulm, Germany) using a load cell for which the forces measured are within 10% to 90% of the limit of the cell. The movable upper stationary pneumatic jaw is fitted with rubber faced grip to securely clamp the plunger plate (500). The stationary lower jaw is a base plate (510) with dimensions of 100mm x 100mm. The surface of the base plate (510) is perpendicular to the plunger plate (500). To fix the plunger plate (500) to the upper jaw, lower the upper jaw down to 20mm above the upper surface (515) of the base plate (510). Close the upper jaw and make sure the plunger plate (500) is securely tightened. Plunger plate (500) has a width of 3.2mm and a length of 100mm. The edge (520) of the plunger plate (500) which will contact the structure has a curved surface with an impacting edge radius of r = 1.6mm. For analysis, set the gauge length to at least 10% higher than the caliper of the structure in its erected configuration (see Fig 17). Zero the crosshead and load cell. The width of the plunger plate (500) should be parallel with the transverse direction of the structure.

Precondition samples at about 23 °C $\pm$ 2°C and about 45% RH $\pm$ 5% RH relative humidity for 2 hours prior to testing. The structure is placed on the base plate, is transformed into its erected configuration and fixed in its erected configuration with substantially maximum possible caliper to the base plate with the outer surface of its first (lower) layer facing towards the upper surface (515) of the base plate (510). The structure can be fixed to the upper surface of the base plate, e.g. by placing adhesive tapes on the lateral edges of the structure's first (lower) layer and fix the tapes to the upper surface of the base plate.

Program the tensile tester to perform a compression test, collecting force and travel distance data at an acquisition rate of 50 Hz as the crosshead descends at a rate of 50 mm/min from starting position to 2mm above base plate (safety margin to avoid destruction of load cell).

If the modulus of the structure is measured directly in an area where a ligament is placed, the force P [N] is the force when the indentation depth h [mm] of the plunger plate into the structure is equal to 50% of the longitudinal dimension of the free intermediate portion of the ligament below the plunger plate.

If the modulus of the structure is measured between two neighboring ligaments, the force P [N] is the force when the indentation depth h [mm] of the plunger plate into the structure is equal to 50% of the longitudinal dimension of the free intermediate portion of the two ligaments nearest to the plunger plate (i.e. the ligaments on each side of the plunger plate as seen along the longitudinal structure dimension). If the free intermediate portion of the two neighboring ligaments, between which the modulus is measured, differ from each other with respect to the longitudinal dimension of their free intermediate portions, the average value over these two free intermediate portions is calculated and the indentation depth h [mm] of the plunger plate into the structure is equal to 50% of this average free longitudinal dimension.

A total of three test specimens are analyzed in like fashion.

The modulus E [N/mm$^2$] is calculated as follows:

$$E = \frac{3P}{8rh}.$$

With r being the impacting edge radius of the plunger plate, i.e. r = 1.6mm

Calculate and report the average of modulus E for all 3 measured specimens.

All testing is performed in a conditioned room maintained at about 23°C + 2°C and about 45%RH $\pm$ 5% relative humidity.

**Example structures**

*Note: the followihg example structures will generally be too small to be suitable for use as barrier leg cuffs of the present invention. Moreover, no elastic elements are provided in the structure. The examples are mainly intended to exemplify and explain in more detail how a "multi-level cell-forming structure" can be made in general. Changing and adapting the dimensions, material and configuration of the structures described below in order to render them suitable for use in barrier leg cuffs is a matter of simple routine work based on the detailed description provided above.*

Making of Example Structure 1:

Cut two pieces of nonwovens of Example 1 in Table 1, each having a longitudinal dimension of 200 mm and a lateral dimension of 25 mm, with a die or razor knife. These nowovens will become the first and second outermost layers of the Example Structure.

Moreover, cut two pieces of nonwoven of Example 1 in Table 1, each having a longitudinal dimension of 40 mm and a lateral dimension of 25 mm. These nonwovens will become the intermediate layers of Example Structure 1 (i.e. Example Structure 1 has two intermediate layers).

Cut 12 ligaments (for Example Structure 1) of Example 4 nonwoven material (see Table 2 above) each having a longitudinal dimension of 10 mm (which will result in a longitudinal dimension of the free intermediate portion of 4 mm in the final structures, while 3 mm on each lateral edge are attached to the respective first or second outermost layer and intermediate layer) and a lateral dimension of 25 mm, with a die or razor knife. Apply a double sided tape (e.g. 3M Double sided medical tape 1524-3M (44g/m$^2$) available from 3M) with length of 3 mm and width of 25 mm on the first surface of the each ligament adjacent the side edge, which will become the first lateral edge of the ligament in the final structure and a second tape on the second surface of each ligament adjacent the side edge, which will become the second lateral edge of the ligament in the final structure. The width of the tape is aligned with the lateral dimension of the ligaments.

Remove one release tape from four of the ligaments (for Example Structure 1)and attach the tape to the first outermost layer such that the lateral dimension of the ligament is aligned with the lateral dimension of the first outermost layer.

Place the first, second, third and fourth ligaments such that the spacing between neighboring ligaments is 5 mm when the ligaments are lying flat on the first outermost layer.

The ligaments should be positioned accordingly, to leave sufficient space at the lateral edges of the first and second outermost layer to allow attaching the first outermost layer to the second outermost layer in the manner described below.

Remove the remaining release layers from the remaining tape pieces on the ligaments attached to the first outermost layer and place the first intermediate on top of the first outermost layer and the ligaments such that the lateral dimension of the first intermediate layer and of the ligaments is congruent with the lateral dimension of the first outermost layer and such that the first intermediate layer is attached to the remaining tape pieces of the ligaments previously attached to the first outermost layer.

Now attach four ligaments (ligaments of second level) in like fashion to the first intermediate layer on the opposite surface compared to the surface where the first four ligaments have previously been attached. Subsequently, place the second intermediate layer on top of the first intermediate layer and the four ligaments of the second level such that the lateral dimension of the second intermediate layer and of the ligaments is congruent with the lateral dimension of the first outermost layer and such that the second intermediate layer is attached to the remaining tape pieces of the ligaments of the second level which were previously attached to the first intermediate layer.

Attach the remaining four ligaments (ligaments of third level) in like fashion to the second intermediate layer on the opposite surface compared to the surface where the four ligaments of the second level have previously been attached. Subsequently, place the second outermost layer on top of the second intermediate layer and the four ligaments of the third level such that the lateral dimension of the first and second outermost layer, of the first and second intermediate layer and of the ligaments are congruent and such that the second outermost layer is attached to the remaining tape pieces of the ligaments of the third level which were previously attached to the second intermediate layer.

To bond the first outermost layer to the second outermost layer in the areas longitudinally outwardly from the area where the ligaments are placed (stop aid), two double-sided tapes (e.g. 3M Double sided medical tape 1524-3M ($44g/m^2$) available from 3M) having a length of 3 mm and a width of 25 mm are provided. A first tape is attached to the first outermost layer towards one of the first outermost layer's lateral edges such that the distance between this first tape and the lateral edge of the adjacent ligament is 40 mm. The second tape is attached to the first outermost layer towards the respective other lateral edge of the first outermost layer such that the distance between this second tape and the lateral edge of the respective adjacent ligament is 40 mm. The width of the first and second tape is aligned with the lateral dimension of the first outermost layer. Pay attention that the first and second tapes are not attached to the second outermost layer before the structure has been transformed into its erected configuration (see next step).

Extend the resulting multilevel cell forming structure along the longitudinal dimension into the erected configuration such that the first and second outermost layer shift relative to each other and the ligaments move in upright position of 90° relative to the first and second outermost layer. Notably, the 90° does not apply to the area longitudinally outwardly from the outermost ligaments (viewed along the longitudinal dimension) because the first and second outermost layers follow a tapered path in this area until the point where they coincide with each other (see e.g. Fig. 1B). Maintain the structure in its erected configuration and attach the first outermost layer to the second outermost layer via the first and second tape to fix the structure in its erected configuration. Release the force and allow the structure to relax.

Example Structure 1

**Making of Example Structure 2:**

Cut two pieces of nonwovens of Example 1 in Table 1, each having a longitudinal dimension of 200 mm and a lateral dimension of 25 mm, with a die or razor knife. These nowovens will become the first and second outermost layers of the Example Structure.

Moreover, cut one pieces of nonwoven of Example 1 in Table 1, having a longitudinal dimension of 40 mm and a lateral dimension of 25 mm. This nonwoven will become the intermediate layer of Example Structure 2 (i.e. Example Structure 2 has one intermediate layer).

Cut 6 ligaments of Example 4 nonwoven material (see Table 2 above) each having a longitudinal dimension of 10 mm (which will result in a longitudinal dimension of the free intermediate portion of 4 mm in the final structures, while 3 mm on each lateral edge are attached to the respective first or second outermost layer and intermediate layer) and a lateral dimension of 25 mm, with a die or razor knife. Apply a double sided tape (e.g. 3M Double sided medical tape 1524-3M ($44g/m^2$) available from 3M) with length of 3 mm and width of 25 mm on the first surface of the each ligament adjacent the side edge, which will become the first lateral edge of the ligament in the final structure and a second tape on the second surface of each ligament adjacent the side edge, which will become the second lateral edge of the ligament in the final structure. The width of the tape is aligned with the lateral dimension of the ligaments.

Remove one release tape from three of the ligaments and attach the tape to the first outermost layer such that the lateral dimension of the ligament is aligned with the lateral dimension of the first outermost layer.

Place the three ligaments such that the spacing between neighboring ligaments is 5mm when the ligaments are lying flat on the first outermost layer.

The ligaments should be positioned accordingly, to leave sufficient space at the lateral edges of the first and second outermost layer to allow attaching the first outermost layer to the second outermost layer in the manner described below.

Cut 2 pieces of elastic film material with length of 60 mm x 25 mm. The elastic film material is a translucent, colorless latex film available from Modulor GmbH, Berlin, Germany, having a thickness of 0.18 to 0.25 mm and a width of 920 mm (Article Number 813963). The elastic film material has a basis weight of 252 g/m$^2$, a tensile strength of 23.3 N/cm and an Elongation at Break of 859% (tensile strength and Elongation at Break were both determined following the Tensile Strength Test Method set out above).

Place a piece of double side tape with length of 5 mm x 25 mm at each lateral edge of the nonwoven web which will become the first intermediate layer and join one piece of elastic film material on each of the lateral edges of the nonwoven web via the double sided tape, such that one of the shorter edges (25 mm wide) of the elastic film material is attached to the lateral edge of the nonwoven web to form a continuous layer made of elastic film material at its lateral edges and nonwoven web in the center.

Remove the remaining release layers from the tape pieces on the ligaments attached to the first outermost layer and place the first intermediate layer on top of the first outermost layer and the ligaments such that the lateral dimension of the first intermediate layer and of the ligaments is congruent with the lateral dimension of the first outermost layer and such that the central nonwoven web of the first intermediate layer is attached to the remaining tape pieces of the ligaments previously attached to the first outermost layer.

Now attach the remaining three ligaments (ligaments of second level) in like fashion to the first intermediate layer on the opposite surface compared to the surface where the first four ligaments have previously been attached. The ligaments are placed such that they can be attached to the central nonwoven web of the intermediate layer. Subsequently, place the second outermost layer on top of the first intermediate layer and the three ligaments of the second level such that the lateral dimensions of the first and second outermost layer, the intermediate layer and the ligaments are congruent and such that the second outermost layer is attached to the remaining tape pieces of the ligaments of the second level which were previously attached to the first intermediate layer.

To bond the first outermost layer to the second outermost layer in the areas longitudinally outwardly from the area where the ligaments are placed (stop aid), four double-sided tapes (e.g. 3M Double sided medical tape 1524-3M (44g/m$^2$) available from 3M) having a length of 3 mm and a width of 25 mm are provided. A first tape is attached to the first outermost layer towards one of the first outermost layer's lateral edges such that the distance between this first tape and the lateral edge of the adjacent ligament is 40 mm. The second tape is attached to the first outermost layer towards the respective other lateral edge of the first outermost layer such that the distance between this second tape and the lateral edge of the respective adjacent ligament is 40 mm Likewise, the third tape is attached to the second outermost layer towards one of the second outermost layer's lateral edges such that the distance between this first tape and the lateral edge of the adjacent ligament is 40 mm. The fourth tape is attached to the second outermost layer towards the respective other lateral edge of the second outermost layer such that the distance between this second tape and the lateral edge of the respective adjacent ligament is 40 mm. The width of the first, second, third and fourth tape is aligned with the lateral dimension of the first and second outermost layer. Pay attention that the first, second, third and fourth tapes are not attached to the intermediate layer before the structure has been transformed into its erected configuration (see next step).

Extend the resulting multilevel cell forming structure along the longitudinal dimension into the erected configuration such that the first and second outermost layer versus each other and the ligaments move in upright position of 90° relative to the first and second outermost layer. Notably, the 90° does not apply to the area longitudinally outwardly from the outermost ligaments (viewed along the longitudinal dimension) because the first and second outermost layers follow a tapered path in this area until the point where they coincide with each other (see e.g. Fig. 1B). Maintain the structure in its erected configuration and attach the first outermost layer to one surface of the intermediate layer via the first and second tape and attach the second outermost layer to the respective other surface of the intermediate layer via the third and fourth tape to fix the structure in its erected configuration. Release the force and allow the structure to relax.

Structure width = 25 mm
a = 3mm
c = 0.5mm
l = 4mm
d = 40 mm
e = 142mm total length

Example Structure 2

Table 3: Caliper of Example Structures in flat and erected configuration

|  | Example Structure 1 | Example Structure 2 |
|---|---|---|
| Caliper of flat structure | 1.8 mm | 1.2 mm |
| Caliper of erected structure | 13.7 mm | 10.7 mm |

**Claims**

1. An absorbent article (20) having a longitudinal dimension and a transverse dimension and comprising
   a liquid pervious topsheet (24),
   a liquid impervious backsheet (26); and
   an absorbent core (28) disposed between the topsheet (24) and the backsheet (26); the absorbent article (20) further comprising a barrier leg cuff (34) disposed adjacent to each of the longitudinal edges of the absorbent article and extending substantially parallel to the longitudinal dimension of the absorbent article (20);
   each barrier leg cuff (34) comprising a structure (100), wherein the structure (100) is attached to the absorbent article (20) with a proximal end, the structure having a longitudinal dimension, which is substantially parallel with the longitudinal dimension of the absorbent article (20), a lateral dimension and a caliper, and comprising one or more elastic elements (195, 196); and/or the structure (100) being associated with one or more elastic elements;
   wherein the structure is a multi-level structure (100), the multi-level structure comprising a first outermost layer (110), a second outermost layer (120), and at least a first intermediate layer (200),
   all layers having a longitudinal dimension parallel to the longitudinal dimension of the multi-level structure and being confined by first and second spaced apart lateral edges, and a lateral dimension parallel to the lateral dimension of the multi-level structure and being confined by two spaced apart longitudinal edges, wherein the layers at least partly overlap each other; with the at least first intermediate layer (200) being positioned superjacent the first outermost layer (110) and subjacent the second outermost layer (120), with each level of the multi-level structure being confined in a direction perpendicular to the longitudinal and lateral dimension by one of the layers forming the superjacent layer of the level and further confined by another of the layers forming the subjacent layer of the level; the first and second outermost layer (110, 120) being able to shift relative to each other along the longitudinal multi-level structure dimension upon release of a contractive force, which has provided by the stretched one or more elastic elements (195, 16) along the longitudinal dimension, whereby the multi-level structure is converted from an initial flat configuration into an erected configuration in a direction perpendicular to the longitudinal and the lateral dimension,
   each level in the multi-level structure comprising one or more ligament (130), each ligament having a first surface (131) and a second surface (132), a longitudinal dimension confined by two spaced apart lateral ligament edges (134), and a lateral dimension confined by two spaced apart longitudinal ligament edges, each ligament (130) being provided between the superjacent layer and the subjacent layer of the respective level and being attached with a portion at or adjacent to one of the ligament's lateral edges (134) in a first ligament attachment region (135) to the surface of the subjacent layer which faces towards the superjacent layer of the respective level, and being further attached with a portion at or adjacent to the ligament's other lateral edge (134) in a second ligament attachment region (136) to the surface of the superjacent layer which faces towards the subjacent layer of the respective level, the region of each ligament between the first and second ligament attachment region forming a free intermediate portion (137),
   the ligaments in each level being spaced apart from one another along the longitudinal dimension of the multi-level structure when the multi-level structure is in its erected configuration, and the attachment of all ligaments is such that the free intermediate portions of all ligaments in the multi-level structure are able to convert from an initial flat configuration to an erected configuration upon release of a contractive force, which has provided by the stretched one or more elastic elements along the longitudinal dimension of the multi-level structure, thus converting the multi-level structure as a whole from an initial flat configuration into an erected configuration, wherein the erection is in the direction perpendicular to the longitudinal and the lateral dimension of the multi-level structure,
   the structure (100) being in the flat configuration when the absorbent article (20) is flattened out and when the one or more elastic elements (195, 196) are stretched along the longitudinal dimension of the structure (100), whereby contraction of the one or more elastic elements (195, 196) applies a force along the longitudinal dimension of the structure, thus converting the structure (100) into the erected, three-dimensional configuration, wherein the structure (100) has a higher caliper in its erected configuration compared to the caliper of the structure in its flat configuration, the erected structure spacing a distal end of the barrier leg cuff (34) away from the topsheet (24) and causing the barrier leg cuff (34) to stand up.

2. The absorbent article of claim 1, wherein the lateral dimension of the structure (100) is from 3 mm to 50 mm.

3. The absorbent article of any of the preceding claims, wherein each barrier leg cuff (34) further comprises a barrier material (50) partially or fully enwrapping the structure (100).

4. The absorbent article of claim 3, wherein the one or more elastics (195, 196) are comprised by or associated with the barrier material (50).

5. The absorbent article of any of the preceding claims, wherein the outer surface of the first outermost layer is attached to any of the following: the topsheet, the backsheet, and/or the barrier material.

6. The absorbent article of claims 1 to 4, wherein the first outermost layer of the structure is formed by any of the following: a portion of the topsheet, or a portion of the backsheet.

7. The absorbent article of any of the preceding claims, wherein the one or more elastic element(s) form one or more portion(s) of the second outermost layer and, optionally form one or more portion(s) of one or more intermediate layer(s).

8. The absorbent article of any of the preceding claims, wherein the one or more elastic element(s) are attached to the second outermost layer and, optionally are attached to the one or more intermediate layer(s).

9. The absorbent article of any of the preceding claims, wherein the second outermost layer is permanently attached (197) to the first outermost layer, to the topsheet, to the backsheet and/or to the barrier material longitudinally outboard of the second ligament attachment region of the ligament comprised by the level to which the second outermost layer forms the superjacent layer and which is closest to the first lateral edge of the structure and wherein the one or more elastic element(s) at least form (a) portion(s) of the second outermost layer between the second ligament attachment region of the ligament comprised by the level to which the second outermost layer forms the superjacent layer and which is closest to the first lateral edge of the structure and the attachment of the second outermost layer to the first outermost layer, to the topsheet, to the backsheet and/or to the barrier material.

10. The absorbent article of claim 9, wherein the one or more intermediate layer(s) are attached to the one or more elastic element(s) which at least form (a) portion(s) of the second outermost layer between the second ligament attachment region of the ligament comprised by the level to which the second outermost layer forms the superjacent layer and which is closest to the first lateral edge of the structure and the attachment of the second outermost layer to the first outermost layer, to the topsheet, to the backsheet and/or to the barrier material.

11. The absorbent article of any of the preceding claims, wherein the second outermost layer is permanently attached to the first outermost layer, to the topsheet, to the backsheet and/or to the barrier material longitudinally outboard of the second ligament attachment region of the ligament comprised by the level to which the second outermost layer forms the superjacent layer and which is closest to the first lateral edge of the structure and wherein the one or more elastic element(s) is attached to the second outermost layer at least between the second ligament attachment region of the ligament comprised by the level to which the second outermost layer forms the superjacent layer and which is closest to the first lateral edge of the structure and the attachment of the second outermost layer to the first outermost layer, to the topsheet, to the backsheet and/or to the barrier material.

12. The absorbent article of any of the preceding claims, wherein, in the flat configuration of the structure, the second outermost layer is releasably attached (198) to the first outermost layer, to the topsheet, to the backsheet and/or to the barrier material in a location which is on the side of the one or more elastic element(s) along the longitudinal dimension of the structure which is opposite from the side where the second outermost layer is permanently attached to the first outermost layer, to the topsheet, to the backsheet and/or to the barrier material, and wherein release of the releasable attachment enables the one or more elastic element(s) to contract and thereby convert the structure from its flat configuration into its erected configuration.

**13.** The absorbent article of any of the preceding claims, wherein the one or more elastic element(s) form (a) portion(s) of the second outermost layer between adjacent second ligament attachment regions of the ligaments to which the second outermost layer forms the superjacent layer.

**14.** The absorbent article of any of the preceding claims, wherein the one or more elastic element(s) are attached to the second outermost layer between adjacent second ligament attachment regions of the ligaments to which the second outermost layer forms the superjacent layer.

**15.** The absorbent article of claim 13 or 14, wherein the second outermost layer is releasably attached to the first outermost layer, to the topsheet, to the backsheet and/or to the barrier material on both sides of the one or more elastic element(s) along the longitudinal dimension of the structure and wherein release of the releasable attachments enables the one or more elastic element(s) to contract and thereby convert the structure from its flat configuration into its erected configuration.

**16.** The absorbent article of any of claims 13 to 15, wherein one or more elastic element(s) form (a) portion(s) of the one or more intermediate layer(s) between adjacent second ligament attachment regions of the ligaments to which the intermediate layer forms the superjacent layer and/or the subjacent layer.

**17.** The absorbent article of any of the preceding claims, wherein, in the structure, the ligaments, in their erected configuration, either adopt a Z-like shape, a C-like shape, a T-like shape, or a Double-T-like shape.

**18.** The absorbent article of any of the preceding claims, wherein the structure comprises one or more stop aid(s) (180, 190) which define(s) the maximum shifting of the first outermost layer relative to the second outermost layer when the force along the longitudinal dimension is applied due to contraction of the one or more elastic elements, wherein the maximum shifting defined by the stop aid is less than the maximum shifting provided by the ligaments in the absence of such stop aid.

**19.** The absorbent article of claim 18, wherein the one or more stop aid is selected from the group consisting of:

a) a layer-to-layer stop aid (180) extending from the first outermost layer to the second outermost layer and being attached to the first outermost layer in a first layer-to-layer stop aid attachment region and being further attached to the second outermost layer in a second layer-to-layer stop aid attachment region, wherein the layer-to-layer stop aid is provided with a layer-to-layer stop aid leeway between the first layer-to-layer stop aid attachment region and the second layer-to-layer stop aid attachment region when the multi-level structure is in its initial flat configuration, said leeway being able to straighten out and/or extend when the multi-level structure is transferred into its erected configuration; and

b) a layer-to-ligament stop aid (190) extending from the first or second outermost layer to one of the ligaments comprised by a level which is farthest away from the respective first or second outermost layer, and being attached to the first or second outermost layer in a first layer-to-ligament stop aid attachment region and being attached to the ligament in a second layer-to-ligament stop aid attachment region, wherein the layer-to-ligament stop aid is provided with a layer-to-ligament stop aid leeway between the first layer-to-ligament stop aid attachment region and the second layer-to-ligament stop aid attachment region when the multi-level structure is in its initial flat configuration, said leeway being able to straighten out and/or extend when the multi-level structure is transferred into its erected configuration; and

c) an enveloping stop aid encircling a least a portion of the first and second outermost layer, of the intermediate layer(s) and of the ligaments between the first and second outermost layer, wherein the enveloping stop aid is attached to the first outermost layer, the second outermost layer, one or more of the intermediate layer(s) and/or one or more ligaments in at least one enveloping stop aid attachment region and wherein the enveloping stop aid is further attached to itself to form a closed loop with a defined circumference around a least a portion of the first and second outermost layer, wherein the circumference of the enveloping stop aid defines the maximum caliper of the multi-level structure in its erected configuration; wherein the caliper is perpendicular to the lateral and longitudinal dimension of the multi-level structure; and

d) any combinations of a) to c).

**20.** The absorbent article of any of the preceding claims, wherein, in the structure, the areas where the ligaments are located and the areas between neighboring ligaments have a modulus of from about 0.01 N/mm$^2$ to about 0.3 N/mm$^2$, measured according to the test method set out herein.

**EP 3 067 027 B1**

**Patentansprüche**

1.  Absorptionsartikel (20) mit einer Längsabmessung und einer Querabmessung und umfassend eine flüssigkeitsdurchlässige Oberschicht (24),

    eine flüssigkeitsundurchlässige Unterschicht (26); und

    einen Absorptionskern (28), der zwischen der Oberschicht (24) und der Unterschicht (26) angeordnet ist;

    wobei der Absorptionsartikel (20) ferner ein Bein-Barrierebündchen (34) umfasst, das angrenzend an jeden der Längsränder des Absorptionsartikels angeordnet ist und sich im Wesentlichen parallel zu der Längsabmessung des Absorptionsartikels (20) erstreckt;

    wobei jedes Bein-Barrierebündchen (34) eine Struktur (100) umfasst, wobei die Struktur (100) mit einem proximalen Ende an dem Absorptionsartikel (20) befestigt ist, wobei die Struktur eine Längsabmessung, die im Wesentlichen parallel zu der Längsabmessung des Absorptionsartikels (20) ist, eine seitliche Abmessung und eine Dicke aufweist und ein oder mehrere elastische Elemente (195, 196) umfasst; und/oder wobei die Struktur (100) mit einem oder mehreren elastischen Elementen verbunden ist;

    wobei die Struktur eine Mehrebenenstruktur (100) ist, wobei die Mehrebenenstruktur eine erste äußerste Schicht (110), eine zweite äußerste Schicht (120) und wenigstens eine erste Zwischenschicht (200) umfasst,

    wobei alle Schichten eine Längsabmessung, die parallel zu der Längsabmessung der Mehrebenenstruktur ist und durch einen ersten und einen zweiten beabstandeten Seitenrand begrenzt wird, und eine seitliche Abmessung, die parallel zu der seitlichen Abmessung der Mehrebenenstruktur ist und durch zwei beabstandete Längsränder begrenzt wird, aufweisen, wobei die Schichten wenigstens teilweise einander überlappen; wobei die wenigstens erste Zwischenschicht (200) über der ersten äußersten Schicht (110) liegend und unter der zweiten äußersten Schicht (120) liegend angeordnet ist, wobei jede Ebene der Mehrebenenstruktur in einer Richtung, die lotrecht zu der Längsabmessung und der seitlichen Abmessung ist, durch eine der Schichten begrenzt wird, welche die überliegende Schicht der Ebene bildet, und ferner durch eine andere der Schichten begrenzt wird, welche die unterliegende Schicht der Ebene bildet;

    wobei die erste und die zweite äußerste Schicht (110, 120) in der Lage sind, sich bei Aufhebung einer Kontraktionskraft, die durch das eine oder die mehreren gedehnten elastischen Elemente (195, 16) entlang der Längsabmessung bereitgestellt wurde, entlang der Längsabmessung der Mehrebenenstruktur relativ zueinander zu verschieben, wodurch die Mehrebenenstruktur in einer Richtung, die lotrecht zu der Längsabmessung und der seitlichen Abmessung ist, von einer anfänglichen flachen Konfiguration in eine aufrechte Konfiguration übergeführt wird,

    wobei jede Ebene in der Mehrebenenstruktur ein oder mehrere Bänder (130) umfasst, wobei jedes Band eine erste Oberfläche (131) und eine zweite Oberfläche (132), eine Längsabmessung, die durch zwei beabstandete seitliche Bandränder (134) begrenzt wird, und eine seitliche Abmessung, die durch zwei beabstandete Längsbandränder begrenzt wird, aufweist, wobei jedes Band (130) zwischen der überliegenden Schicht und der unterliegenden Schicht der jeweiligen Ebene bereitgestellt ist und in einem ersten Bandbefestigungsbereich (135) mit einem Abschnitt an oder angrenzend an einen der Seitenränder (134) des Bands an der Oberfläche der unterliegenden Schicht befestigt ist, die der überliegenden Schicht der jeweiligen Ebene zugewandt ist, und ferner in einem zweiten Bandbefestigungsbereich (136) mit einem Abschnitt an oder angrenzend an den anderen Seitenrand (134) des Bands an der Oberfläche der überliegenden Schicht befestigt ist, die der unterliegenden Schicht der jeweiligen Ebene zugewandt ist, wobei der Bereich jedes Bands zwischen dem ersten und dem zweiten Bandbefestigungsbereich einen freien Zwischenabschnitt (137) bildet,

    wobei die Bänder in jeder Ebene entlang der Längsabmessung der Mehrebenenstruktur voneinander beabstandet sind, wenn die Mehrebenenstruktur sich in ihrer aufrechten Konfiguration befindet, und die Befestigung aller Bänder derart ist, dass die freien Zwischenabschnitte aller Bänder in der Mehrebenenstruktur bei Aufhebung einer Kontraktionskraft, die durch das eine oder die mehreren gedehnten elastischen Elemente entlang der Längsabmessung der Mehrebenenstruktur bereitgestellt wurde, von einer anfänglichen flachen Konfiguration in eine aufrechte Konfiguration übergeführt werden können, wodurch die Mehrebenenstruktur als Ganzes von einer anfänglichen flachen Konfiguration in eine aufrechte Konfiguration übergeführt wird, wobei die Aufrichtung in der Richtung erfolgt, die lotrecht zu der Längsabmessung und der seitlichen Abmessung der Mehrebenenstruktur ist,

    wobei sich die Struktur (100) in der flachen Konfiguration befindet, wenn der Absorptionsartikel (20) flachgedrückt wird und wenn das eine oder die mehreren elastischen Elemente (195, 196) entlang der Längsabmessung der Struktur (100) gedehnt werden, wobei eine Kontraktion des einen oder der mehreren elastischen Elemente (195, 196) eine Kraft entlang der Längsabmessung der Struktur ausübt, wodurch die Struktur (100) in die aufrechte, dreidimensionale Konfiguration übergeführt wird, wobei die Struktur (100) in ihrer aufrechten Konfiguration eine höhere Dicke im Vergleich zu der Dicke der Struktur in ihrer flachen Konfiguration besitzt, wobei die aufrechte Struktur ein distales Ende des Bein-Barrierebündchens (34) von der Oberschicht (24) weg beabstandet und bewirkt, dass sich das Bein-Barrierebündchen (34) aufrichtet.

34

**2.** Absorptionsartikel nach Anspruch 1, wobei die seitliche Abmessung der Struktur (100) von 3 mm bis 50 mm beträgt.

**3.** Absorptionsartikel nach einem der vorstehenden Ansprüche, wobei jedes Bein-Barrierebündchen (34) ferner ein Barrierematerial (50) umfasst, das die Struktur (100) teilweise oder vollständig umhüllt.

**4.** Absorptionsartikel nach Anspruch 3, wobei das eine oder die mehreren elastischen Elemente (195, 196) in dem Barrierematerial (50) enthalten oder damit verbunden sind.

**5.** Absorptionsartikel nach einem der vorstehenden Ansprüche, wobei die Außenoberfläche der ersten äußersten Schicht an einem von Folgendem befestigt ist: der Oberschicht, der Unterschicht und/oder dem Barrierematerial.

**6.** Absorptionsartikel nach den Ansprüchen 1 bis 4, wobei die erste äußerste Schicht der Struktur durch eines von Folgendem gebildet wird: einem Abschnitt der Oberschicht oder einem Abschnitt der Unterschicht.

**7.** Absorptionsartikel nach einem der vorstehenden Ansprüche, wobei das eine oder die mehreren elastischen Elemente einen oder mehrere Abschnitte der zweiten äußersten Schicht bilden und wahlweise einen oder mehrere Abschnitte von einer oder mehreren Zwischenschichten bilden.

**8.** Absorptionsartikel nach einem der vorstehenden Ansprüche, wobei das eine oder die mehreren elastischen Elemente an der zweiten äußersten Schicht befestigt sind und wahlweise an der einen oder den mehreren Zwischenschichten befestigt sind.

**9.** Absorptionsartikel nach einem der vorstehenden Ansprüche, wobei die zweite äußerste Schicht längs außerhalb des zweiten Bandbefestigungsbereichs des Bands, das in der Ebene enthalten ist, zu der die zweite äußerste Schicht die überliegende Schicht bildet und die am nächsten an dem ersten Seitenrand der Struktur liegt, an der ersten äußersten Schicht, an der Oberschicht, an der Unterschicht und/oder an dem Barrierematerial permanent befestigt ist (197) und wobei das eine oder die mehreren elastischen Elemente wenigstens (a) einen Abschnitt bzw. Abschnitte der zweiten äußersten Schicht zwischen dem zweiten Bandbefestigungsbereich des Bands, das in der Ebene enthalten ist, zu der die zweite äußerste Schicht die überliegende Schicht bildet und die am nächsten an dem ersten Seitenrand der Struktur liegt, und der Befestigung der zweiten äußersten Schicht an der ersten äußersten Schicht, an der Oberschicht, an der Unterschicht und/oder an dem Barrierematerial bilden.

**10.** Absorptionsartikel nach Anspruch 9, wobei die eine oder mehreren Zwischenschichten an dem einen oder den mehreren elastischen Elementen befestigt sind, die wenigstens (a) einen Abschnitt bzw. Abschnitte der zweiten äußersten Schicht zwischen dem zweiten Bandbefestigungsbereich des Bands, das in der Ebene enthalten ist, zu der die zweite äußerste Schicht die überliegende Schicht bildet und die am nächsten an dem ersten Seitenrand der Struktur liegt, und der Befestigung der zweiten äußersten Schicht an der ersten äußersten Schicht, an der Oberschicht, an der Unterschicht und/oder an dem Barrierematerial bilden.

**11.** Absorptionsartikel nach einem der vorangehenden Ansprüche, wobei die zweite äußerste Schicht längs außerhalb des zweiten Bandbefestigungsbereichs des Bands, das in der Ebene enthalten ist, zu der die zweite äußerste Schicht die überliegende Schicht bildet und die am nächsten an dem ersten Seitenrand der Struktur liegt, an der ersten äußersten Schicht, an der Oberschicht, an der Unterschicht und/oder an dem Barrierematerial permanent befestigt ist und wobei das eine oder die mehreren elastischen Elemente wenigstens zwischen dem zweiten Bandbefestigungsbereich des Bands, das in der Ebene enthalten ist, zu der die zweite äußerste Schicht die überliegende Schicht bildet und die am nächsten an dem ersten Seitenrand der Struktur liegt, und der Befestigung der zweiten äußersten Schicht an der ersten äußersten Schicht, an der Oberschicht, an der Unterschicht und/oder an dem Barrierematerial an der zweiten äußersten Schicht befestigt sind.

**12.** Absorptionsartikel nach einem der vorangehenden Ansprüche, wobei in der flachen Konfiguration der Struktur die zweite äußerste Schicht an einer Stelle, die auf der Seite des einen oder der mehreren elastischen Elemente entlang der Längsabmessung der Struktur liegt, die der Seite gegenüberliegt, auf der die zweite äußerste Schicht an der ersten äußersten Schicht, an der Oberschicht, an der Unterschicht und/oder an dem Barrierematerial permanent befestigt ist, lösbar an der ersten äußersten Schicht, an der Oberschicht, an der Unterschicht und/oder an dem Barrierematerial befestigt ist (198), und wobei es das Lösen der lösbaren Befestigung dem einen oder den mehreren elastischen Elementen ermöglicht, sich zu kontrahieren und dadurch die Struktur von ihrer flachen Konfiguration in ihre aufrechte Konfiguration überzuführen.

**13.** Absorptionsartikel nach einem der vorangehenden Ansprüche, wobei das eine oder die mehreren elastischen Elemente (a) einen Abschnitt bzw. Abschnitte der zweiten äußersten Schicht zwischen benachbarten zweiten Bandbefestigungsbereichen der Bänder bilden, zu denen die zweite äußerste Schicht die überliegende Schicht bildet.

**14.** Absorptionsartikel nach einem der vorangehenden Ansprüche, wobei das eine oder die mehreren elastischen Elemente zwischen benachbarten zweiten Bandbefestigungsbereichen der Bänder, zu denen die zweite äußerste Schicht die überliegende Schicht bildet, an der zweiten äußersten Schicht befestigt sind.

**15.** Absorptionsartikel nach Anspruch 13 oder 14, wobei die zweite äußerste Schicht auf beiden Seiten des einen oder der mehreren elastischen Elemente entlang der Längsabmessung der Struktur an der ersten äußersten Schicht, an der Oberschicht, an der Unterschicht und/oder an dem Barrierematerial lösbar befestigt ist und wobei es das Lösen der lösbaren Befestigungen dem einen oder den mehreren elastischen Elementen ermöglicht, sich zu kontrahieren und dadurch die Struktur von ihrer flachen Konfiguration in ihre aufrechte Konfiguration überzuführen.

**16.** Absorptionsartikel nach einem der Ansprüche 13 bis 15, wobei ein oder mehrere elastische Elemente (a) einen Abschnitt bzw. Abschnitte der einen oder mehreren Zwischenschichten zwischen benachbarten zweiten Bandbefestigungsbereichen der Bänder bilden, zu denen die Zwischenschicht die überliegende Schicht und/oder die unterliegende Schicht bildet.

**17.** Absorptionsartikel nach einem der vorstehenden Ansprüche, wobei in der Struktur die Bänder in ihrer aufrechten Konfiguration entweder eine Z-ähnliche Form, eine C-ähnliche Form, eine T-ähnliche Form oder eine Doppel-T-ähnliche Form annehmen.

**18.** Absorptionsartikel nach einem der vorstehenden Ansprüche, wobei die Struktur ein oder mehrere Stopphilfen (180, 190) umfasst, welche die maximale Verschiebung der ersten äußersten Schicht relativ zu der zweiten äußersten Schicht entlang der Längsabmessung bestimmen, wenn die Kraft entlang der Längsabmessung aufgrund der Kontraktion des einen oder der mehreren elastischen Elemente ausgeübt wird, wobei die durch das Stopphilfen bestimmte maximale Verschiebung geringer ist als die maximale Verschiebung, die durch die Bänder bei Nichtvorhandensein einer solchen Stopphilfe bereitgestellt wird.

**19.** Absorptionsartikel nach Anspruch 18, wobei die eine oder die mehreren Stopphilfen ausgewählt sind aus der Gruppe bestehend aus:

a) einer Schicht-zu-Schicht-Stopphilfe (180), die sich von der ersten äußersten Schicht zu der zweiten äußersten Schicht erstreckt und in einem ersten Schicht-zu-Schicht-Stopphilfe-Befestigungsbereich an der ersten äußersten Schicht befestigt ist und ferner in einem zweiten Schicht-zu-Schicht-Stopphilfe-Befestigungsbereich an der zweiten äußersten Schicht befestigt ist, wobei die Schicht-zu-Schicht-Stopphilfe mit einem Schicht-zu-Schicht-Stopphilfe-Spielraum zwischen dem ersten Schicht-zu-Schicht-Stopphilfe-Befestigungsbereich und dem zweiten Schicht-zu-Schicht-Stopphilfe-Befestigungsbereich versehen ist, wenn die Mehrebenenstruktur sich in ihrer anfänglichen flachen Konfiguration befindet, wobei der Spielraum in der Lage ist, sich gerade auszurichten und/oder auszudehnen, wenn die Mehrebenenstruktur in ihre aufrechte Konfiguration übergeführt wird; und

b) einer Schicht-zu-Band-Stopphilfe (190), die sich von der ersten oder der zweiten äußersten Schicht zu einem der Bänder erstreckt, das in einer Ebene enthalten ist, die am weitesten von der jeweiligen ersten oder zweiten äußersten Schicht entfernt liegt, und in einem ersten Schicht-zu-Band-Stopphilfe-Befestigungsbereich an der ersten oder der zweiten äußersten Schicht befestigt ist und in einem zweiten Schicht-zu-Band-Stopphilfe-Befestigungsbereich an dem Band befestigt ist, wobei die Schicht-zu-Band-Stopphilfe mit einem Schicht-zu-Band-Stopphilfe-Spielraum zwischen dem ersten Schicht-zu-Band-Stopphilfe-Befestigungsbereich und dem zweiten Schicht-zu-Band-Stopphilfe-Befestigungsbereich versehen ist, wenn die Mehrebenenstruktur sich in ihrer anfänglichen flachen Konfiguration befindet, wobei der Spielraum in der Lage ist, sich gerade auszurichten und/oder auszudehnen, wenn die Mehrebenenstruktur in ihre aufrechte Konfiguration übergeführt wird; und

c) einer umhüllenden Stopphilfe, die wenigstens einen Abschnitt der ersten und der zweiten äußersten Schicht, der Zwischenschicht bzw. Zwischenschichten und der Bänder zwischen der ersten und der zweiten äußersten Schicht umgibt, wobei die umhüllende Stopphilfe in mindestens einem Befestigungsbereich der umhüllenden Stopphilfe an der ersten äußersten Schicht, der zweiten äußersten Schicht, einer oder mehreren der Zwischenschichten und/oder einem oder mehreren Bändern befestigt ist und wobei die umhüllende Stopphilfe ferner an sich selbst befestigt ist, um eine geschlossene Schleife mit einem definierten Umfang um wenigstens einen Abschnitt der ersten und der zweiten äußersten Schicht zu bilden, wobei der Umfang der umhüllenden Stopphilfe die maximale Dicke der Mehrebenenstruktur in ihrer aufrechten Konfiguration bestimmt; wobei die Dicke lotrecht zu der seitlichen und der Längsabmessung der Mehrebenenstruktur ist; und

d) beliebigen Kombinationen von a) bis c).

**20.** Absorptionsartikel nach einem der vorstehenden Ansprüche, wobei in der Struktur die Bereiche, in denen die Bänder angeordnet sind, und die Bereiche zwischen benachbarten Bändern einen Modul von etwa 0,01 N/mm$^2$ bis etwa 0,3 N/mm$^2$, gemessen nach dem hierin dargelegten Prüfverfahren, aufweisen.

**Revendications**

**1.** Article absorbant (20) ayant une dimension longitudinale et une dimension transversale et comprenant

une feuille de dessus perméable aux liquides (24),

une feuille de fond imperméable aux liquides (26) ; et

une âme absorbante (28) disposée entre la feuille de dessus (24) et la feuille de fond (26) ;

l'article absorbant (20) comprenant en outre un revers de jambe de barrière (34) disposé adjacent à chacun des bords longitudinaux de l'article absorbant et s'étendant essentiellement parallèle à la dimension longitudinale de l'article absorbant (20) ;

chaque revers de jambe de barrière (34) comprenant une structure (100), dans lequel la structure (100) est fixée à l'article absorbant (20) avec une extrémité proximale, la structure ayant une dimension longitudinale, qui est essentiellement parallèle à la dimension longitudinale de l'article absorbant (20), une dimension latérale et une épaisseur, et comprenant un ou plusieurs éléments élastiques (195, 196) ; et/ou la structure (100) étant associée à un ou plusieurs éléments élastiques ;

dans lequel la structure est une structure multiniveau (100), la structure multiniveau comprenant une première couche externe (110), une deuxième couche externe (120) et au moins une première couche intermédiaire (200) toutes les couches ayant une dimension longitudinale parallèle à la dimension longitudinale de la structure multiniveau et étant confinée par des premier et deuxième bords latéraux espacés, et une dimension latérale parallèle à la dimension latérale de la structure multiniveau et étant confinée par deux bords longitudinaux espacés, dans lequel les couches se chevauchent au moins partiellement l'une l'autre ; avec au moins la première couche intermédiaire (200) étant positionnée surjacente à la première couche externe (110) et sous-jacente à la deuxième couche externe (120), avec chaque niveau de la structure multiniveau confiné dans une direction perpendiculaire à la dimension longitudinale et latérale par une des couches formant la couche surjacente du niveau et confiné en outre par une autre des couches formant la couche sous-jacente du niveau ;

les première et deuxième couches externes (110, 120) étant capables de se décaler l'une par rapport à l'autre le long de la dimension longitudinale de structure multiniveau lors de la libération d'une force de contraction, qui est conférée par le ou les éléments élastiques étirés (195, 16) le long de la dimension longitudinale, de telle manière que la structure multiniveau passe d'une configuration plate initiale à une configuration dressée dans une direction perpendiculaire à la dimension longitudinale et à la dimension latérale,

chaque niveau dans la structure multiniveau comprenant un ou plusieurs ligaments (130), chaque ligament possédant une première surface (131) et une deuxième surface (132), une dimension longitudinale confinée par deux bords latéraux de ligament espacés (134), et une dimension latérale confinée par deux bords longitudinaux de ligament espacés, chaque ligament (130) étant fourni entre la couche surjacente et la couche sous-jacente du niveau respectif et étant fixé avec une partie au niveau de, ou adjacente à, un des bords latéraux (134) du ligament dans une première région d'attachement de ligament (135) à la surface de la couche sous-jacente qui fait face à la couche surjacente du niveau respectif, et étant en outre fixé avec une partie au niveau de, ou adjacente à l'autre bord latéral (134) du ligament dans une deuxième région d'attachement de ligament (136) à la surface de la couche surjacente qui fait face à la couche sous-jacente du niveau respectif, la région de chaque ligament entre les première et deuxième régions d'attachement de ligament formant une partie intermédiaire libre (137),

les ligaments dans chaque niveau étant espacés les uns des autres le long de la dimension longitudinale de la structure multiniveau lorsque la structure multiniveau se trouve dans sa configuration dressée, et l'attachement de tous les ligaments est tel que les parties intermédiaires libres de tous les ligaments dans la structure multiniveau sont aptes à passer d'une configuration plate initiale à une configuration dressée lors de la libération d'une force de contraction, qui est conférée par le ou les éléments élastiques étirés le long de la dimension longitudinale de la structure multiniveau, faisant ainsi passer la structure multiniveau dans son ensemble d'une configuration plate initiale à une configuration dressée, dans lequel le redressement est dans la direction perpendiculaire à la dimension longitudinale et à la dimension latérale de la structure multiniveau,

la structure (100) étant dans une configuration plate lorsque l'article absorbant (20) est aplati et lorsque le ou les éléments élastiques (195, 196) sont étirés le long de la dimension longitudinale de la structure (100), de telle manière qu'une contraction du ou des éléments élastiques (195, 196) applique une force le long de la dimension longitudinale de la structure, transformant ainsi la structure (100) en une configuration tridimensionnelle dressée, dans lequel la structure (100) a une épaisseur plus élevée dans sa configuration dressée par comparaison avec l'épaisseur de la structure dans sa configuration plate, la structure dressée espaçant une extrémité distale du revers de jambe de barrière (34) à l'écart de la feuille de dessus (24) et faisant en sorte que le revers de jambe de barrière (34) se dresse.

2. Article absorbant selon la revendication 1, dans lequel la dimension latérale de la structure (100) va de 3 mm à 50 mm.

3. Article absorbant selon l'une quelconque des revendications précédentes, dans lequel chaque revers de jambe de barrière (34) comprend en outre un matériau de barrière (50) enveloppant partiellement ou complètement la structure (100).

4. Article absorbant selon la revendication 3, dans lequel le ou les élastiques (195,196) sont compris par le, ou associés au, matériau de barrière (50).

5. Article absorbant selon l'une quelconque des revendications précédentes, dans lequel la surface externe de la première couche externe est fixée à n'importe lequel des éléments suivants : la feuille de dessus, la feuille de fond et/ou le matériau de barrière.

6. Article absorbant selon les revendications 1 à 4, dans lequel la première couche externe de la structure est formée par n'importe lequel des éléments suivants : une partie de la feuille de dessus ou une partie de la feuille de fond.

7. Article absorbant selon l'une quelconque des revendications précédentes, dans lequel le ou les élément(s) élastique(s) forment une ou plusieurs partie(s) de la deuxième couche externe et, éventuellement forment une ou plusieurs partie(s) d'une ou plusieurs couche(s) intermédiaire(s).

8. Article absorbant selon l'une quelconque des revendications précédentes, dans lequel le ou les élément(s) élastique(s) sont fixés à la deuxième couche externe et, éventuellement, sont fixés à la ou aux couche(s) intermédiaire(s).

9. Article absorbant selon l'une quelconque des revendications précédentes, dans lequel la deuxième couche externe est fixée de manière permanente (197) à la première couche externe, à la feuille de dessus, à la feuille de fond et/ou au matériau de barrière longitudinalement à l'extérieur de la deuxième région d'attachement de ligament du ligament compris par le niveau auquel la deuxième couche externe forme la couche surjacente et qui est le plus proche du premier bord latéral de la structure et dans lequel le ou les élément(s) élastique(s) forment au moins une ou des partie(s) de la deuxième couche externe entre la deuxième région d'attachement de ligament du ligament compris par le niveau auquel la deuxième couche externe forme la couche surjacente et qui est le plus proche du premier bord latéral de la structure et l'attachement de la deuxième couche externe à la première couche externe, à la feuille de dessus, à la feuille de fond et/ou au matériau de barrière.

10. Article absorbant selon la revendication 9, dans lequel la ou les couche(s) intermédiaire(s) sont fixées à l'élément élastique ou aux éléments élastiques qui forment au moins une ou des partie(s) de la deuxième couche externe entre la deuxième région d'attachement de ligament du ligament compris par le niveau auquel la deuxième couche externe forme la couche surjacente et qui est le plus proche du premier bord latéral de la structure et l'attachement de la deuxième couche externe à la première couche externe, à la feuille de dessus, à la feuille de fond et/ou au matériau de barrière.

11. Article absorbant selon l'une quelconque des revendications précédentes, dans lequel la deuxième couche externe est fixée de manière permanente à la première couche externe, à la feuille de dessus, à la feuille de fond et/ou au matériau de barrière longitudinalement à l'extérieur de la deuxième région d'attachement de ligament du ligament compris par le niveau auquel la deuxième couche externe forme la couche surjacente et qui est le plus proche du premier bord latéral de la structure et dans lequel le ou les élément(s) élastique(s) sont fixés à la deuxième couche externe au moins entre la deuxième région d'attachement de ligament du ligament compris par le niveau auquel la deuxième couche externe forme la couche surjacente et qui est le plus proche du premier bord latéral de la structure et l'attachement de la deuxième couche externe à la première couche externe, à la feuille de dessus, à la feuille de fond et/ou au matériau de barrière.

12. Article absorbant selon l'une quelconque des revendications précédentes, dans lequel, dans la configuration plate de la structure, la deuxième couche externe est fixée de façon libérable (198) à la première couche externe, à la feuille de dessus, à la feuille de fond et/ou au matériau de barrière à un endroit qui se trouve sur le côté du ou des élément(s) élastique(s) le long de la dimension longitudinale de la structure qui est opposée au côté où la deuxième couche externe est fixée de manière permanente à la première couche externe, à la feuille de dessus, à la feuille de fond et/ou au matériau de barrière, et dans lequel une libération de l'attachement libérable permet à l'élément élastique ou aux éléments élastiques de se contracter et de faire passer de ce fait la structure de sa configuration plate à sa configuration dressée.

13. Article absorbant selon l'une quelconque des revendications précédentes, dans lequel le ou les élément(s) élastique(s) forment une ou des partie(s) de la deuxième couche externe entre des deuxièmes régions d'attachement de ligament adjacentes des ligaments auxquels la deuxième couche externe forme la couche surjacente.

14. Article absorbant selon l'une quelconque des revendications précédentes, dans lequel le ou les élément(s) sont fixés à la deuxième couche externe entre des deuxièmes régions d'attachement de ligament adjacentes des ligaments auxquels la deuxième couche externe forme la couche surjacente.

15. Article absorbant selon la revendication 13 ou 14, dans lequel la deuxième couche externe est fixée de façon libérable à la première couche externe, à la feuille de dessus, à la feuille de fond et/ou au matériau de barrière sur l'un et l'autre des côtés du ou des élément(s) élastique(s) le long de la dimension longitudinale de la structure et dans lequel la libération des attachements libérables permet à l'élément élastique ou aux éléments élastiques de se contracter et de faire passer de ce fait la structure de sa configuration plate à sa configuration dressée.

16. Article absorbant selon l'une quelconque des revendications 13 à 15, dans lequel un ou plusieurs élément(s) élastique(s) forment une ou des partie(s) de la ou des couche(s) intermédiaire(s) entre les deuxièmes régions d'attachement de ligament adjacentes des ligaments auxquels la couche intermédiaire forme la couche surjacente et/ou la couche sous-jacente.

17. Article absorbant selon l'une quelconque des revendications précédentes, dans lequel, dans la structure, les ligaments, dans leur configuration dressée, adoptent l'une ou l'autre forme parmi une forme de type Z, une forme de type C, une forme de type T, ou une forme de type double-T.

18. Article absorbant selon l'une quelconque des revendications précédentes, dans lequel la structure comprend un ou plusieurs auxiliaire(s) d'arrêt (180,190) qui défini(ssen)t le décalage maximal de la première couche externe par rapport à la deuxième couche externe lorsque la force le long de la dimension longitudinale est appliquée du fait de la contraction du ou des éléments élastiques, dans lequel le décalage maximal défini par l'auxiliaire d'arrêt est inférieur au décalage maximal conféré par les ligaments en l'absence d'un tel auxiliaire d'arrêt.

19. Article absorbant selon la revendication 18, dans lequel le ou les auxiliaires d'arrêt sont choisis dans le groupe constitué de :

a) un auxiliaire d'arrêt de couche à couche (180) s'étendant de la première couche externe à la deuxième couche externe et étant fixé à la première couche externe dans une première région d'attachement d'auxiliaire d'arrêt de couche à couche et étant en outre fixé à la deuxième couche externe dans une deuxième région d'attachement d'auxiliaire d'arrêt de couche à couche, dans lequel l'auxiliaire d'arrêt de couche à couche est pourvu d'une marge d'auxiliaire d'arrêt de couche à couche entre la première région d'attachement d'auxiliaire d'arrêt de couche à couche et la deuxième région d'attachement d'auxiliaire d'arrêt de couche à couche lorsque la structure multiniveau se trouve dans sa configuration plate initiale, ladite marge étant apte à se redresser et/ou à s'étendre lorsque la structure multiniveau est transférée dans sa configuration dressée ; et
b) un auxiliaire d'arrêt de couche à ligament (190) s'étendant de la première ou deuxième couche externe à un des ligaments compris par un niveau qui est le plus éloigné de la première ou deuxième couche externe respective, et étant fixé à la première ou deuxième couche externe dans une première région d'attachement d'auxiliaire d'arrêt de couche à ligament et étant fixé au ligament dans une deuxième région d'attachement d'auxiliaire d'arrêt de couche à ligament, dans lequel l'auxiliaire d'arrêt de couche à ligament est pourvu d'une marge d'auxiliaire d'arrêt de couche à ligament entre la première région d'attachement d'auxiliaire d'arrêt de couche à ligament et la deuxième région d'attachement d'auxiliaire d'arrêt de couche à ligament lorsque la structure multiniveau se trouve dans sa configuration plate initiale, ladite marge étant apte à se redresser et/ou à s'étendre lorsque la structure multiniveau est transférée dans sa configuration dressée ; et
c) un auxiliaire d'arrêt enveloppant entourant au moins une partie des première et deuxième couches externes, de la ou des couche(s) intermédiaire(s) et des ligaments entre les première et deuxième couches externes, dans lequel l'auxiliaire d'arrêt enveloppant est fixé à la première couche externe, à la deuxième couche externe, à une ou plusieurs de la ou des couche(s) intermédiaire(s) et/ou à un ou plusieurs ligaments dans au moins une région d'attachement d'auxiliaire d'arrêt enveloppant et dans lequel l'auxiliaire d'arrêt enveloppant est en outre fixé à lui-même pour former une boucle fermée avec une circonférence définie autour d'au moins une partie des première et deuxième couches externes, dans lequel la circonférence de l'auxiliaire d'arrêt enveloppant définit l'épaisseur maximale de la structure multiniveau dans sa configuration dressée ; dans lequel l'épaisseur est perpendiculaire aux dimensions latérale et longitudinale de la structure multiniveau ; et
d) n'importe quelles combinaisons de a) à c).

20. Article absorbant selon l'une quelconque des revendications précédentes, dans lequel, dans la structure, les zones où se trouvent les ligaments et les zones entre des ligaments voisins ont un module allant d'environ 0,01 N/mm$^2$ à environ 0,3 N/mm$^2$, mesuré selon le procédé de test présenté ici.

Fig. 1a

Fig. 1b

Fig. 2a

Fig. 2b

Fig. 3a

Fig. 3b

**Fig. 4**

EP 3 067 027 B1

Fig. 5a

Fig. 5b

Fig. 5c

Fig. 6a

Fig. 6b

Fig. 6c

EP 3 067 027 B1

Fig. 7a

Fig. 7b

Fig. 7c

EP 3 067 027 B1

**Fig. 8a**

**Fig. 8b**

## Fig. 9a

## Fig. 9b

## Fig. 10

Fig. 11a

Fig. 11b

Fig. 11c

Fig. 12a

Fig. 12b

Fig. 12c

EP 3 067 027 B1

Fig. 13a

Fig. 13b

Fig. 13c

**Fig. 14a**

**Fig. 14b**

Fig. 15a

Fig. 15b

EP 3 067 027 B1

Fig. 16a

Fig. 16b

Fig. 17

F

500

520

100

α

α=90°

515

510

**Fig. 18**

## Fig. 19

## Fig. 20

Fig. 21

Fig. 22

Fig. 23

Fig. 24

Fig. 25

Fig. 26

Fig. 27

EP 3 067 027 B1

**Fig. 28**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4808178 A
- US 4909803 A, Aziz
- US 4695278 A, Lawson
- US 4795454 A, Dragoo
- US 4704116 A, Enloe
- US 7189219 B, Kasai
- US 6186996 B
- US 2006142723 A
- EP 1132068 A2
- EP 0904759 A2
- US 3860003 A
- US 5221274 A
- US 5554145 A
- US 5569234 A
- US 5580411 A
- US 6004306 A